# EUROPEAN PATENT SPECIFICATION

(11) **EP 3 927 370 B1**
(45) Date of publication and mention of the grant of the patent: **24.04.2024**
(21) Application number: 20711461.2
(22) Date of filing: 18.02.2020
(51) Int. Cl.: A61K 39/00, A61P 35/00, A61P 37/04, C12N 5/0784, C12N 5/0783

(54) **METHODS FOR PRODUCING AUTOLOGOUS T CELLS USEFUL TO TREAT CANCERS AND COMPOSITIONS THEREOF**
VERFAHREN ZUR HERSTELLUNG AUTOLOGER T-ZELLEN ZUR BEHANDLUNG VON KREBS UND ZUSAMMENSETZUNGEN DAVON
PROCÉDÉS DE PRODUCTION DE LYMPHOCYTES T AUTOLOGUES UTILISÉS POUR TRAITER LES CANCERS, ET COMPOSITIONS ASSOCIÉES

(30) Priority: 19.02.2019 US 201962807644 P; 29.03.2019 US 201962826974 P; 27.11.2019 US 201962941610 P; 27.11.2019 US 201962941614 P
(43) Date of publication of application: 29.12.2021
(60) Divisional application: 24162638.1
(73) Proprietor: Myst Therapeutics, LLC, New York, NY 10010 (US)
(72) Inventor: LANGER, Timothy J., New York, New York 10010 (US); CECCARELLI, Jacob, New York, New York 10010 (US)
(74) Representative: J A Kemp LLP
(86) International application number: PCT/US2020/018711
(87) International publication number: WO 2020/172202

(56) References cited:
- WO-A1-2016/053339
- WO-A1-2018/183485
- US-A1- 2017 224 800
- RAMI YOSSEF ET AL: "Enhanced detection of neoantigen-reactive T cells targeting unique and shared oncogenes for personalized cancer immunotherapy", JCI INSIGHT, vol. 3, no. 19, 4 October 2018 (2018-10-04), XP055542887, DOI: 10.1172/jci.insight.122467
- Y. LI ET AL: "MART-1-Specific Melanoma Tumor-Infiltrating Lymphocytes Maintaining CD28 Expression Have Improved Survival and Expansion Capability Following Antigenic Restimulation In Vitro", THE JOURNAL OF IMMUNOLOGY, vol. 184, no. 1, 30 November 2009 (2009-11-30), pages 452-465, XP055210596, ISSN: 0022-1767, DOI: 10.4049/jimmunol.0901101

## Description

### Field

The present disclosure provides methods for manufacturing T cells. In certain embodiments, methods for manufacturing T cells which express a novel group of cell surface receptors that recognize peptides on the surface of a target cell are provided. The present disclosure also provides populations of T cells produced by methods described herein and pharmaceutical compositions thereof.

### Background

The process for producing autologous tumor infiltrating lymphocyte (TIL) therapies for use in cancer is lengthy, involves a low number of reactive cells and is not suited for commercial applications. Therefore it would be desirable to develop improvements to the T cell manufacturing process to overcome these limitations. Provided herein are embodiments that meet such needs. Yossef et al. JCI Insight, 3 (2018) describes a method of enhanced detection of neoantigen-reactive T cells targeting unique and shared oncogenes for personalized cancer immunotherapy.

### Summary of the invention

The invention is defined in the independent claim. In particular, the invention provides a method for manufacturing T cells for use in a therapeutic cell composition, wherein the T cells express a T cell receptor (TCR) that recognizes an antigen on the surface of a target cell, the method comprising:
a. processing a biological sample containing a population of T lymphocyte cells obtained from a donor subject that has a tumor to produce a first population of T lymphocyte cells;
b. stimulating the first population with one or more first T-cell stimulating agents of lymphocytes under conditions for expansion of T cells in the population to produce a second population of activated T cells wherein the stimulation is performed in a closed system using serum free medium, wherein the one or more first T-cell stimulating agents is or comprises recombinant IL-2;
c. co-culturing the second population of T cells in the presence of antigen presenting cells that present one or more non-native peptide on a major histocompatibility complex (MHC) in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor;
d. selecting, from the third population of cells, the population of T cells containing endogenous TCR that are reactive to peptides present on the APCs based on one or more upregulation markers expressed on reactive or activated T cells in a closed system to produce a fourth population containing the selected T cells; and
e. expanding the fourth population of selected cells in the presence of one or more second T-cell stimulating agents of lymphocytes under conditions to expand T cells in the population in a closed system using serum free medium to produce a composition of expanded T cells for use as a therapeutic cell composition, wherein the one or more second T-cell stimulating agents is or comprises recombinant IL-2.

Further aspects of the invention and preferred embodiments are defined in the dependent claims. Any aspects, embodiments and examples of the present disclosure which do not fall under the scope of the appended claims do not form part of the invention and are merely provided for illustrative purposes.

### Summary of the technical teachings

The technical information set out below may in some respects go beyond the disclosure of the invention, which is defined by the appended claims. The additional technical information is provided to place the actual invention in a broader technical context and to illustrate possible related technical developments. Such additional technical information which does not fall within the scope of the appended claims, is not part of the invention. Accordingly, any aspects, embodiments and examples of the present disclosure which do not fall under the scope of the appended claims do not form part of the invention and are merely provided for illustrative purposes. The invention provides a method for manufacturing T cells for use in a therapeutic cell composition, wherein the T cells express a T cell receptor (TCR) that recognizes an antigen on the surface of a target cell, the method comprising: (1) processing a biological sample containing a population of T lymphocytes obtained from a donor subject that has a tumor to produce a first population of T lymphocyte cells; (2) stimulating the first population with one or more first T-cell stimulating agents of lymphocytes under conditions for expansion of T cells in the population to produce a second population of activated T cells wherein the stimulation is performed in a closed system using serum free medium, wherein the one or more first T-cell stimulating agents is or comprises recombinant IL-2; (3) co-culturing the second population of T cells in the presence of antigen presenting cells that present one or more non-native peptides on a major histocompatibility complex (MHC) in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor; (4) selecting, from the third population of cells, the population of T cells containing endogenous TCR that are reactive to peptides present on the APCs based on one or more upregulation markers expressed on reactive or activated T cells in a closed system to produce a fourth population containing the selected T cells; and (5) expanding the fourth population of selected cells in the presence of one or more second T-cell stimulating agents of lymphocytes under conditions to expand T cells in the population in a closed system using serum free medium to produce a composition of expanded T cells for use as a therapeutic cell composition, wherein the one or more second T-cell stimulating agents is or comprises recombinant IL-2.

The method comprises expanding T cells *in vitro.* In some of any of the embodiments, the antigen presenting cells are nucleated cells such as dendritic cells, mononuclear phagocytes, B lymphocytes, endothelial cells or thymic epithelium. In some of any of the embodiments, the antigen presenting cells are dendritic cells. In some of any of the embodiments, the T cells are autologous the subject. In some of any of the embodiments, the T cells are autologous to a subject having a tumor. In some of any such embodiments, the T cells are autologous to the patient. In some of any of the embodiments, the T cells are obtained from a healthy donor. In some of any of the embodiments, the MHC molecule is a class I molecule. In some of any of the embodiments, the MHC molecule is a class II molecule. In some of any of the embodiments, the MHC molecule is of class I and II. In some of any embodiments, the T cells are CD4+ cells. In some of any of the embodiments, the T cells are CD8+ cells. In some of any of the embodiments, the T cells are CD4+ cells and CD8+ cells.

The T cell stimulating agents are recombinant IL-2 and optionally one or more of an anti-CD3 antibody; an anti-CD28 antibody; or a recombinant cytokine selected from among IL-7, IL-15 and IL-21.

The first T cell stimulating agents are recombinant IL-2 and optionally one or more of an anti-CD3 antibody; an anti-CD28 antibody; or a recombinant cytokine selected from among IL-7, IL-15 and IL-21. The first T cell stimulating agent is or comprises recombinant IL-2. In some of any of the embodiments, the concentration of recombinant IL-2 is from 100 IU/mL to 6000 IU/mL. In some of any of the embodiments, the concentration of recombinant IL-2 is from 300 IU/mL to 1000 IU/mL. In some embodiments, the concentration of recombinant IL-2 is at or about 300 IU/mL.

In some of any of the embodiments, the T cell stimulating agent further comprises an anti-CD3 antibody, such as OKT3. In some embodiments, the concentration of the anti-CD3 antibody is at or about 50 ng/mL. The second T cell stimulating agents are recombinant IL-2 and optionally one or more of an anti-CD3 antibody; an anti-CD28 antibody; or a recombinant cytokine selected from among IL-7, IL-15 and IL-21. The second T-cell stimulating agents is or comprises recombinant IL-2. In some of any of the provided embodiments, the concentration of recombinant IL-2 is from 100 IU/mL to 6000 IU/mL. In some of any of the embodiments, the concentration of recombinant IL-2 is from 300 IU/mL to 1000 IU/mL. In some embodiments, the concentration of recombinant IL-2 is at or about 300 IU/mL. In some of any of the embodiments, the T cell stimulating agent further comprises an anti-CD3 antibody, such as OKT3. In some embodiments, the concentration of the anti-CD3 antibody is at or about 50 ng/mL.

In some of any of the embodiments, the one or more non-native peptide comprises an individual peptide or a pool of peptides. Non-native peptides are peptide corresponding to the nonsynonymous somatic mutations associated in the patient's tumor. In some of any of the provided embodiments, the one or more non-native peptides are loaded on antigen presenting cells by transfection of in vitro transcribed synthesized minigene constructs encoding for nonsynonomous somatic mutated amino acids. In some embodiments, the mutated amino acids are flanked on each side by 12 amino acids from endogenous proteins, in tandem, wherein the transcribed minigene constructs generate individual peptides. In some of any of the embodiments, non-native peptides are expressed on nucleated cells by electroporation of peptide pools associated with nonsynonymous somatic mutations into the antigen presenting cells. In some embodiments, the mutated amino acids are flanked on each side by 12 amino acids from endogenous proteins, in tandem, wherein the transcribed minigene constructs generate individual peptides. In some of any of the embodiments, non-native peptides are expressed on antigen presenting cells by synthesizing minigene constructs encoding for nonsynonymous somatic mutated amino acids, flanked on each side by 12 amino acids from endogenous proteins in tandem, then transcribed using *in-vitro* RNA transcription to generate individual peptides and then transfected into the antigen presenting cells.

In some of any of the embodiments, non-native peptides are expressed on nucleated cells by peptide pulsing, such as by electroporation, of peptide pools consisting of 12 long peptides associated with nonsynonymous somatic mutations into the antigen presenting cells. In some of any of the embodiments, the one or more non-native peptides are loaded on antigen presenting cells by peptide pulse. In some embodiments, the peptide pulse is carried out by electroporation. In some of any of the embodiments, non-native peptides are expressed on nucleated cells by peptide pulsing, such as by electroporation, of peptide pools associated with nonsynonymous somatic mutations into the antigen presenting cells. In some cases, each peptide of the peptide pool is 9 to 15 amino acids in length, such as at or about 9 amino acids, 10 amino acids, 11 amino acids, 12 amino acids, 13 amino acids, 14 amino acids or 15 amino acids in length. In some cases, each peptide of the peptide pools is a 12 amino acid long peptide. In some of any of the embodiments, the one or more non-native peptide is 5-30 amino acids. In some of any of the embodiments, the one or more non-native peptide is 12-25 amino acids. In some of any of the embodiments, the one or more non-native peptide is at or about 25 amino acids or at or about 12 amino acids.

In some of any of the embodiments, the ratio of peptide pools to antigen presenting cells during the peptide pulsing (e.g. electroporation) is between 0.001 and 100 µg per 1 million cells, between 0.01 and 100 µg per 1 million cells, between 0.1 and 100 µg per 1 million cells, between 1 and 100 µg per 1 million cells, between 10 and 100 µg per 1 million cells.

In some of any of the embodiments, the concentration of peptide pools for the pulsing (e.g. electroporation) is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL.

In some embodiments, the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL. In some of any of the embodiments, the ratio of individual peptides to antigen presenting cells during the peptide pulsing (e.g. electroporation) is between 0.001 and 100 µ per 1 million cells, between 0.01 and 100 µg per 1 million cells, between 0.1 and 100 µg per 1 million cells, between 1 and 100 µg per 1 million cells, between 10 and 100 µg per 1 million cells. In some embodiments, the concentration of individual peptides for the peptide pulse is between at or about 0.0001 µg/mL and at or about 40 µg/mL or at or about 0.001 µg/mL and at or about 40 µg/mL. In some embodiments, the concentration of individual peptides for the peptide pulsing (e.g. electroporation)is between at or about 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL. In some embodiments, the concentration of individual peptides for the peptide pulse is between at or about 0.0001 µg/mL and at or about 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, or at or about 0.0001 µg/mL and at or about 0.001 µg/mL.

In some of any of the embodiments, non-native peptides are derived from endosomal or lysosomal proteins. In some of any of the embodiments, non-native peptides are derived from cytosolic proteins.

In some of any of the embodiments, the co-culture ratio of antigen presenting cells to T cells is between 20:1 and 1:1, between 15:1 and 1:1, between 10:1 and 1:1, between 5:1 and 1:1, between 2.5:1 and 1:1.

In some of any of the embodiments, the co-culture ratio of antigen presenting cells to T Cells is between 20:1 and 1:1, between 15:1 and 1:1, between 10:1 and 1:1, between 5:1 and 1:1, or between 2.5:1 and 1:1. In some of any of the embodiments, the co-culture ratio of antigen presenting cells to T cells is or is about 1:1.

In some of any of the embodiments, separation of antigen presenting cells is using magnetic separation, gravimetric separation, selective binding or cell sorting using flow cytometry.

Provided herein is a method of manufacturing T cells for use in a therapeutic cell composition, wherein the T cells express a T cell receptor (TCR) that recognizes an antigen on the surface of a target cell: (a) processing a biological sample containing a population of T lymphocyte cells obtained from a donor subject that has a tumor to produce a first population of T lymphocyte cells; (b) stimulating the first population with one or more first T-cell stimulating agents of lymphocytes under conditions for expansion of T cells in the population to produce a second population of activated T cells, wherein the one or more T-cell stimulating agents is or includes recombinant IL-2 at a concentration of 300 IU/mL to 1000 IU/mL and wherein the stimulation is performed in a closed system using serum free medium; (c) co-culturing the second population of T cells in the presence of autologous dendritic cells loaded with one or more non-native peptides for presenting the one or more non-native peptide on a major histocompatibility complex (MHC), wherein the co-culturing is in a closed system using serum free medium and wherein the one or more non-native peptides are peptides corresponding to nonsynonymous somatic mutations associated in the tumor of the subject and are loaded with the dendritic cells at a concentration of less than 0.02 µg/mL per individual peptide, to produce a third population of cells containing T cells comprising endogenous T cell receptors reactive to mutation encoding peptides of the tumor; (d) selecting, from the third population of cells, the population of T cells containing endogenous TCR that are reactive to peptides present on the APCs based on one or more upregulation markers expressed on reactive or activated T cells in a closed system to produce a fourth population containing the selected T cells; and (e) expanding the fourth population of selected cells in the presence of one or more second T-cell stimulating agents of lymphocytes under conditions to expand T cells in the population, wherein the one or more second T-cell stimulating agents is or includes recombinant IL-2 at a concentration of 300 IU/mL to 1000 IU/mL and wherein the expanding is in a closed system using serum free medium to produce a composition of expanded T cells for use as a therapeutic cell composition.

In some of any of the embodiments, the concentration of each individual peptide of the one or more non-native peptide is 0.00001 µg/mL and at or about 0.01 µg/mL. In some of any of the embodiments, the concentration of each individual peptide of the one or more non-native peptide is 0.0001 µg/mL and 0.001 µg/mL. In some of any of the embodiments, the one or more non-native peptide comprises an individual peptide or a pool of individual peptides. In some of any of the embodiments, each of the one or more non-native peptides are 12 to 25 amino acids in length. In some embodiments, the one or more non-native peptide is at or about 25 amino acids in length. In some of any of the provided embodiments, the co-culture ratio of dendritic cells to T Cells is between 5:1 and 1:5 or is between 3:1 and 1:3. In some embodiments, the co-culture ratio is or is about 1:1. In some of any of the provided embodiments, the co-culture ratio of dendritic cells to T cells is or is about 1:1. In some of any of the provided embodiments, the co-culturing is for 2 hours to 24 hours. In some of any of the provided embodiments, the co-culturing is for at or about 6 hours.

In some of any of the embodiments, the selection is performed using a florescence based cell sorter. In some of any of the embodiments, selection is performed using a florescence based cell sorter in a closed system using serum free medium. In some of any of the embodiments, the selection is by 1 run, 2 runs, 3 runs or 4 runs by the fluorescence based cell sorter. In some of any of the embodiments, selection is performed at rate between 10,000 and 100,000 cells/ second using a florescent based disposable fluidics cell sorter. In some of any of the embodiments, the closed system is a bag system.

In some of any of the embodiments, upregulation markers are surface expressed proteins on the T cell that are only expressed when the T cells endogenous TCR recognizes a peptide expressed by the APCs.

In some of any of the embodiments, the one or more upregulation markers are selected from the group consisting of CD107, CD107a, CD39, CD103, CD137 (4-1BB), CD59, CD69, CD90, CD38, CD30, CD154, CD252, CD134 (OX40), CD258, CD256, PD-1, TIM-3 and LAG-3. In some of any of the embodiments, the one or more upregulation marker is selected from the group consisting of CD38, CD39, CD6, CD90, CD134 and CD137. In some of any of the embodiments, the one or more upregulation maker is CD134 and/or CD137. In some of any of the embodiments, the one or more upregulation marker is selected from the group consisting of CD107, CD107a, CD39, CD103, CD59, CD90, CD38, CD30, CD154, CD252, CD134, CD258 and CD256. In some of any of the embodiments, the one or more T upregulation marker is selected from the group consisting of CD107a, CD39, CD103, CD59, CD90 and CD38. In some of any of the embodiments, the one or more T upregulation marker comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38. In some of any of the embodiments, the one or more T cell upregulation marker further comprises CD137. In some of any of the embodiments, the one or more T cell upregulation marker comprises at least two markers selected from CD107a and CD137, CD38 and CD137, CD103 and CD137, CD59 and CD137, CD90 and CD137 and CD38 and CD137. In some of any of the provided embodiments, the one or more upregulation marker further comprises at least one marker selected from the group consisting of PD-1, TIM-3 and LAG-3.

In some of any of the embodiments, stimulating the first population is for 7 to 21 days. In some embodiments, stimulating the first population is for 7 to 14 days. In some of any of the provided embodiments, expanding the fourth population is for 7 to 21 days. In some embodiments, expanding the fourth population is for 7 to 14 days.

In some of any of the embodiments, the time from processing the biological samples containing the population of lymphocytes to producing the expanded T cells is less than 30 days. In some of any of the embodiments, the time from enriching the population of lymphocytes to producing the expanded T cells is less than 30 days. In some of any of the embodiments, the population of lymphocytes from the biological sample are from tumor infiltrating lymphocytes, lymph lymphocytes or peripheral blood mononuclear cells. In some of any of the embodiments, the population of lymphocytes from the biological sample are derived from tumor infiltrating lymphocytes, lymph lymphocytes or peripheral blood mononuclear cells.

In some of any of the embodiments, the tumor is a tumor of an epithelial cancer. In some of any of the embodiments, the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer. In some of any of the provided embodiments, the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

In some of any of the embodiments, the population of lymphocytes are processed from a resected tumor, such as tumor fragments from the resected tumor. In some of any of the embodiments, the tumor is a melanoma.

In some of any of the provided embodiments, the population of lymphocytes are processed as a single cell suspension obtained by homogenization and/or enzymatic digestion of one or more tumor fragments from a resected tumor. In some of any of the provided embodiments, the population of lymphocytes are processed as a single cell suspension obtained by homogenization and enzymatic digestion of one or more tumor fragments from a resected tumor. In some of any of the embodiments, the enzymatic digestion is by a collagenase. In some embodiments, the collagenase is a collagenase type IV. In some embodiments, the collagenase is a collagenase type I/II. In some of any of the embodiments, the tumor is a colorectal cancer (CRC).

In some of any of the embodiments, the composition of expanded cells are used to treat a cancer patient.

In some of any of the embodiments, the method further comprises harvesting cells produced by the method. In some of any of the embodiments, the method further comprises formulating the harvested cells with a cryoprotectant.

In some of any of the embodiments, the population of T cells of the therapeutic composition produced by the method express greater than or equal to 1 T cell surface receptor that recognizes greater than or equal to 1 specific antigen on a target cell expressing nonsynonymous mutations.

In some of any of the embodiments, the population of T cells of the therapeutic composition produced by the method is enriched for tumor-reactive T cells or T cells that are surface positive for one or more T cell upregulation marker expressed on reactive or activated T cells compared to the first population of T cells, such as enriched 10-fold to 1000-fold. In some of any of the embodiments, the population of T cells of the therapeutic composition produced by the method is enriched for tumor-reactive T cells or T cells that are surface positive for one or more T cell upregulation marker expressed on reactive or activated T cells compared to the second population of T cells, such as enriched 10-fold to 1000-fold. In some of any of the embodiments, the population of T cells of the therapeutic composition produced by the method is enriched for tumor-reactive T cells or T cells that are surface positive for one or more T cell upregulation marker expressed on reactive or activated T cells compared to the third population of T cells, such as enriched 1.5-fold to 50-fold.

In some of any of the embodiments, the population of T cells of the therapeutic composition produced by the method are able to produce IFNgamma at a concentration of greater than at or about 30 pg/mL following antigen-specific stimulation. In some embodiments, the IFNgamma produced following antigen-specific stimulation is as greater than at or about 40 pg/mL, greater than at or about 50 pg/mL, greater than at or about 60 pg/mL, greater than at or about 70 pg/mL, greater than at or about 80 pg/mL or greater than at or about 90 pg/mL.

We also describe a population of T cells produced by any of the provided methods wherein the population of T cells express greater than or equal to 1 T cell surface receptor that recognizes greater than or equal to 1 specific antigen on a target cell expressing nonsynonymous mutations.

In some of any of the disclosures, the population of T cells produced by the provided methods is enriched for tumor-reactive T cells or T cells that are surface positive for one or more T cell upregulation marker expressed on reactive or activated T cells compared to the first population of T cells, such as enriched 10-fold to 1000-fold. In some of any of the disclosures, the population of T cells produced by the provided methods is enriched for tumor-reactive T cells or T cells that are surface positive for one or more T cell upregulation marker expressed on reactive or activated T cells compared to the second population of T cells, such as enriched 10-fold to 1000-fold. In some of any of the disclosures, the population of T cells produced by the provided methods is enriched for tumor-reactive T cells or T cells that are surface positive for one or more T cell upregulation marker expressed on reactive or activated T cells compared to the third population of T cells, such as enriched 1.5-fold to 50-fold.

In some of any of the provided disclosures, the population of T cells produced by the provided methods is able to produce IFNgamma at a concentration of greater than at or about 30 pg/mL, such as greater than at or about 60 pg/mL, following antigen-specific stimulation. In some of any of the disclosures, the population of T cells produced by the method is able to produce IFNgamma at a concentration of greater than at or about 30 pg/mL following antigen-specific stimulation. In some disclosures, the IFNgamma produced following antigen-specific stimulation is as greater than at or about 40 pg/mL, greater than at or about 50 pg/mL, greater than at or about 60 pg/mL, greater than at or about 70 pg/mL, greater than at or about 80 pg/mL or greater than at or about 90 pg/mL.

We also describea population of T cells that express greater than or equal to 1 T cell surface receptor that recognizes greater than or equal to 1 specific antigen on a target cell expressing nonsynonymous mutations; (a) enriching a population of lymphocytes obtained from a donor subject to produce a first population of T lymphocytes; (b) stimulating the first population with one or more T-cell stimulating agents of lymphocytes to produce a second population of activated T cells wherein the stimulation is performed in a closed system using serum free medium; (c) co-culturing the second population of T cells in the presence of antigen presenting cells that present one or more non-native peptide on a major histocompatibility complex (MHC) in a closed system using serum free medium, said one or more non-native peptides are peptides corresponding to nonsynonymous somatic mutations associated in the tumor of a subject, to produce a third population of cells containing T cells comprising endogenous T cell receptors reactive to mutation encoding peptides of the tumor (tumor reactive T cells); (d) separating antigen presenting cells from T cells in the third population of cell in a closed system; (e) after the separating, Selecting T cells containing endogenous TCR that are reactive to peptides present on the APC based on upregulation markers on T cells in a closed system to produce a fourth population containing the selected cells; and (f) expanding the fourth population of selected cells in the presence of one or more T-cell stimulating agents of lymphocytes in a closed system using serum free medium.

The cell surface marker is a TCR. In some of any of the provided disclosures, the target cell is a cancer cell. In some of any of the provided disclosures, the cancer cell is an epithelial malignancy. In some of any of the provided disclosures, the antigen is a neoantigen.

In some of any of the provided disclosures, the time from enriching the population of lymphocytes to producing the expanded T cells is less than 30 days. In some of any of the provided disclosures, the T cells are used to treat a cancer patient.

We also describe a pharmaceutical composition comprising the therapeutic composition of T cells produced by any of the methods provided herein. In some of any of the disclosures, the pharmaceutical composition comprises a therapeutic dose of the T cells.

We also describe a composition comprising tumor-reactive T cells, wherein at least at or about 40%, at least at or about 50%, at least at or about 60%, at least at or about 70%, at least at or about 80%, or at least at or about 90% of the total cells or total T cells in the composition are tumor reactive T cells or are surface positive for one or more T cell upregulation marker expressed on reactive or activated T cells. In some of any of the disclosures, the one or more T cell upregulation marker is selected from the group consisting of CD107, CD107a, CD39, CD103, CD137 (4-1BB), CD59, CD90, CD38, CD30, CD154, CD252, CD134, CD258, CD256, PD-1, TIM-3 and LAG-3. In some of any of the disclosures, the one or more T cell upregulation marker is CD137 and/or CD 134. In some of any of the disclosures, the one or more T cell upregulation marker is selected from the group consisting of CD107, CD107a, CD39, CD103, CD59, CD90, CD38, CD30, CD154, CD252, CD134, CD258 and CD256. In some of any of the disclosures, the one or more T cell upregulation marker is selected from the group consisting of CD107a, CD39, CD103, CD59, CD90 and CD38. In some of any of the disclosures, the one or more T cell upregulation marker comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38. In some of any of the disclosures, the one or more T cell upregulation marker further comprises CD137. In some of any of the embodiments, the one or more reactive T cell upregulation marker comprises at least two markers selected from CD107a and CD137, CD38 and CD137, CD103 and CD137, CD59 and CD137, CD90 and CD137 and CD38 and CD137. In some of any of the provided disclosures, the one or more T cell marker upregulation further comprises at least one marker selected from the group consisting of PD-1, TIM-3 and LAG-3.

In some of any of the disclosures, the T cells are CD3+ T cells or comprise CD4+ T cells and/or CD8+ T cells. In some of any of the disclosures, the T cells comprise CD4+ T cells and CD8+ T cells, wherein the ratio of CD8+ T cells to CD4+ T cells is between at or about 1:100 and at or about 100:1, between at or about 1:50 and at or about 50:1, between at or about 1:25 and at or about 25: 1, between at or about 1: 10 and at or about 10:1, between at or about 1:5 and at or about 5:1, or between at or about 1:2.5 and at or about 2.5:1. In some of any of the disclosures, the number of tumor reactive T cells or total T cells surface positive for the T cell activation marker, or of viable cells thereof, in the composition is between at or about 0.5 x 10⁸ and at or about 50 x 10⁹, between at or about 0.5 x 10⁸ and at or about 30 x 10⁹, between 0.5 x 10⁸ and at or about 12 x 10⁹, between at or about 0.5 x 10⁸ and at or about 60 x 10⁸, between at or about 0.5 x 10⁸ and at or about 15 x 10⁸, between at or about 0.5 x 10⁸ and at or about 8 x 10⁸, between at or about 0.5 x 10⁸ and at or about 3.5x 10⁸, between at or about 0.5 x 10⁸ and at or about 1 x 10⁸, between 1 x 10⁸ and at or about 50 x 10⁹, between at or about 1 x 10⁸ and at or about 30 x 10⁹, between 1 x 10⁸ and at or about 12 x 10⁹, between at or about 1 x 10⁸ and at or about 60 x 10⁸, between at or about 1 x 10⁸ and at or about 15 x 10⁸, between at or about 1 x 10⁸ and at or about 8 x 10⁸, between at or about 1 x 10⁸ and at or about 3.5x 10⁸, between at or about 3.5 x 10⁸ and at or about 50 x 10⁹, between at or about 3.5 x 10⁸ and at or about 30 x 10⁹, between at or about 3.5 x 10⁸ and at or about 12 x 10⁹, between at or about 3.5 x 10⁸ and at or about 60 x 10⁸, between at or about 3.5 x 10⁸ and at or about 15 x 10⁸, between at or about 3.5 x 10⁸ and at or about 8 x 10⁸, between at or about 8 x 10⁸ and at or about 50 x 10⁹, between at or about 8 x 10⁸ and at or about 30 x 10⁹, between at or about 8 x 10⁸ and at or about 12 x 10⁹, between at or about 8 x 10⁸ and at or about 60 x 10⁸, between at or about 8 x 10⁸ and at or about 15 x 10⁸, between at or about 15 x 10⁸ and at or about 50 x 10⁹, between at or about 15 x 10⁸ and at or about 30 x 10⁹, between at or about 15 x 10⁸ and at or about 12 x 10⁹, between at or about 15 x 10⁸ and at or about 60 x 10⁸, between at or about 60 x 10⁸ and at or about 50 x 10⁹, between at or about 60 x 10⁸ and at or about 30 x 10⁹, between at or about 60 x 10⁸ and at or about 12 x 10⁹, between at or about 12 x 10⁹ and at or about 50 x 10⁹, between at or about 12 x 10⁹ and at or about 30 x 10⁹, or between at or about 30 x 10⁹ and at or about 60 x 10⁹, each inclusive. In some of any of the provided disclosures, the composition comprises a pharmaceutically acceptable excipient. In some of any of the disclosures, the composition comprises a cryoprotectant. In some of any of the disclosures, the composition is sterile.

We also describe a method of treating a subject having cancer, the method comprising administering a therapeutic dose of any of the pharmaceutical compositions provided herein. In some of any of the disclosures, the therapeutically effective dose is between 1 x 10⁹ and 10 x 10⁹ T cells. In some of any of the disclosures, the therapeutically effective dose is from more than 1 million to less than 100 million T cells per kilogram of body weight. In some of any of the disclosures, the therapeutically effective dose is from at or about 10 million to at or about 50 million T cells per kilogram of body weight. In some of any of the disclosures, the therapeutically effective dose is from more than 1 million to less than 10 million T cells per kilogram of body weight. In some of any of the disclosures, the therapeutically effective dose is about 5 million T cells per kilogram of body weight. In some of any of the disclosures, the therapeutically effective dose is about 10 million T cells per kilogram of body weight.

### Brief Description of the Drawings

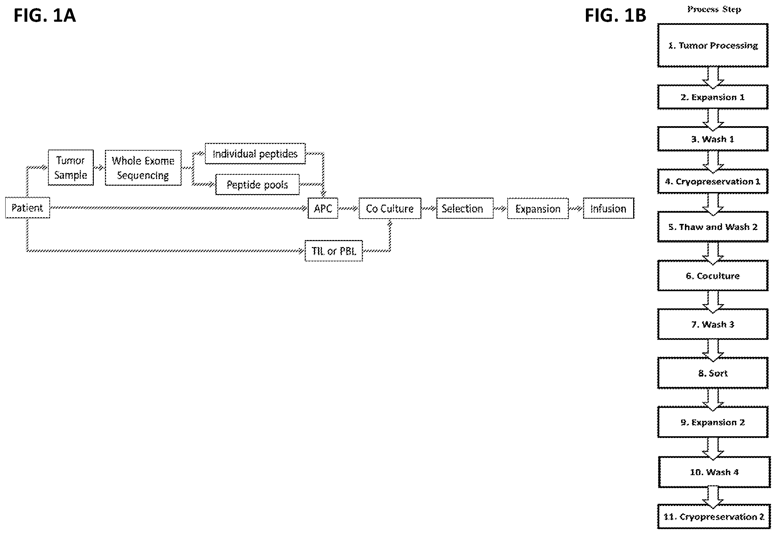

**FIG. 1A and 1B** depict process flow charts reflecting the methods described herein. **FIG. 1A** represents the step wise administration of the co-culture experiments described in Example 6 while **FIG. 1B** depicts a full process flow chart for the generation of a population of patient specific tumor-derived infiltrating T cells.

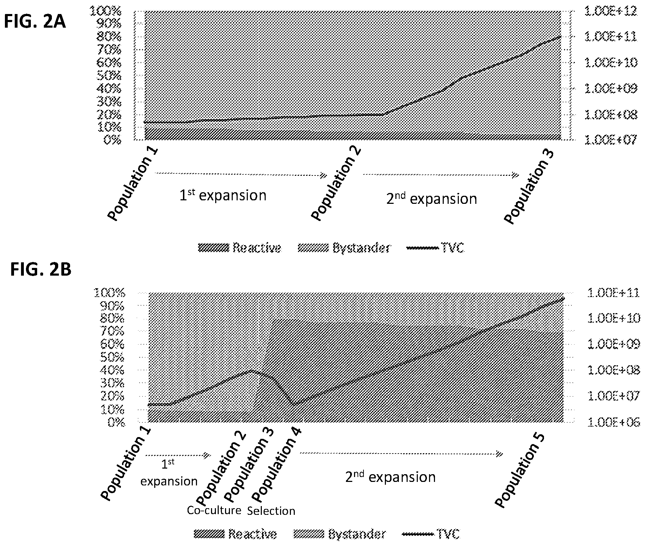

**FIG. 2A** depicts exemplary kinetics and T cell neoantigen reactivity in a typical TIL expansion process involving a bulk expansion of T cells with a first initial expansion and a second rapid expansion wherein reactivity remains low throughout the process, including within the final product. **FIG. 2B** further depicts the exemplary kinetics of a TIL expansion process involving a first initial expansion, followed by an enrichment of tumor-reactive T cells by co-culture with neoantigen peptide-presenting antigen presenting cells, selection of tumor-reactive cells for T cell activation (upregulation) markers, and a second expansion of enriched reactive cells.

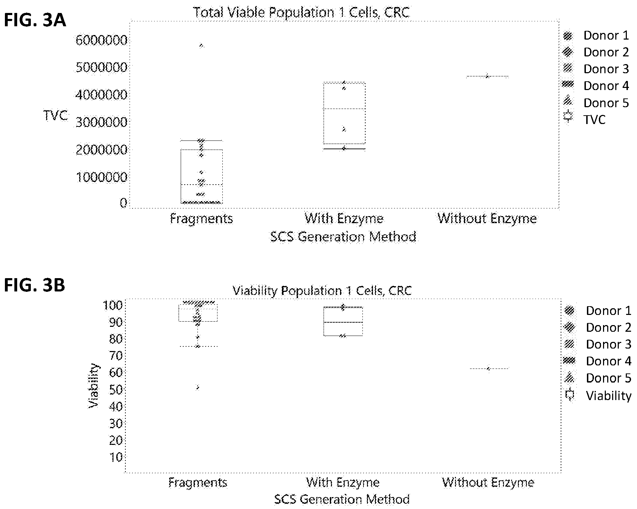

**FIG. 3A** depicts the generation of total viable Population 1 cells from patient derived CRC tumor tissue using fragment culture, homogenization with enzyme, and homogenization without enzyme. Digestion with and without enzyme both yielded more total cells than culture from fragments. Percent viability of these cells is shown in **FIG. 3B****.** Viabilities of cultures generated from fragments and digested with enzyme were higher than those derived using homogenization without enzyme.

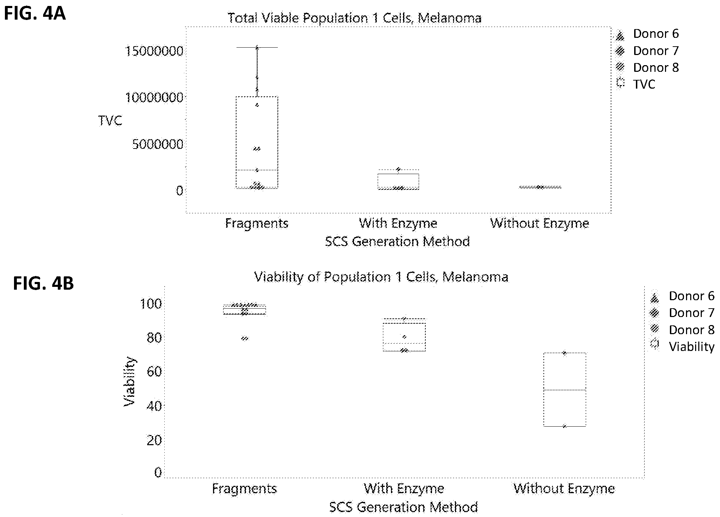

**FIG. 4A** depicts the generation of Population 1 cells from patient derived melanoma tumor tissue using fragment culture or homogenization with or without enzyme. Fragment culture yielded more total cells than cultures initiated from single cell suspensions. Percent viability of these cells is shown in **FIG. 4B****.** The population generated from fragments showed higher viability than cells from single cell suspensions.

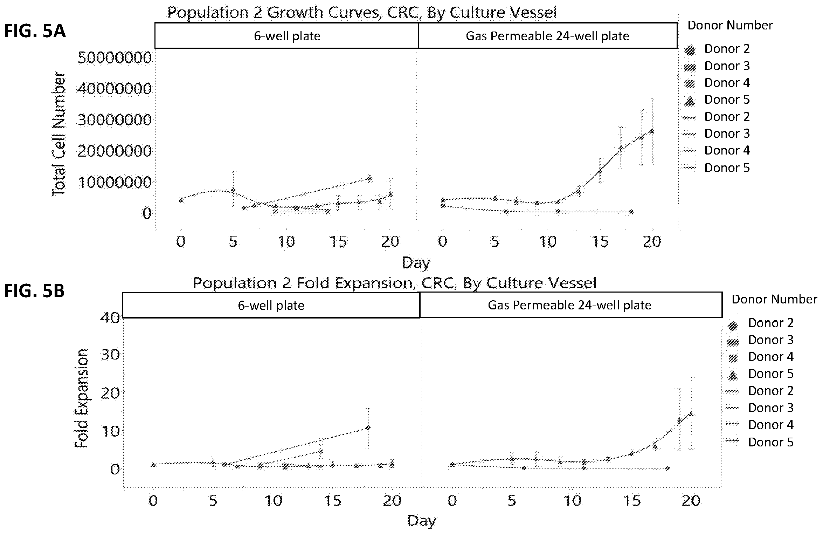

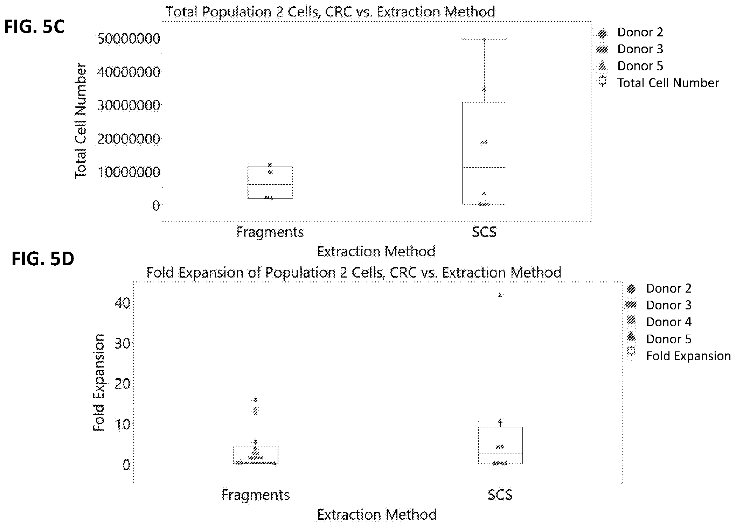

**FIG. 5** depicts growth curves **(****FIG. 5A****)** as well as fold expansion **(****FIG. 5B****)** of Population 2 cells derived from primary CRC tumors in either a conventional 6-well culture plate or a 24-well gas permeable culture plate. **FIG. 5** also depicts total cell number **(****FIG. 5C****)** as well as fold expansion **(****FIG. 5D****)** of Population 2 cells derived from primary CRC tumors contrasted by cellular extraction method, either fragment or single cell suspension culture.

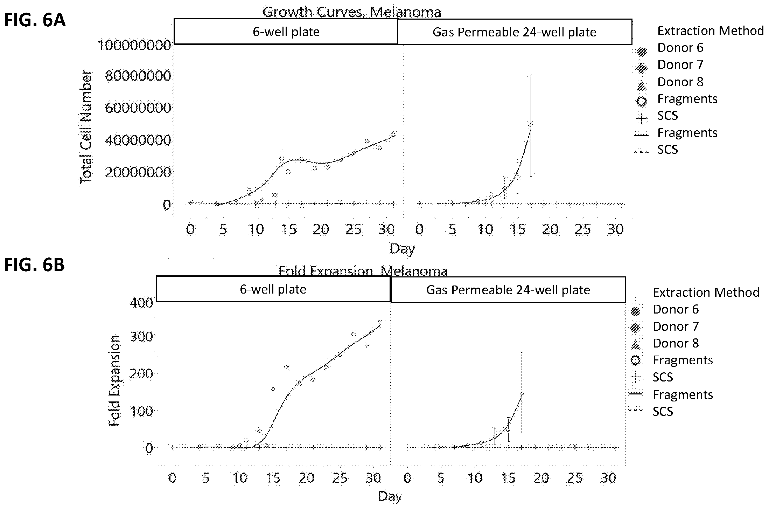

**FIG. 6** depicts growth curves **(****FIG. 6A****)** as well as fold expansion **(****FIG. 6B****)** of Population 2 cells derived from primary melanoma tumors in either a 6-well culture plate or a 24-well gas permeable culture plate.

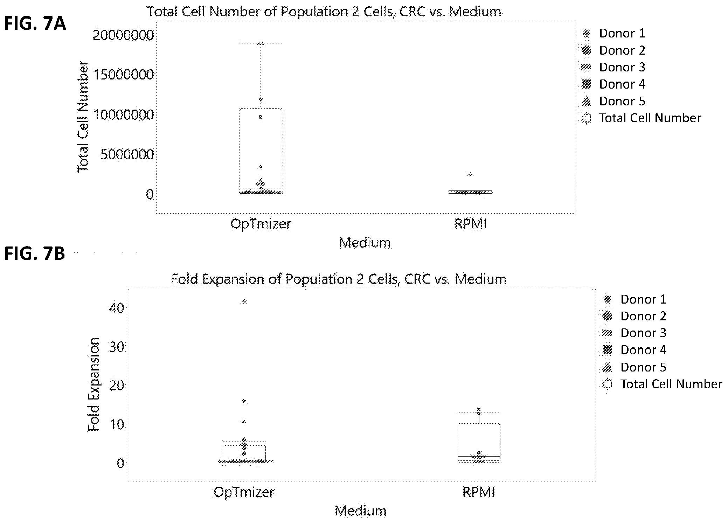

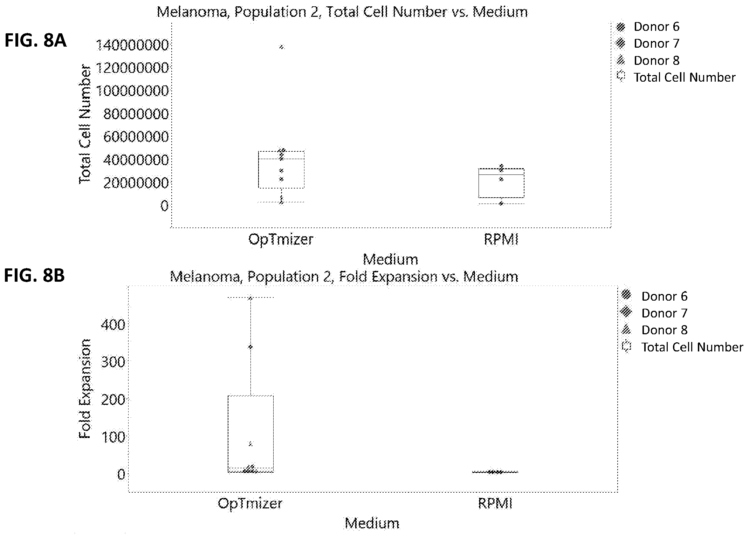

**FIG. 7** depicts total cell number **(****FIG.** 7A) as well as fold expansion **(****FIG. 7B****)** of Population 2 cells derived from primary CRC tumors using serum free OpTmizer or RPMI media supplemented with 5% human serum. Similarly, **FIG. 8** depicts total cell number **(****FIG. 8A****)** as well as fold expansion **(****FIG. 8B****)** of Population 2 cells derived from primary melanoma tumors using serum free OpTmizer or RPMI media supplemented with 5% human serum.

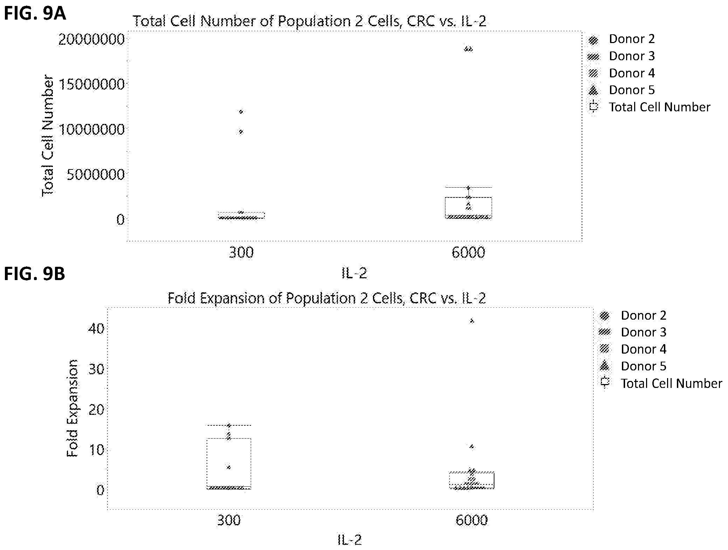

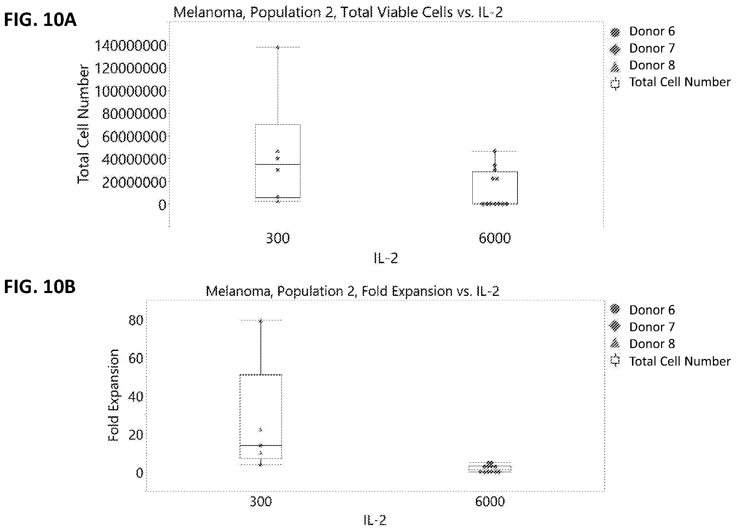

**FIG. 9** depicts total cell number **(****FIG. 9A****)** as well as fold expansion **(****FIG. 9B****)** of Population 2 cells derived from CRC tumors and cultured in media supplemented with either a low concentration (300 IU/mL) or a high concentration (600 IU/mL) of recombinant human IL-2. These data are similarly depicted for melanoma tumor derived cells in **FIG. 10A and 10B****.** A high concentration of IL-2 was not observed to be necessary for cellular expansion.

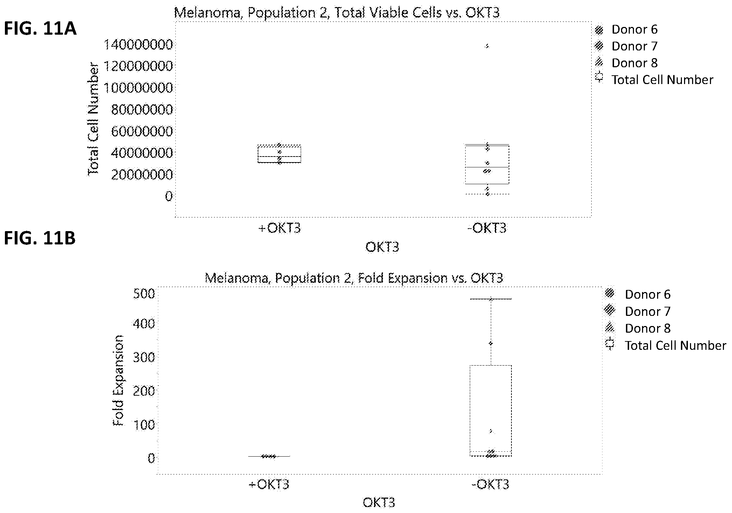

**FIG. 11A** depicts Population 2 total cell number and **FIG. 11B** depicts fold expansion from melanoma derived cell cultures that were unstimulated or stimulated with OKT3, an anti-CD3 monoclonal antibody, were observed to be largely similar.

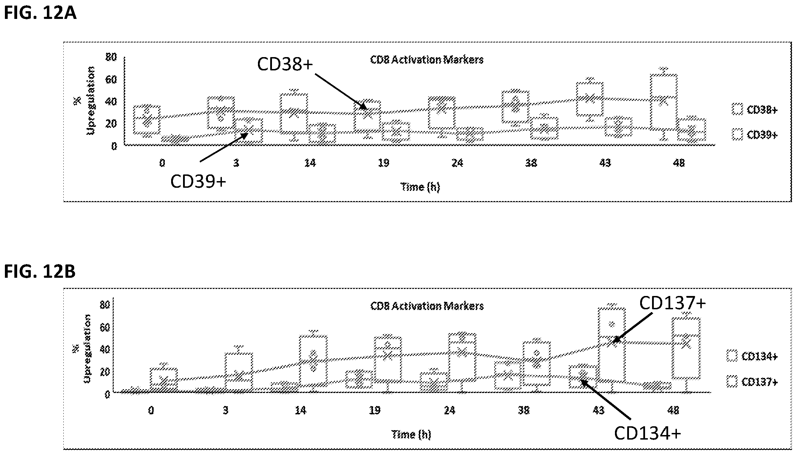

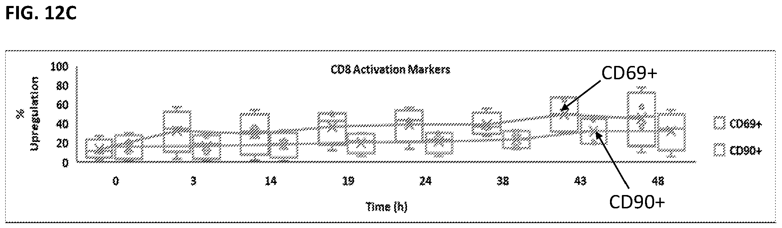

FIG. 12A-C depict percent upregulation of activation markers on CD8+ T cells, CD38 and CD39 **(****FIG. 12A****),** CD134 and CD137 **(****FIG. 12B****),** and CD69 and CD90 **(****FIG. 12C****),** between 0 and 48 hours after activation with OKT3.

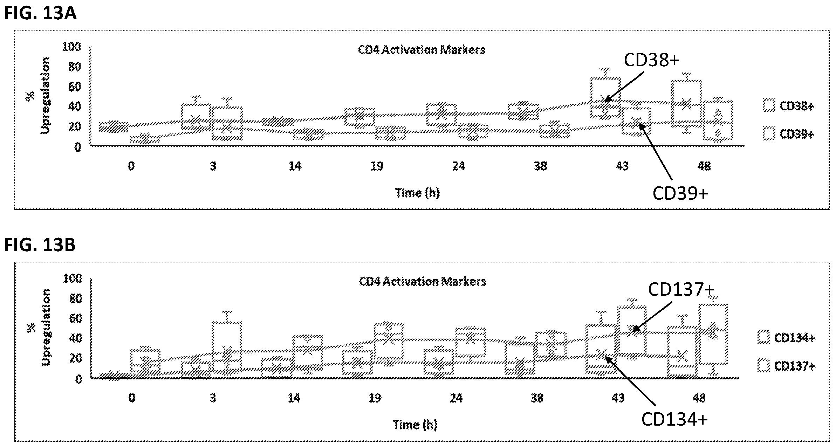

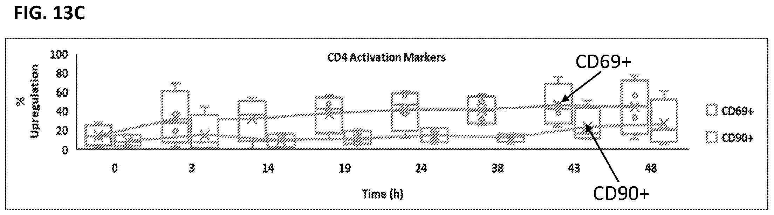

FIG. 13A-C depict percent upregulation of activation markers on CD4+ T cells, CD38 and CD39 **(****FIG. 13A****),** CD134 and CD137 **(****FIG. 13B****),** and CD69 and CD90 **(****FIG. 13C****),** between 0 and 48 hours after activation with OKT3.

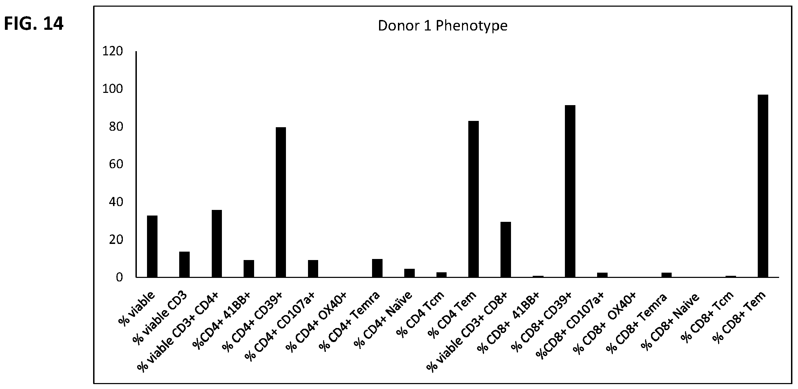

**FIG. 14** depicts expression of selected exemplary markers in a single cell suspension culture generated from a CRC tumor on Day 0.

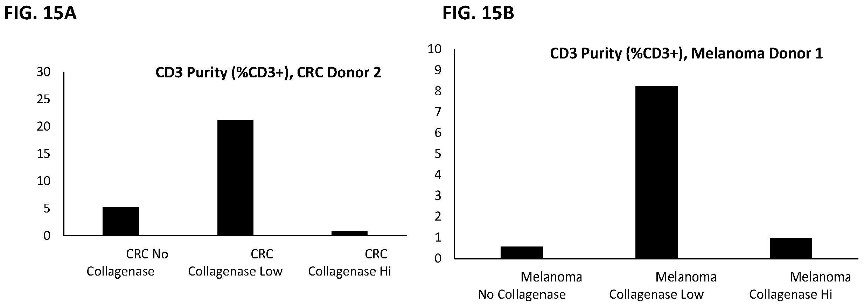

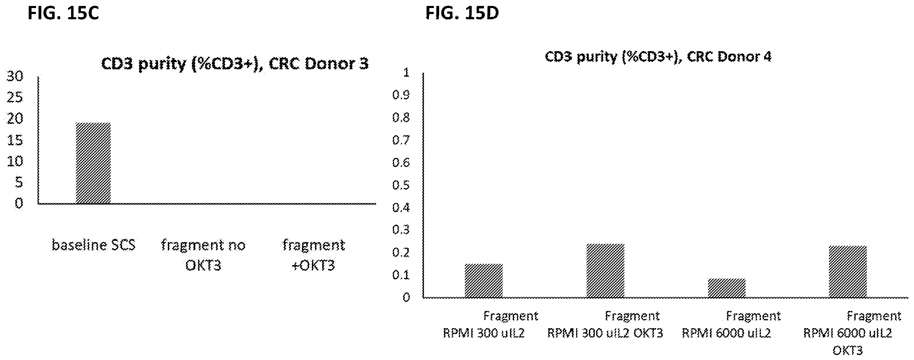

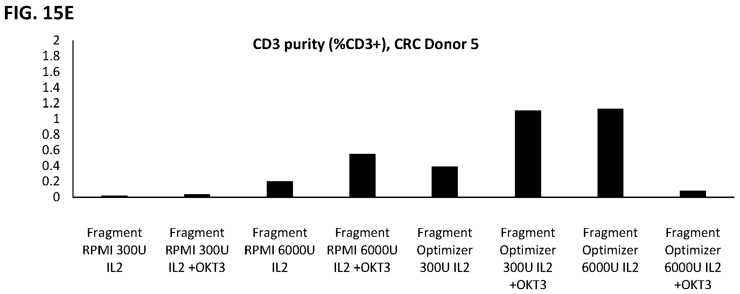

FIG. 15A-E depict CD3+ cell purity as a percent of Population 1 cells. **FIG. 15A** depicts the purity of cells from Day 0 SCS from a CRC tumor after homogenization without enzyme, with 1 mg/ml (low) enzyme, and 5 mg/ml (high) enzyme. These data are similarly shown for a melanoma derived culture in **FIG. 15B****.** **FIG. 15C** depicts the purity of CD3+ Population 1 cells from Day 0 (baseline SCS) and Day 6 from fragments cultured in the presence or absence of OKT3 stimulation. **FIG. 15D** shows the relative purity of CD3+ cells from a CRC donor on Day 11 using fragments cultured in media supplemented with either 6000 IU/mL (high) or 300 lU/mL (low) recombinant IL-2. **FIG. 15E** depicts Population 1 cells (Day 9) from fragments cultured in either serum free OpTmizer media or RPMI with either OKT3 stimulation and/or IL-2 at high or low concentrations. These observations support that SCSs from tumor biopsies of CRC patients may be more capable of providing a greater number of T cells for expansion than cells obtained from culture of tumor fragments.

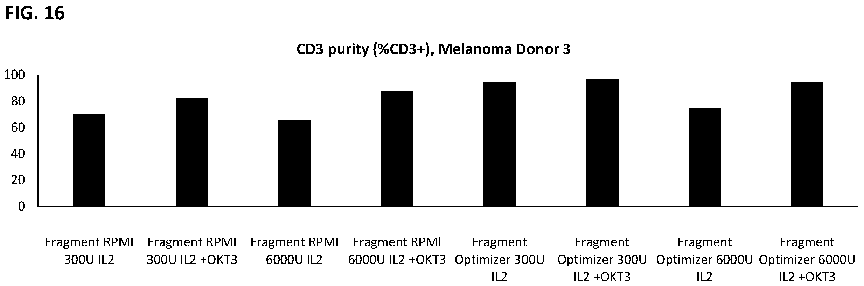

**FIG. 16** depicts the purity of CD3+ Population 1 cells derived from a melanoma patient as fragment cultures from Day 9 at high and low IL-2 concentrations and with serum containing RPMI medium or serum free OpTmizer.

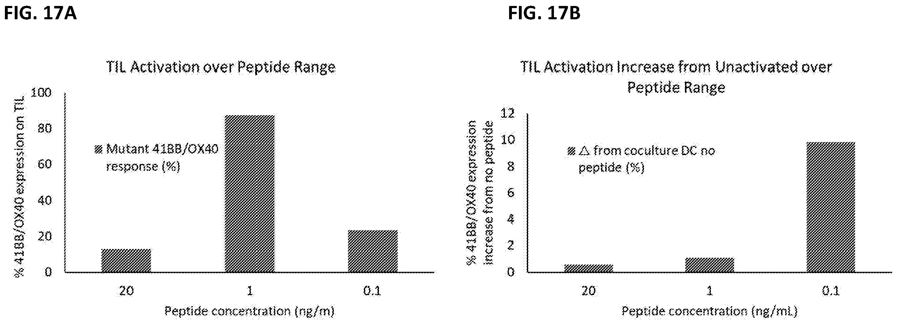

**FIG. 17A** depicts the generation of Population 3 cells following co-culture with dendritic cells loaded with peptide at concentrations from 0.1 ng/mL to 20 ng/mL. **FIG. 17B** depicts the fold increase in the same experiment from T cells which were co-cultured with unloaded dendritic cells **(****FIG. 17B****).**

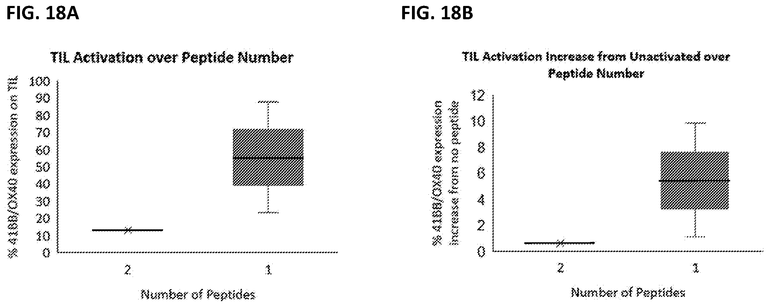

**FIG. 18A** compares stimulation with one peptide or two peptides reported as % 41BB/OX40 expression. **FIG. 18B** depicts stimulation with one peptide or two peptides reported as fold increase from T cells which were unactivated.

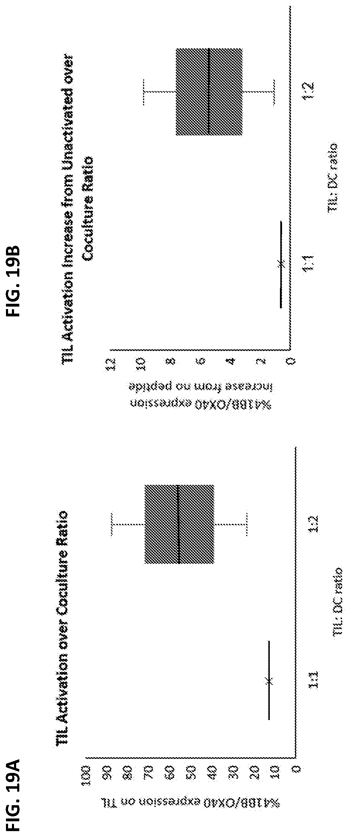

**FIG. 19A** compares two T cell to dendritic cell ratios, 1: 1 and 1:2, reported as % 41BB/OX40 expression. **FIG. 19B** compares two T cell to dendritic cell ratios, 1: 1 and 1:2, reported as fold increase from T cells which were unactivated.

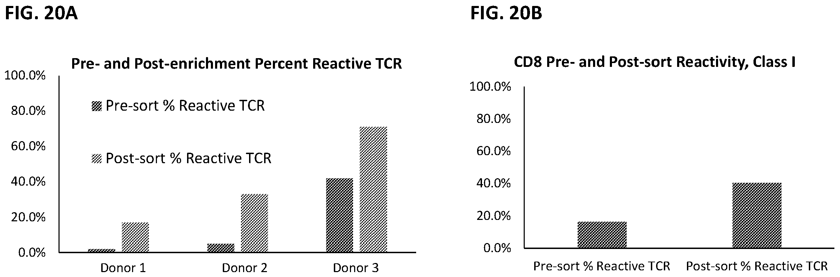

**FIG. 20A** depicts percent neoantigen reactive TCR before and after coculture with autologous neoantigen peptides and sorting of T cells sourced from the peripheral blood of three healthy donors. **FIG. 20B** depicts average class I reactivity pre- and post-coculture and sorting of CD8+ cells.

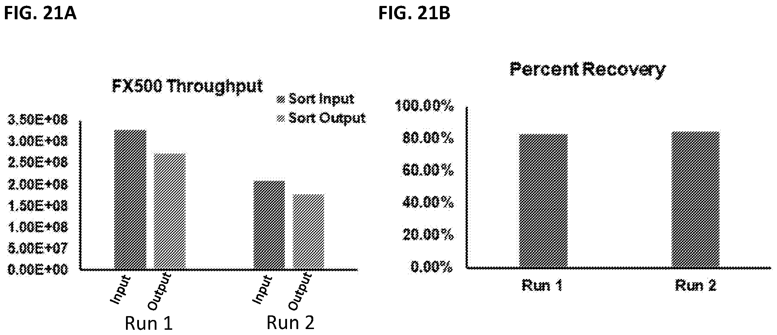

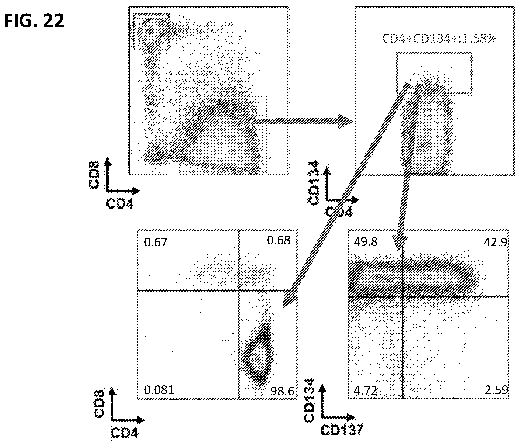

**FIG. 21A and FIG. 21B** depict recovery from cell sorting using the Sony FX500 as both total cell input and output for two independent runs **(****FIG. 21A****)** and percent recovery **(****FIG. 21B****).** **FIG. 22** depicts purity and gating of a CD4+ population from cell sorting using Sony FX500. The results demonstrate a high recovery of cells after selection and sorting of cells positive for upregulation markers.

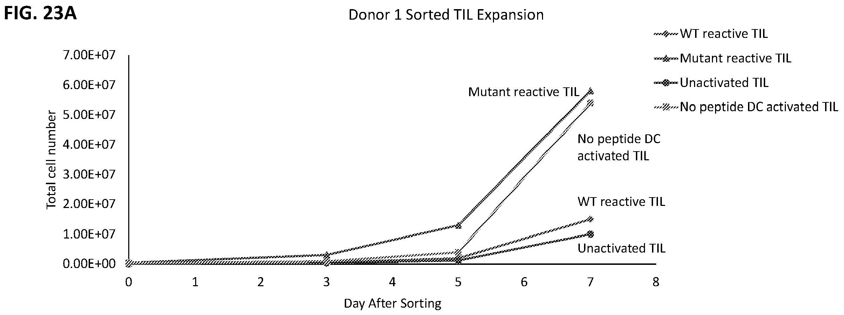

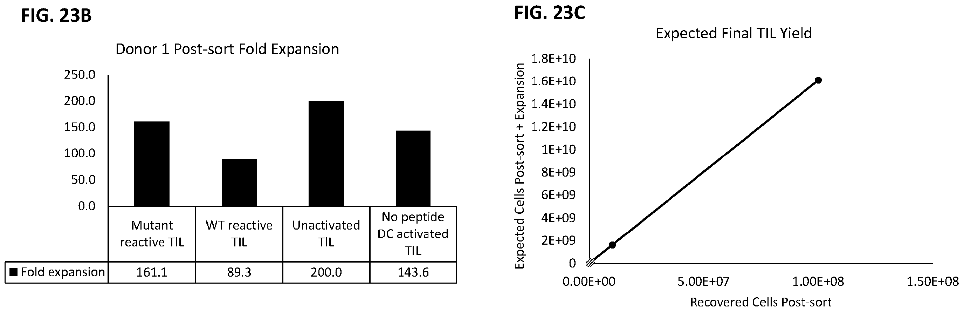

**FIG. 23A****-****FIG. 23C** depict expansion of tumor infiltrating T lymphocytes after sorting. **FIG. 23A** depicts total cell number and **FIG. 23B** depicts fold expansion, of Population 5 cells derived from Population 4 cells following co-culture with or without dendritic cells loaded with wild-type peptide, tumor associated peptide, or no peptide. Projected cell numbers after expansion of Population 4 cells into Population 5 cells at various cell recovery numbers post-sort are shown in **FIG. 23C****.**

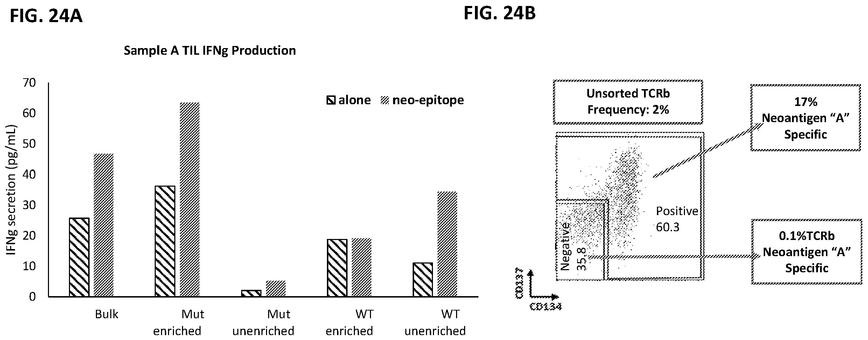

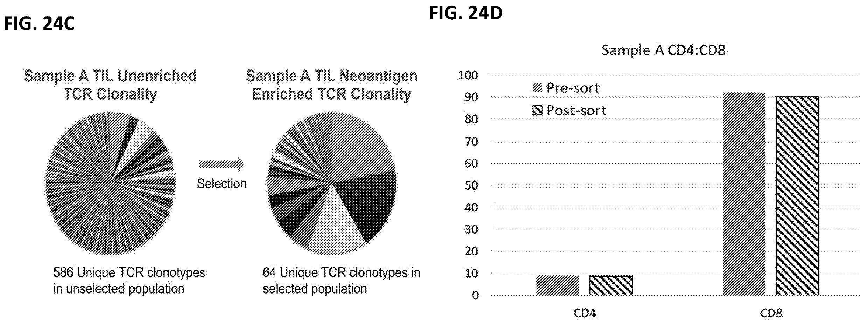

**FIG. 24A** depicts measured IFN-gamma secretion within a bulk co-culture, positive sorted (selected) population by expression of CD137 and/or CD134 from bulk co-culture cells (enriched), or negative sorted (unselected) population form bulk co-culture cells, following stimulation with mutant (mut) peptide or normal, wild-type (WT) peptide from an ovarian cancer patient. **FIG. 24B** depicts enrichment of neoantigen specific population of the tumor-reactive specific cells in the positive sort and negative sort compared to the bulk unsorted T cells. **FIG. 24C** depicts the number of TCR clonotypes present in the unselected and selected populations and demonstrates that the diversity of incoming TCRs is high in the unsorted T cell population and that there is enrichment of unique TCR clones in the selected population. **FIG. 24D** depicts the pre- and post-sort cell populations from Sample A which were observed to contain CD4+ and CD8+ cells, indicating that class I and class II reactive cells are present in the enriched population.

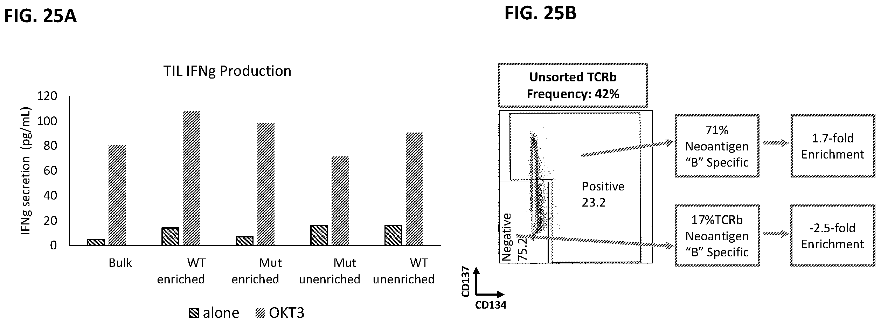

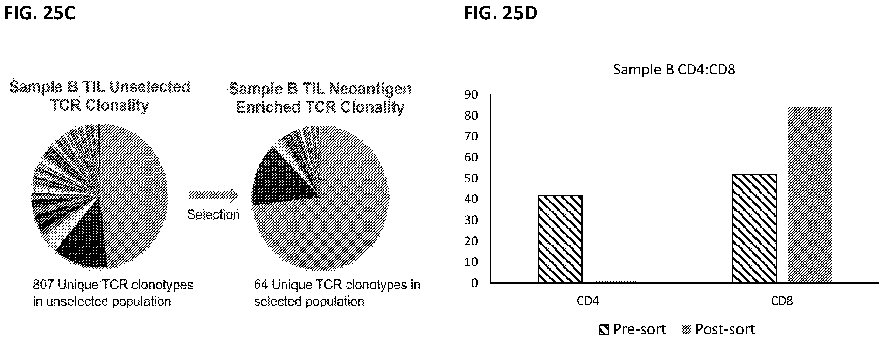

**FIG. 25A** depicts measured IFN-gamma secretion within a bulk co-culture, positive sorted (selected) population by expression of CD137 and/or CD134 from bulk co-culture cells (enriched), or negative sorted (unselected) population from bulk co-culture cells, following stimulation with anti-CD3 (OKT3) from colorectal cancer patient. **FIG. 25B** depicts enrichment of neoantigen specific population of the tumor-reactive specific cells in the positive sort and negative sort compared to the bulk unsorted T cells. **FIG. 25C** depicts the TCR clonality profile present in the unselected and selected populations. **FIG. 25D** depicts the pre- and post-sort cell populations which were observed to contain CD4+ and CD8+ cells, indicating that class I and class II reactive cells are present in the enriched population.

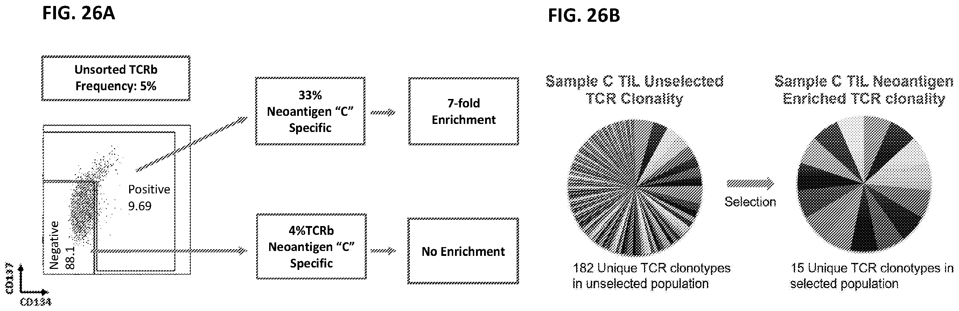

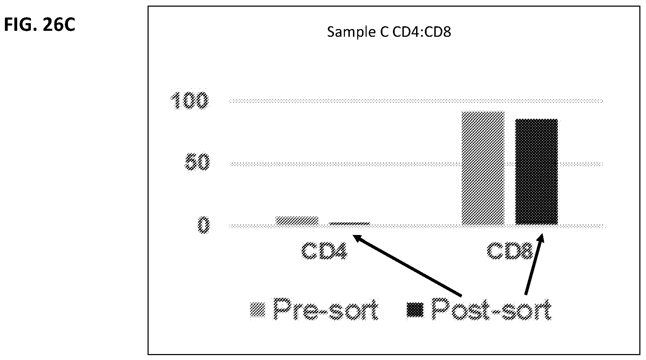

**FIG. 26A** depicts enrichment of neoantigen specific population of tumor-reactive specific cells in a bulk co-culture, positive sorted (selected) population by expression of CD137 and/or CD134 from bulk co-culture cells (enriched), or negative sorted (unselected) population fromm bulk co-culture cells. **FIG. 26B** depicts the TCR clonality profile present in the unselected and selected populations. **FIG. 26C** depicts Pre- (bulk) and post-sort cell populations, which were observed to contain both CD4+ class I reactive and CD8+ class II reactive cells.

### Detailed Description

The invention provides a method for manufacturing T cells for use in a therapeutic cell composition, wherein the T cells express a T cell receptor (TCR) that recognizes an antigen on the surface of a target cell, the method comprising: (1) processing a biological sample containing a population of T lymphocyte cells obtained from a donor subject hat has a tumor to produce a first population of T lymphocyte cells; (2) stimulating the first population with one or more first T-cell stimulating agents of lymphocytes under conditions for expansion of T cells in the population to produce a second population of activated T cells wherein the stimulation is performed in a closed system using serum free medium, wherein the one or more first T-cell stimulating agents is or comprises recombinant IL-2; (3) co-culturing the second population of T cells in the presence of antigen presenting cells that present one or more non-native peptide on a major histocompatibility complex (MHC) in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor; (4) selecting, from the third population of cells, the population of T cells containing endogenous TCR that are reactive to peptides present on the APCs based on one or more upregulation markers expressed on reactive or activated T cells in a closed system to produce a fourth population containing the selected T cells; (5) expanding the fourth population of selected cells in the presence of one or more second T-cell stimulating agents of lymphocytes under conditions to expand T cells in the population in a closed system using serum free medium to produce a composition of expanded T cells for use as a therapeutic cell composition, wherein the one or more second T-cell stimulating agents is or comprises recombinant IL-2.

In accordance with embodiments herein, methods or processes for manufacturing T cell preparations are provided which may be useful for treating patients with a pathological disease or condition. In contrast to known production methods, the methods and processes described herein can be completed in a significantly shorter time and recover a higher number of endogenous TCR-expressing T cells, thereby offering a significant advantage to bring cells into the clinic in therapeutic doses. Also provided herein are populations of T cells produced by methods described herein and pharmaceutical compositions thereof.

The provided methods relate to producing a T cell therapy reactive to tumor-associated antigens, such as neoantigens. Cancer cells accumulate lots of different DNA mutations as part of the tumorigenic process. These mutations can cause amino acid changes in protein coding regions. For a mutation to be recognized by the immune system the protein needs to be processed intracellularly and presented on the surface with the Major Histocompatibility Complex (MHC). Peptide neoantigens (also referred to herein as neoepitopes or peptide neoepitopes) are the mutant peptides presented by the MHC complex that can be recognized by a T-cell via TCR binding. In order for the immune system to recognize the mutation, it must be expressed on the surface of the cancer cell via the MHC complex and the T cell must have a TCR that recognizes the mutated peptide. These neoantigens may be presented by MHC class I and MHC class II, and are recognized by CD8+ and CD4+ T cells respectively.

The method described is used to manufacture T cells which express cell surface receptors, wherein the cell surface receptor is a T cell receptor (TCR) or novel group of TCRs. The population of T cells is or includes reactive T cells that express a T cell receptor (TCR) able to recognize peptide antigens on the surface of a target cells. Specifically, for an antigen to be recognized by the immune system the protein needs to be processed intracellularly to peptide fragments that are then presented on the surface with the Major Histocompatibility Complex (MHC). A TCR has two protein chains, which are designed to bind with specific peptides presented by a major histocompatibility complex (MHC) protein on the surface of certain cells. Since TCRs recognize peptides in the context of MHC molecules expressed on the surface of a target cell, TCRs have the potential to recognize antigens not only presented directly on the surface of target cells, e.g. cancer cells, but also presented by antigen-presenting cells, such as are present in tumor, inflammatory and infected microenvironments, and in secondary lymphoid organs. Reactive T cells expressing such cell surface receptors may be used to target and kill any target cell, including, but not limited to, infected cells, damaged cells, or dysfunctional cells. Thus, according to the embodiments described herein, the manufactured T cells expressing the cell surface receptor may be used to target and kill any target cell, including, but not limited to, infected cells, damaged cells, or dysfunctional cells. Examples of such target cells may include cancer cells, virally infected cells, bacterially infected cells, dysfunctionally activated inflammatory cells (e.g., inflammatory endothelial cells), and cells involved in dysfunctional immune reactions (e.g., cells involved in autoimmune diseases).

In some embodiments, a "T cell receptor" or "TCR" is a molecule that contains a variable α and β chains (also known as TCRα and TCRβ, respectively) or a variable γ and δ chains (also known as TCRγ and TCRδ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a T cells (or T lymphocytes) where it is generally responsible for recognizing antigens bound to major histocompatibility complex (MHC) molecules.

In some aspects, the reactive T cells are tumor-reactive T cells that recognize a cancer neoantigen. The majority of neoantigens arise from passenger mutations, meaning they do not infer any growth advantage to the cancer cell. A smaller number of mutations actively promote tumor growth, these are known as driver mutations. Passenger mutations are likely to give rise to neoantigens that are unique to each patient and may be present in a subset of all cancer cells. Driver mutations give rise to neoantigens that are likely to be present in all the tumor cells of an individual and potentially shared. In some embodiments of the provided method, the population of T cells contain tumor-reactive T cells that can recognize neoantigens containing passenger and/or driver mutations.

The provided methods can be used for the *ex vivo* production of a T cell therapy, including for the *ex vivo* expansion of autologous tumor-reactive T cells. In some aspects, neoantigens are ideal targets for immunotherapies because they represent disease-specific targets. For example, such antigens generally are not present in the body before the cancer developed and are truly cancer specific, not expressed on normal cells and are not subjected to off target immune toxicity. Thus, the unique repertoire of neoantigens specific to the patient can elicit a strong immune response specific to the cancer cells, avoiding normal cells. This is an advantage over other cell therapy targets that may not be disease-specific targets, since even low levels of target antigen on normal cells can lead to severe fatal autoimmune toxicity in the context of engineered therapies that target common antigens. For example an anti MAGE-A3-TCR program in melanoma patients was halted due to study related deaths attributed to cross reactivity with a similar target MAGE-A12, which is expressed at a low level in the brain. A significant challenge in cancer immunotherapy has been the identification of cancer targets.

Recent clinical studies have demonstrated that T cells isolated from surgically resected tumors possess TCRs that recognize neoantigens, and expanding these neoantigen reactive TIL populations and re-infusing them into the patient can in some cases result in a dramatic clinical benefit. This personalized therapy has generated remarkable clinical responses in certain patients with common epithelial tumors.

Existing methods for obtaining and generating tumor-reactive T cells are not entirely satisfactory. For example, direct isolation of tumor-reactive T cells from a subject without expansion is not feasible because therapeutically effective amounts of such cells cannot be obtained. As an alternative, attempts have been made to identify TCRs specific to a desired neoantigen for recombinant engineering of the TCR into T cells for use in adoptive cell therapy methods. Such approaches, however, produce only a single TCR against a specific neoantigen and thereby lack diversity to recognize a broader repertoire of multiple tumor-specific mutations. Other methods involve bulk expansion of T cells from a tumor source, which has the risk of expanding T cells that are not reactive to a tumor antigen and/or that may include a number of bystander cells that could exhibit inhibitory activity. For example, tumor regulatory T cells (Tregs) are a subpopulation of CD4⁺ T cells, which specialize in suppressing immune responses and could limit reactivity of a T cell product. These further approaches that have sought to expand tumor-reactive T cells *ex vivo* are not selective such that non-reactive T cells in the culture may preferentially expand over reactive T cells resulting in a final product that lacks satisfactory reactivity and/or in which the number of tumor-reactive T cells remains insufficient. Methods to produce tumor-reactive T cells for therapy are needed.

The provided embodiments relate to improved methods for identifying and expanding T cells *ex vivo,* including tumor-reactive T cells, for use in T cell therapy. In some embodiments, the provided methods improve or increase the growth and survival of T cells, such as tumor-reactive T cells, outside of the body. In particular embodiments, the methods enrich for expansion of reactive T cells compared to non-reactive T cells and promote their survival and growth in culture *ex vivo.* In some embodiments, the resulting methods can be carried out in a closed system. The methods in some embodiments are carried out in an automated or partially automated fashion.

The provided methods result in an enriched population of T cells reactive to patient specific mutations, such as based on selection of upregulation markers after presentation of mutant antigens and/or based on expansion of T cells enriched for tumor-reactive T cells following co-culture with antigen presenting cells presenting peptide neoepitopes. For example, the methods of culturing the cells include methods to proliferate and expand cells, particularly involving steps to enrich for proliferation and expansion of tumor-reactive T cells such as by selection of such cells, or based on upregulation of a cell surface molecule on activated T cells (T cell activation marker) that are associated with or indicative of tumor-reactive T cells. The provided methods results in a product containing tumor reactive T cells that can target many mutations and/or that contains hundreds of TCRs that are reactive to different tumor antigens. Thus, such tumor reactive T cells offer advantages compared to existing methods in which cells are transduced to express a single neoepitope reactive TCR.

In some embodiments of the provided methods a source of potential tumor peptides is used to identify TCRs that are reactive to neoantigens in a process that includes expansion of the T cells reactive to the tumor neoantigenic peptides. Provided methods include *ex vivo* co-culture methods in which a population of T cells that have been expanded from T cells present in or from a biological sample (e.g. tumor fragments or peripheral blood or other source of T cells) is incubated in the presence of antigen-presenting cells that have been contacted with, or made to present, the neoantigenic peptides. In particular aspects, the T cells and antigen-presenting cells are autologous to the tumor-bearing subject from which the peptides were identified. The provided methods further include steps to separate, enrich for and/or select for tumor-reactive T cells from the co-culture prior to or in connection with their further *ex vivo* expansion.

FIG. 1A depicts a schematic of an exemplary process for manufacturing a T cell therapeutic composition in accord with the provided methods. In the exemplary process a tumor sample is obtained from a patient for identification and generation of peptides for use in co-culturing methods with antigen presenting cells (APCs) presenting the peptides and autologous antigen T cells obtained from the same subject. In some cases, a population of T cells from the patient, e.g. containing tumor infiltrating lymphocytes (TIL) or peripheral blood lymphocytes (PBL), is stimulated under conditions to expand the cells, prior to co-culture with antigen presenting cells that have been contacted or exposed to peptide neoepitopes for presentation on a major histocompability complex. Following co-culture under conditions in which the antigen presenting cells present peptides in the context of a major histocompatibility complex, tumor-reactive T cells or T cells surface positive for one or more T cell activation marker (also called an upregulation marker or reactive T cell marker, e.g. CD70a) associated with tumor reactive T cells can be selected and cultured under conditions for expansion in accord with the provided methods, such as incubation with a T cell stimulatory agent(s) (e.g. recombinant IL-2, anti-CD3 and/or anti-CD28). The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion. FIG. 1B depicts an exemplary process in which a cryopreservation step can be carried out after one or more of the steps.

The provided methods offer advantages compared to existing methods for producing and expanding TILs because the provided methods involve steps to enrich for tumor reactive cells, such as by the co-culturing step with peptide-presenting APCs followed by selection of reactive T cell clones that have upregulated one or more T cell activation marker. By virtue of this process, the initial small population of tumor reactive T cells expanded from the biological sample (e.g. tumor) are enriched for cells that are or likely to be tumor reactive cells before a subsequent second expansion step, thereby promoting preservation and expansion of cells of interest and limiting expansion of bystander T cells that are not reactive to a tumor antigen and/or that may include cells that exhibit inhibitory activity **(****FIG. 2A****).** This is in contrast to existing methods that involve passive expansion of bulk T cells in which all T cells from a tumor are subjected to a first initial expansion, e.g. with high IL-2 concentrations, followed by a second rapid expansion of T cells present after the initial expansion. In such other methods, while total viable cells (TVC) can be greatly expanded by these alternative processes, there is no step of actively ensuring that tumor reactive T cells are predominantly propagated **(****FIG. 2B****).** Further, the provided methods are carried out to maximize the numbers of tumor reactive cells that may be collected, for example by co-culturing all of the cells propagated after the first expansion with peptide-presenting APCs, and then by selecting from among all of the bulk cells after the co-culturing for cells positive for the one or more activation markers before the subsequent second expansion. In aspects of the provided methods, all steps of the method are carried out in a closed system.

The provided methods include one or more features that provide for or relate to an improved, more efficient and/or more robust process for producing a tumor-reactive T cell therapeutic composition ex vivo. In particular, the disclosure relates to methods that provide advantages over available methods for producing a TIL therapeutic cell composition. Such advantages include, for example, reduced cost, streamlining, improved enrichment of tumor-reactive T cells in the therapeutic composition, and increased efficacy of the therapeutic composition, including among different subjects and tumor conditions.

Among the findings herein is the observation that lower concentrations of recombinant IL-2 can be employed during one or both expansion steps with success. Many existing methods use high concentrations of IL-2 of 6000 lU/mL for T cell expansion of TIL. However, high IL-2 concentrations can increase the cost of the process and may be limiting. In some cases, high IL-2 concentrations may lead to negative impacts on T cell differentiation by driving effector T cell differentiation over early memory T cells that may be more desirable in a therapeutic T cell composition. The provided methods can be carried out with concentrations that are several-fold lower than 6000 IU/mL, such as concentrations less than at or about 1000 IU/mL, for example from at or at about 300 IU/mL to at or about 1000 IU/mL. In particular embodiments, the concentration of IL-2 is at or about 300 IU/mL.

The population of T cells is obtained from a biological sample known to contain T cells. In some embodiments, the population of T cells is enriched from a biological sample from a subject, in particular a human subject. The biological sample can be any sample containing a bulk population of T cells. In some embodiments, the biological sample is or includes peripheral blood mononuclear cells. In some embodiments, the biological sample is a peripheral blood or serum sample. In some embodiments, the biological sample is a lymph node sample. In some embodiments, the biological sample is a tumor sample. In some aspects, the bulk T cells can include tumor-infiltrating T cells (TILs). In some embodiments, the subject is a human subject. In some embodiments the subject is a subject having a cancer, viral infection, bacterial infection, or is a subject with an inflammatory condition. The subject has a cancer.

In aspects of the provided methods, the starting source of cells in the method can be tumor fragments (e.g. 1-8 mm diameter fragments) or can be a single cell suspension preparation from enzymatic digestion of tumor fragments. It is found herein that, while certain sources may be superior for some tumor types, both fragments and single cell suspensions can support T cell expansion and enrichment of tumor-reactive T cells. In some cases, the tumor cell source can be chosen depending on the tumor type or cancer, such as to optimize or increase expansion and enrichment of tumor-reactive T cells from the tumor. In one example, the cancer is a melanoma and the starting population of lymphocytes are tumor fragments, such as from a resected tumor. In another example, the cancer is a colorectal cancer and the starting population of lymphocytes is a single cell suspension obtained by enzymatic digestion, e.g. collagenase, of tumor fragments.

In some embodiments, the methods include a step of co-culturing initially expanded T cells with autologous antigen presenting cells that have been loaded with peptide. Findings herein demonstrate that relatively low concentrations of peptide or a peptide pool (containing a plurality of peptides, e.g. 2, 3, 4, 5, 6, 7, 8, 9, 10, 20, 30, 40, 50, 60, 70, 80, 90 or 100 more, or any value between any of the foregoing), such where each individual peptide is less than 20 ng/mL, and even as low as 0.1 ng/mL, can lead to an increase in activation of T cell during the culture. In some embodiments, this can lead to an improved enrichment of tumor-reactive T cells in the co-culture prior to selection of cells positive for one or more T cell activation marker (i.e. upregulation marker or reactive T cell marker). In some embodiments, the co-culturing step in the provided methods include a ratio of tumor-derived cells containing T cells to autologous APCs (e.g. dendritic cells) of at or about 1:5 to at or about 5:1, such as 1:3 to at or about 3:1, for example as at or about 1:1, and involves loading the APCs with an individual peptide or a pool of peptides. In some embodiments, the APCs are loaded with a concentration of peptide or peptide pool in which the individual peptide, or individual peptides of the pool of peptides on average, is less than at or about 20 ng/mL, such as from at or about 0.1 ng/mL to at or about 1 ng/mL, for example at or about 0.1 ng/mL.

The provided methods include enriching T cells, such as CD3+ T cells or a CD4 and/or CD8 subset thereof, further based on one or more marker whose expression is upregulated on (e.g. compared to resting or non-activated T cells) or specific to reactive or activated T cells (hereinafter "reactive T cell marker" or T cell activation marker). Reactive T cells will express certain reactive markers when their endogenous TCR recognizes an antigen on a target cell or tissue, such as when a TCR recognizes a neoantigen on the tumor. Exemplary reactive T cell markers include one or more, such as two, three, four or more of, CD107, CD107a, CD39, CD103, CD137 (4-1BB), CD59, CD90, CD38, CD30, CD154, CD252, CD134 (OX40), CD258, CD256, PD-1, TIM-3 or LAG-3. The enrichment or selection for cells positive for one or more such upregulation marker on reactive or activated T cells can be carried out prior to or during one or more steps of the expansion method. In particular embodiments, the provided methods include enrichment or selection for cells positive for one or more upregulation marker on reactive or activated T cells after activation of a population of T cells by the co-culture incubation with peptide-presenting APCs (e.g. DCs). In some embodiments, the step of selecting cells positive for one or more upregulation marker on reactive or activated T cells from the co-culture can result in 2-fold or greater enrichment of antigen-specific tumor-reactive T cells and/or a substantial decrease in TCR clonality evidencing enrichment of TCR clonotypes consistent with enrichment of tumor-reactive T cells. Furthermore, such enriched T cells can exhibit an improved ability to produce IFN-gamma following antigen-specific stimulation compared to non-selected T cells or bulk T cells from the co-culture.

In some embodiments, the methods produce or expand T cells for use in adoptive cell therapy for treating a disease or condition in which cells or tissue associated with the disease or condition is known or suspected of expressing an antigen target recognized by the T cells. In some embodiments, the T cell therapy is autologous to the subject. In some embodiments, the T cell therapy is allogeneic to the subject.

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

If a definition set forth herein is contrary to or otherwise inconsistent with a definition set forth in the patents, applications, published applications and other publications that are referenced, the definition set forth herein prevails over the definition that is in the reference.

The section headings used herein are for organizational purposes only and are not to be construed as limiting the subject matter described.

### I. EX VIVO EXPANSION OF TUMOR-REACTIVE T CELLS

The provided methods involve the *ex vivo* expansion and production of a T cell therapeutic composition, particularly for use in connection with treating cancer. In some embodiments, the method of manufacturing involves the growth and manipulation of patient cells outside of the body.The methods relate to methods for expanding T cells containing an endogenous TCR specific to a tumor-associated antigen (hereinafter "tumor reactive T cells"). For purposes of this disclosure, reference to tumor reactive T cells includes T cells that exhibit reactivity to a tumor antigen or that are likely or suspected of being tumor reactive T cells due to upregulation or positive surface expression of a protein expressed on the T cell that are only expressed when the T cells endogenous TCR recognizes a peptide expressed by the APCs, e.g. T cell activation marker. In some aspects, the frequency of these cells can be low and in order to expand these cells to a therapeutic dose *ex vivo* methods for enrichment and expansion are necessary.

A population containing T cells (hereinafter also called first population of T cells) is a population of T cells that is obtained, selected or isolated from a biological sample from a subject, such as a human subject, in which the biological sample contains T cells. In some embodiments, the population containing T cells can be from any biological source sample that is known or suspected of containing T cells that are or that may include or potentially could include tumor reactive T cells. The sample can include a tumor sample containing tumor infiltrating lymphocytes (TILs), a blood sample (e.g. apheresis or leukapheresis sample) containing peripheral blood mononuclear cells (PBMCs) or a lymph node sample. In some embodiments, the sample is a tumor sample or a tumor fragment containing tumor infiltrating lymphocytes or TILs. The population containing T cells can be directly obtained from a subject (e.g. healthy or cancer subject), such as by selection of T cells or a subset thereof from the biological sample from the subject. The biological sample is from a subject that has a tumor and in particular embodiments contains tumor reactive T cells or that has the potential to or that may contain tumor reactive T cells that can be enriched by the provided methods.

The invention provides a method for manufacturing T cells for use in a therapeutic cell composition, wherein the T cells express a T cell receptor (TCR) that recognizes an antigen on the surface of a target cell, the method comprising: (1) processing a biological sample containing a population of T lymphocyte cells obtained from a donor subject to produce a first population of T lymphocyte cells; (2) stimulating the first population with one or more first T-cell stimulating agents of lymphocytes under conditions for expansion of T cells in the population to produce a second population of activated T cells wherein the stimulation is performed in a closed system using serum free medium, wherein the one or more first T-cell stimulating agents is or comprises recombinant IL-2; (3) co-culturing the second population of T cells in the presence of antigen presenting cells that present one or more non-native peptide (e.g. peptide neoepitopes) on a major histocompatibility complex (MHC) (MHC-associated non-native peptide), in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor; (4) selecting, from the third population, the population of T cells containing endogenous TCR that are reactive to peptides present on the APCs based on one or more upregulation markers expressed on reactive or activated T cells in a closed system to produce a fourth population containing the selected T cells; and (5) expanding the fourth population of selected cells in the presence of one or more second T-cell stimulating agents of lymphocytes under conditions to expand T cells in the population in a closed system using serum free medium to produce a composition of expanded T cells for use as a therapeutic cell composition, wherein the one or more second T-cell stimulating agents is or comprises recombinant IL-2. In some embodiments, the selecting of the T cells in (4) can involve depleting or removing the APCs and/or can include selection of T cells based on upregulation markers on reactive or activated T cells. One or more of the steps are carried out in a closed system using serum free medium..

In some embodiments, the T-cell stimulating agents additionally include anti-CD3 (e.g. anti-CD3 antibody, such as OKT3), anti-CD28 reagents (e.g. anti-CD28 antibody), such as an anti-CD3 antibody (e.g. OKT3) and an anti-CD28 antibody and/or one or more recombinant cytokine (e.g. IL-7, IL-21 and/or IL-15) . In particular embodiments, the incubation or culture of T cells also is carried out with nutrient containing media so that the cells can survive outside of the body.

The methods include incubation with recombinant IL-2, optionally alone or in combination with one or more other recombinant cytokines (e.g. IL-7, IL-21 and/or IL-15) and, in some cases, one or more other T cell stimulating agents to provide a primary and secondary (costimulatory) signal to the cells. Standard methods for culturing T cells to provide a primary and secondary (costimulatory) signal to the cells, involve incubation with T cell stimulating agents provided by anti-CD3 (e.g. OKT3) and anti-CD28 reagents. In some embodiments, the T cell stimulatory agents additionally include an anti-CD3 antibody (e.g. OKT3) and an anti-CD28 antibody. Typically such stimulations also include one or more additional recombinant cytokine (e.g. IL-7, IL-21 and/or IL-15) and nutrient containing media so that the cells can survive outside of the body.

Provided methods for expansion of tumor-reactive T cells involve a first expansion involving culturing the selected or isolated population containing T cells (i.e. the first population of T cells) with a recombinant cytokine from one or more of (e.g. IL-7, IL-21 and/or IL-15, andincluding recombinant IL-2. In some cases, the T cell stimulatory agent(s) further provide a primary and secondary (costimulatory) signal to the cells, such as provided by anti-CD3 (e.g. OKT3) and anti-CD28 reagents, such as an anti-CD3 antibody (e.g. OKT3) and an anti-CD28 antibody. The initial or first expansion results in a second population of T cells that is enriched for T cells as a result of expansion or proliferation of T cells present in the first population.

In the provided methods, tumor reactive T cells are identified or enriched from the stimulated T cells expanded in the first step by one or more further steps that include *ex vivo* co-culture of the stimulated T cells (second population of T cells) with antigen presenting cells (APCs) and one or a plurality of peptides that include neoepitopes of a tumor antigen (APCs/peptide neoepitopes). Provided methods include *ex vivo* co-culture in which the second population of T cells are incubated with APCs, such as autologous APCs or artificial antigen presenting cells (aAPCs), that have been exposed to or contacted with one or more peptides, e.g. synthetic peptides, under conditions in which the APCs have been induced to present one or more peptides from a tumor-associated antigen. In some embodiments, the population of T cells are autologous T cells from a subject with a tumor and the source of synthetic peptides are tumor antigenic peptides from a tumor antigen of the same subject. In some embodiments, cells from the *ex vivo* co-culture are a population of cells (third population) that include tumor reactive T cells that recognize or are activated by a peptide presented on an MHC of an APC in the culture. In some embodiments, cells from the *ex vivo* co-culture represent a source of cells that are enriched for tumor reactive T cells.

The tumor reactive T cells are be further enriched by separation or selection of cells that express one or more upregulation marker, such as an activation marker, associated with tumor-reactive T cells (the further separation or selection producing a fourth population of T cells of the enriched tumor reactive T cells). The T cell activation markers can include cell surface markers whose expression is upregulated or specific to T cells that have been exposed to antigen and activated. Exemplary of such markers are described below.

In particular embodiments, the provided methods include enriching from a biological sample (directly sourced from a sample in vivo or from an ex vivo coculture with antigen presenting cells (APCs)) T cells that have an endogenous TCR that recognize tumor-associated antigens, e.g. neoantigens, such as by selecting for T cells that are surface positive for one or more T cell activation marker (e.g. CD107, CD107a, CD039, CD134, CD137, CD59, CD69, CD90, CD38, or CD103).

In particular embodiments, a second expansion is performed on T cells enriched or isolated from the co-culture, such as after separation or selection of tumor reactive T cells or T cells that are surface positive for one or more T cell activation markers associated with tumor reactive T cells. The second expansion involves incubation to further stimulate T cells with a T cell stimulatory agent(s), wherein the T cell stimulatory agent is or comprises IL-2, and the T cell stimulatory agent optionally comprises further T cell stimulatory agent(s) such as anti-CD3 antibody (e.g. OKT3), anti-CD28 antibody, and additional recombinant cytokine(s) (e.g. IL-7, IL-21 and/or IL-15). The T cells, such as tumor reactive T cells or T cells that are surface positive for one or more T cell activation markers associated with tumor reactive T cells, are allowed to expand for a certain number of days as desired and/or until a therapeutic dose or harvest dose is met. The composition of expanded T cells can then be harvested and formulated for administration to a subject for treatment of a cancer in the subject.

T cells containing an endogenous TCR are enriched by separating the antigen presenting cells from the population of T cells. Such cells are enriched by selecting T cells that are surface positive for one or more activation markers associated with tumor-reactive T cells. A second expansion is performed on T cells enriched or isolated from the co-culture, such as after separation or selection of tumor reactive T cells or T cells that are surface positive for one or more T cell activation markers associated with tumor reactive T cells. The second expansion involves incubation to further stimulate T cells with a T cell stimulatory agent(s), wherein the T cell stimulatory agent is or comprises IL-2, and optionally further comprises T cell stimulatory agents such as anti-CD3 antibody (e.g. OKT3), anti-CD28 antibody and recombinant cytokine(s) (e.g. IL-7, IL-21 and/or IL-15).

One or more of the steps can be carried out in serum-free media. In one embodiment, the serum free medium is OpTmizer CTS (LifeTech), Immunocult XF (Stemcell technologies), CellGro (CellGenix), TexMacs
(Miltenyi), Stemline (Sigma), Xvivo15 (Lonza), PrimeXV (Irvine Scientific), or Stem XVivo (RandD systems). The serum-free medium can be supplemented with a serum substitute such as ICSR (immune cell serum replacement) from LifeTech. The level of serum substitute (e.g., ICSR) can be, e.g., up to 5%, e.g., about 1%, 2%, 3%, 4%, or 5%. In some embodiments, the serum-free media contains 0.5 mM to 5 mM of a dipeptide form of L-glutamine, such L-alanyl-L-glutamine (Glutamax^{™}). In some embodiments, the concentration of the dipeptide form of L-glutamine, such as L-alanyl-L-glutamine, is from or from about 0.5 mM to 5 mM, 0.5 mM to 4 mM, 0.5 mM to 3 mM, 0.5 mM to 2 mM, 0.5 mM to 1 mM, 1 mM to 5 mM, 1 mM to 4 mM, 1 mM to 3 mM, 1 mM to 2 mM, 2 mM to 5 mM, 2 mM to 4 mM, 2 mM to 3 mM, 3 mM to 5 mM, 3 mM to 4 mM or 4 mM to 5 mM, each inclusive. In some embodiments, the concentration of the dipeptide form of L-glutamine, such as L-alanyl-L-glutamine, is or is about 2 mM.

In connection with the provided methods, the methods result in enrichment of T cells containing an endogenous TCR specific to a tumor-associated antigen to maximize expansion of desired therapeutic cells. The T cells, such as tumor reactive T cells or T cells that are surface positive for one or more T cell upregulation markers or activation markers associated with tumor reactive T cells, are allowed to expand for a certain number of days as desired and/or until a therapeutic dose or harvest dose is met. The composition of expanded T cells can then be harvested and formulated for administration to a subject for treatment of a cancer in the subject.

### A. Neoepitope identification and peptide generation

The provided methods include a step of generating or identifying *in silico* a plurality of peptides (also referred to as "P" or "n-mers") that contain at least one cancer-specific cancer neoepitope, and a further step of filtering in silico the peptides to so obtain a subset of neoepitope sequences. In some embodiments, at least one synthetic peptide is prepared using sequence information from the subset of neoepitope sequences, and the synthetic peptide is then employed in methods to enrich for tumor-reactive T cells in accord with the provided methods.

In some embodiments, the cancer-specific cancer neoepitope is determined by identifying or isolating a tumor-associated antigen or peptide sequence thereof from a cancer cell from a subject. The cancer cell may be obtained from any bodily sample derived from a patient which contains or is expected to contain tumor or cancer cells. The bodily sample may be any tissue sample such as blood, a tissue sample obtained from the primary tumor or from tumor metastases, a lymph node sample or any other sample containing tumor or cancer cells.

In some embodiments, the tumor is a hematological tumor. Non- limiting examples of hematological tumors include leukemias, including acute leukemias (such as l lq23- positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some embodiments, the tumor is a solid tumor. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some embodiments, the cancer is a gastrointestinal cancer involving a cancer of the gastrointestinal tract (GI tract), including cancers or the upper or lower digestive tract, or an accessory organ of digestion, such as esophagus, stomach, biliary system, pancreas, small intestine, large intestine, rectum or anus. In some embodiments, the cancer is an espphageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular embodiments, the cancer is a colorectal cancer.

In some embodiments, the tumor is from a breast cancer, such as a ductal carcinoma or a lobular carcinoma. In some embodiments, the tumor is from a prostate cancer. In some embodiments, tumor is from a skin cancer, such as a basal cell carcinoma, a squamous cell carcinoma, a Kaposi's sarcoma, or a melanoma. In some embodiments, the tumor is from a lung cancer, such as an adenocarcinoma, a bronchiolaveolar carcinoma, a large cell carcinoma, or a small cell carcinoma. In some embodiments, the tumor is from a brain cancer, such as a glioblastoma or a meningioma. In some embodiments, the tumor is from a gastrointestinal cancer, such as any described above. In some embodiments, the tumor is from a colon cancer. In some embodiments, the tumor is from a liver cancer, such as a hepatocellular carcinoma. In some embodiments, the tumor is from a pancreatic cancer. In some embodiments, the tumor is from a kidney cancer, such as a renal cell carcinoma. In some embodiments, the tumor is from a testicular cancer.

In some embodiments, the cancer is not a melanoma. Melanoma is a cancer that generally has a high mutational rate. High tumor mutation burden has been thought to be a particularly desired prognostic marker for success related to treatment with an immunotherapy targeting tumor neoantigens (Simpson et al., Journal of Clinical Oncology 2017, 35:15_suppl, 9567-9567; McGranahan et al. Science 2016, 351:1463-1469) In some embodiments, the provided methods can be used in cancers that have a lower tumor mutation burden, since the methods are carried out to actively (as opposed to passively) enrich for tumor reactive T cells.

In some embodiments, the subject is a subject with a tumor mutational burden (TMB) of less than 8 mutations. TMB includes the number of non-synomymous mutations per tumor. In some embodiments, TMB can be calculated by counting the number of synonymous and non-synonymous mutations across a 0.8- to 1.2-megabase (Mb) region, and reporting the result as mutations/Mb. In some embodiments, TMB can be determined by next generation sequencing (NGS) on tumor tissue samples. In some cases, whole exome sequencing can be used or computational germline status filtering can be used (Chalmers et al. Genome Med 2017 9:34). In some embodiments, the subject has a TMB of less than at or about 60 mutations/Mb, such as less than at or about 55 mutations/Mb, less than at or about 50 mutations/Mb, less than at or about 45 mutations/Mb, less than at or about 40 mutations/Mb, less then at or about 30 mutations/Mb, less than at or about 25 mutations per Mb, or less than at or about 20 mutations/Mb, or any value between any of the foregoing. In some embodiments, the subject has a TMB of less than at or about 41 mutations/Mb, less than at or about 40 mutations/Mb, less than at or about 39 mutations/Mb, less than at or about 38 mutations/Mb, less than at or about 37 mutations/Mb or less.

In some embodiments, the peptide (P) is a tumor-associated antigen derived from premalignant conditions, such as variants of carcinoma *in situ,* or vulvar intraepithelial neoplasia, cervical intraepithelial neoplasia, or vaginal intraepithelial neoplasia.

In some aspects, nucleic acid from such cells of the tumor or cancer is obtained and sequenced. In embodiments, the protein-coding region of genes in a genome is obtained, such as by omics analysis, such as by analysis of whole genomic sequencing data, exome sequencing data, and/or transcriptome data. To identify tumor-specific sequences, sequencing data can be compared to a reference sequencing data, such as data obtained from a normal cell or noncancerous cell from the same subject. In some embodiments, next-generation sequencing (NGS) methods are used.

In some embodiments, the methods include a step of using matched normal omics data of a tumor. In such methods, the *in silico* analysis involves an omics analysis to identify mutations in the tumor relative to normal tissue of the same patient, such as non-diseased tissue of the same patient. It is generally contemplated that matched normal omics data are whole genomic sequencing data, exome sequencing data, and/or transcriptome data, and that the matched normal omics data are matched against normal before treatment of the patient. In a particular embodiment, whole exome sequencing is performed on healthy and diseased tissue to identify somatic mutations associated with the tumor.

In some embodiments, omics data are obtained from one or more patient biopsy samples following standard tissue processing protocol and sequencing protocols. In particular embodiments, the data are patient matched tumor data (e.g., tumor versus same patient normal). In some cases, non- matched or matched versus other reference (e.g., prior same patient normal or prior same patient tumor, or homo statisticus) are also deemed suitable for use herein. The omics data may be fresh omics data or omics data that were obtained from a prior procedure (or even different patient). For example, neoepitopes may be identified from a patient tumor in a first step by whole genome and/or exome analysis of a tumor biopsy (or lymph biopsy or biopsy of a metastatic site) and matched normal tissue (i.e., non-diseased tissue from the same patient such as peripheral blood). In some embodiments, genomic analysis can be processed via location-guided synchronous comparison of the so obtained omics information.

The genomic analysis can be performed by any number of analytic methods. In particular embodiments, the methods include WGS (whole genome sequencing) and exome sequencing of both tumor and matched normal sample using next generation sequencing such as massively parallel sequencing methods, ion torrent sequencing, pyrosequencing. Computational analysis of the sequence data may be performed in numerous manners. In some embodiments, the data format is in SAM, BAM, GAR, or VCF format. As an example, analysis can be performed *in silico* by location-guided synchronous alignment of tumor and normal samples as, for example, disclosed in US 2012/0059670A1 and US 2012/0066001 A1 using BAM files and BAM servers. Alternative file formats for sequence analysis (e.g., SAM, GAR, FASTA, etc.) are also contemplated.

In some of any embodiments, peptides (P) comprising neoantigens arising from a missense mutation encompass the amino acid change encoded by 1 or more nucleotide polymorphisms. Peptides (P) comprising neoantigens that arise from frameshift mutations, splice site variants, insertions, inversions and deletions should encompass the novel peptide sequences and junctions of novel peptide sequences. Peptides (P) comprising neoantigens with novel post-translational modifications should encompass the amino acids bearing the post-translational modification(s), such as a phosphate or glycan.

Once these mutations are identified, neoepitopes are then identified. Neoepitopes are mutant peptides that are recognized by a patient's T cells. These neoepitopes must be presented by a tumor or antigen presenting cell by the MHC complex and then be recognized by a TCR on the T cell. In some embodiments, the provided methods include a step of calculation of one or more neoepitopes to define neoepitopes that are specific to the tumor and patient. Consequently, it should be recognized that patient and cancer specific neoepitopes can be identified from omics information in an exclusively *in silico* environment that ultimately predicts potential epitopes that are unique to the patient and tumor type. In particular aspects, the so identified cancer neoepitopes are unique to the patient and the particular cancer in the patient (e.g., having a frequency of less than 0.1% of all neoepitopes, and more typically less than 0.01% in a population of cancer patients diagnosed with the same cancer), but that the so identified cancer neoepitopes have a high likelihood of being presented in a tumor.

In some of any embodiments, the length of the peptide (P) depends on the specific application and is typically between about 5 to about 50 amino acids. In preferred embodiments, the peptide (P) is between about 7 to 35 amino acids, e.g., 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids. In some aspects, the methods can be carried out with an individual peptide that includes a change(s) (e.g. mutations) in the amino acid sequences. In some aspects, the methods can be carried out with a pool of peptides, where peptides of the pool contain a change(s) (e.g. mutations) in the amino acid sequences. The pool of peptides can include tens to hundreds of individual peptides. In some cases, the pool of peptides includes 2, 3, 4, 5, 6, 7, 8, 9, 10, 11, 12, 13, 14, 15, 16, 17, 18, 19, 20, 30, 40, 50, 60, 70, 80, 90, 100 or more individual peptides, or any value between any of the foregoing. The pool of peptides can represent one neo-antigen or can represent several neo-antigens. In some cases, a pool of peptides can include multiple overlapping peptides of the same neo-antigen. Thus, for a tumor-associated antigen, the antigen may be divided into 7 to 35 amino acid, e.g., 25 amino acid, peptides (P) wherein each peptide (P) contains a unique composition of amino acids; or, the peptides (P) can be overlapping peptide pools wherein an antigen is divided into a set number of 7 to 35 amino acid, e.g., 25 amino acid, peptides (P) that have overlapping sequences. In some cases, each of the peptides of the overlapping pool of an antigen can be offset by a set number of amino acid residues, such as 5, 6, 7, 8, 9, 10, 11, 12, 13, 15 or 15 amino acids. In some embodiments, each of the peptides of the overlapping pool of an antigen is offset by 10 amino acids. In some embodiments, each of the peptides of the overlapping pool of an antigen is offset by 12 amino acids. For example, an overlapping peptide pool comprising a 100 amino acid antigen may be divided into eight 25 amino acid peptides (P) that are each offset by 12 amino acids (i.e., each subsequent 25 amino acid peptide comprising a 100 amino acid peptide sequence starts at the 13^{th} amino acid position from the prior peptide). Those skilled in the art understand that many permutations exist for generating a peptide pool from an antigen.

The neoepitope sequences as contemplated herein can be defined as sequence stretches with relatively short length (e.g., 5-30 mers, more typically 7- 11 mers, or 12-25 mers) wherein such stretches include the change(s) (e.g. mutations) in the amino acid sequences. Most typically, the change(s) is/are located centrally or near the center (e.g., less than 4, or less than 5, or less than 6 amino acids from center position). In particular aspects, neoepitope sequences contemplated herein will especially include those in which a single amino acid is exchanged relative to the matched normal sequence, and in which the position of the changed amino acid is centrally located, or near the center of the neoepitope sequence (e.g., in a 9-mer, the changed amino acid is at position 2, 3, 4, or 5, and more typically at position 3, 4, or 5, and most typically at position 4 or 5). It should be appreciated that a single amino acid change may be presented in numerous neoepitope sequences that include the changed amino acid, depending on the position of the changed amino acid.

In particular embodiments, neoepitopes will be calculated to have a length of between 2-50 amino acids, more typically between 5-30 amino acids, and most typically between 9-15 amino acids. For example, where the epitope is to be presented by the MHC-I complex, a typical epitope length will be about 8- 11 amino acids, while the typical epitope for presentation via MHC-II complex will have a length of about 13- 17 amino acids. As will be readily appreciated, since the position of the changed amino acid in the neoepitope may be other than central, the actual peptide sequence and with that the actual topology of the neoepitope may vary considerably. Moreover, where the neoepitope is presented to an immune competent (or other) cell as a synthetic peptide, it should be appreciated that the synthetic peptide may be significantly longer than the peptide portion that is ultimately bound by the MHC-I or MHC-II system to so allow for proteolytic processing in the cell. For example, contemplated synthetic peptides may therefore have between 8 and 15 amino acids upstream and downstream of the changed amino acid.

Various algorithms have been developed and can be used to map T cell epitopes (both MHC Class I and Class II-restricted) within protein molecules of various origins. In some embodiments, many programs utilize availability of the large-scale peptide-MHC binding affinity matrix from experimental measurements to train machine learning (ML)-based classifiers to distinguish MHC-binders from non-binders (see e.g., Zhao et al. (2018) PLoS Comput Biol 14(11): e1006457). Exemplary predictor methods for MHC class I (e.g. 9-mer) include *smm,* smmpmbec, ann (NetMHC3.4), NetMHC4, PickPocket, consensus, NetMHCpan2.8, NetMHCpan3, NetMHCpan4, NetMHCcons, mhcflurry, mhcflurry_pan, or MixMHCpred. Exemplary predictor methods for MHC class II (e.g. 15-mer) include NetMHCIIpan, NetMHCII2.3, nn_align, smm_align, consensus, comblib, tepitope, or mhcflurry. Any of such methods can be used.

In embodiments where the synthetic peptide is used for direct MHC-I binding, the overall length will be between 8 and 10 amino acids. In embodiments, where the synthetic peptide is used for direct MHC-II binding, the overall length will be between 12 and 25 amino acids, such as between 14 and 20 amino acids. In some cases, where the synthetic peptide is processed in the cell (typically via proteasome processing) prior to MHC presentation, the overall length will typically be between 10 and 40 amino acids, with the changed amino acid at or near a central position in the synthetic peptide. In some embodiments, a peptide for MHC-I binding is a 9-mer. In some embodiments, a peptide for MHC-II binding is a 23-mer. In some embodiments, a peptide for MHC-II binding is a 25-mer.

As an example, a peptide (P) can include 0-25 amino acids on either side flanking the amino acid change or novel junction that arises due to a mutation. In one embodiment, the peptide (P) is a neoantigen sequence that comprises the 12 amino acids on either side flanking the amino acid change that arises from a single nucleotide polymorphism, for example, a 25 amino acid peptide, wherein the 13^{th} amino acid is the amino acid residue resulting from the single nucleotide polymorphism. In some embodiments, the peptide (P) is a neoantigen sequence that comprises the 12 amino acids on either side flanking an amino acid with a novel post-translational modification, for example, a 25 amino acid peptide, wherein the 13^{th} amino acid is the amino acid residue resulting from the novel post- translational modification site. In other embodiments, the peptide (P) is a neoantigen sequence that comprises 0-12 amino acids on either side flanking a novel junction created by an insertion, deletion or inversion. In some cases, the peptide (P) comprising neoantigens resulting from novel sequences can encompass the entire novel sequence, including 0-25 amino acids on either side of novel junctions that may also arise.

In some embodiments, further downstream analysis may be performed on the so identified sequence differences to identify those that lead to a new peptide sequence based on the cancer and patient specific mutation. Neoepitopes may therefore be identified by considering the type (e.g., deletion, insertion, transversion, transition, translocation) and impact of the mutation (e.g., non-sense, missense, frame shift, etc.), and may as such serve as a content filter through which silent and other non-relevant (e.g., non-expressed) mutations are eliminated.

In some embodiments, identified neoepitopes can be further filtered *in silico* against an identified patient HLA- type. Such HLA-matching is thought to ensure strong binding of the neoepitopes to the MHC-I complex of nucleated cells and the MHC-II complex of specific antigen presenting cells. Targeting both antigen presentation systems is particularly thought to produce a therapeutically effective and durable immune response involving both the cellular and the humoral branches of the immune system. It should also be appreciated that thusly identified HLA-matched neoepitopes can be biochemically validated *in vitro.*

HLA determination for both MHC-I and MHC-II can be done using various methods. In some embodiments, the HLA-type can be predicted from omics data *in silico* using a reference sequence containing most or all of the known and/or common HLA-types. For example, a patient' s HLA-type is ascertained (using wet chemistry or *in silico* determination), and a structural solution for the HLA-type is calculated or obtained from a database, which is then used as a docking model *in silico* to determine binding affinity of the neoepitope to the HLA structural solution. Suitable systems for determination of binding affinities include the NetMHC platform (see e.g., Nucleic Acids Res. 2008 Jul 1; 36(Web Server issue): W509-W512.), HLAMatchmaker (http://www. epitopes.net/downloads.html), and IEDB Analysis Resource (http://tools.immuneepitope.org/ mhcii/). Neoepitopes with high affinity (e.g., less than 100 nM, less than 75 nM, less than 50 nM for MHC-I; less than 500 nM, less than 300 nM, less than 100 nM for MHC-II) against the previously determined HLA-type are then selected. In calculating the highest affinity, modifications to the neoepitopes may be implemented by adding N- and/or C-terminal modifications to the epitope to further increase binding of a synthetic neoepitope to the HLA-type of the patient. Thus, neoepitopes may be native as identified or further modified to better match a particular HLA-type. In some embodiments, neoepitopes can be scored/ranked based on allele frequency multiplied by the transcripts per million number to get a likelihood score. This score can then be further augmented using HLA information and calculated or actual binding affinity to the patient' s HLA type.

Among provided embodiments are embodiments in which the neoepitopes are compared against a database that contains known human sequences to so avoid use of a human-identical sequence.

After the *in silico* identification of suitable neoepitope sequences, corresponding synthetic peptides are then prepared *in vitro* (e.g. , using solid phase synthesis). In particular embodiments, a library of synthetic peptides is prepared representing a plurality of different neoepitopes from the subject. The library can include 100, 1000, 10000 or more different peptides. To obtain a synthetic antibody against the identified neoepitope(s), it is contemplated that the epitope identified is prepared *in vitro* to yield a synthetic peptide.

Various methods can be used to prepare synthetic peptides. For example, peptides with cancer neoepitope sequences can be prepared on a solid phase (e.g., using Merrified synthesis), via liquid phase synthesis, or from smaller peptide fragments. Peptide epitopes can be obtained by chemical synthesis using a commercially available automated peptide synthesizer. In some embodiments, the peptides can be synthesized, for example, by using the Fmoc-polyamide mode of solid-phase peptide synthesis which is disclosed by Lu et al (1981).J. Org. Chem. 46,3433 and the references therein. In some aspects, peptides can be produced by expression of a recombinant nucleic acid in a suitable host and with a suitable expression system. In some aspects, recombinant methods can be used where multiple neoepitopes are on a single peptide chain, such as with spacers between neoepitopes or cleavage sites).

The peptides can be purified by any one, or a combination of techniques such as recrystallization, size exclusion chromatography, ion-exchange chromatography, hydrophobic interaction chromatography, and reverse-phase high performance liquid chromatography using e.g. acetonitrile/water gradient separation. In some embodiments, peptides can be precipitated and further purified, for example by high performance liquid chromatography (HPLC). Analysis of peptides can be carried out using thin layer chromatography, electrophoresis, in particular capillary electrophoresis, solid phase extraction (CSPE), reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

### B. Selection and Stimulation of a Population of T cells

The provided methods include obtaining and enriching or selecting a population of T cells from a biological sample for use as a first or input population of T cells. In some cases, the first population of T cells is one that is known or likely to contain T cells reactive to a tumor antigen or that are capable of being reactive to a tumor antigen, such as following an *ex vivo* co-culture with an autologous source of tumor antigen. For example, typically the first population of T cells is from a biological sample from a tumor. The biological smaple is from a subject with a tumor. The first population of T cells is further stimulated with one or more T cell stimulatory agent(s) (e.g. one or more recombinant cytokines,) in a closed system using serum free medium, wherein the one or more T-cell stimulating agents is or comprises recombinant IL-2, to produce a second or stimulated population of T cells containing T cells that have expanded following the stimulation.

In some embodiments, the incubation with the T cell stimulatory agent(s) is carried out directly on an input or first population of T cells selected from a biological sample from a subject, wherein the population of T cells selected from the biological sample (e.g. autologous T cells from the subject) is incubated with the T cell stimulatory agent(s). In other embodiments, the input population of T cells includes T cells that are likely to be or are suspected to be tumor reactive T cells, in which such cells are first selected from a population of T cells selected from a biological sample from a subject by selecting for cells positive for a surface marker that is upregulated on activated T cells (e.g. 4-1BB or OX40). In such embodiments, the incubation with the T cell stimulatory agent(s) is carried out after the enriching for the population of T cell cells comprising tumor-reactive T cells. In the provided embodiments, the incubation with the T cell stimulatory agent(s) is carried out before the coculturing of such T cells (stimulated T cells) with the APCs/peptide neoepitopes.

In some cases, conditions for stimulating the T cells by culture with one or more T cell stimulatory agent(s) results in activation of the cells and expansion or outgrowth of T cells present in the first or input population of T cells. In some embodiments, the conditions for stimulating the T cells with one or more T cell stimulatory agent(s) can include culturing the T cells under condition that results in bulk expansion of the T cells.

In the provided methods, the stimulated composition of T cells is then employed in subsequent downstream steps for enrichment and expansion of tumor reactive T cells, including steps that include co-culture of the stimulated T cells with antigen presenting cells (APCs) in the presence of T cell neopitope (mutated) peptide antigens to produce, yield or to pull out T cells that are tumor reactive T cells. The provided methods also include a step for selecting or enriching T cells reactive to a tumor antigen (tumor reactive T cells), after co-culturing T cells with APCs/peptide neoepitopes. The tumor reactive T cell populations are cultured under conditions for expansion, such as to produce a therapeutic T cell composition.

The input or first population of T cells is incubated in the presence of a T cell stimulatory agent(s). The incubation is carried out under conditions in which the T cell stimulatory agent(s) activates or stimulates the cells or promotes expansion of T cells present in the input or first population of T cells.

In some embodiments, the T cell stimulatory agent(s) include a further recombinant T cell stimulating cytokine, such as IL-7, IL-15 and/or IL-21. The T cell stimulating cytokine includes IL-2, optionally alone or in combination with another cytokine from among IL-7, IL-15 and/or IL-21.

In some embodiments, the T cell stimulatory (agent(s) can additionally include an agent or agents that engage CD3 and/or a costimulatory molecule, such as CD28. The T cell stimulatory agent(s) can include an anti-CD3 antibody, such as OKT3, and/or an anti-CD28 agent (presented by APC's or as a soluble antibody). In embodiments, prior to and/or during at least a portion of the co-culturing of T cells with APCs, the T cells selected from the biological sample (i.e. input or first population) are incubated in the presence of a T cell stimulatory agent(s), such as an anti-CD3 (e.g. OKT3)/anti-CD28 antibody. Thus, either before the coculture in the presence of APCs or after the selection of reactive cells, the T cells are incubated with one or more T-cell stimulating agents of lymphocytes, such as but not limited to anti-CD3 antibody (e.g. OKT3) and anti-CD28 (presented by APC's or as soluble antibodies), to produce a second population of T cells that include activated or stimulated T cells. In particular embodiments, one or more recombinant cytokines also are present as additional T cell stimulatory agents during the incubation.

In some embodiments, the incubation with one or more T cell stimulatory agent(s), such as an anti-CD3/anti-CD28 antibody, can be continued for a time period sufficient to activate or stimulate the cells. In some embodiments, the incubation with the T cell stimulatory agent(s), such as an anti-CD3/anti-CD28 antibody, is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days. In some embodiments, the incubation with the T cell stimulatory agent(s), such as anti-CD3/anti-CD28 antibody and, in some cases one or more recombinant cytokines, is for 12 hours to 96 hours, such as 24 hours to 48 hours, and generally at or about 48 hours.

In some embodiments, the cells are washed one or more times during the culturing to remove agents present during the incubation or culturing and/or to replenish the culture medium with one or more additional agents. In some embodiments, the cells are washed during the incubation or culturing to reduce or remove the T cell stimulatory agent(s) prior to completion of the culturing.

In some embodiments, the methods of stimulating T cells provided herein include incubation with a T cell stimulatory agent(s) at a temperature suitable for the growth of human T lymphocytes, for example, at least about 25 degrees Celsius, generally at least about 30 degrees, and generally at or about 37 degrees Celsius. In some embodiments, the methods of culturing or incubation is carried out in serum-free media.

### 1. Selecting a Population of T cells

The provided methods include processing a biological sample containing a population of T lymphocyte cells obtained from a donor subject that has a tumor to produce a first population of T lymphocyte cells. In some embodiments, the processing includes selecting or obtaining a population of T cells from a biological sample, which can be used as the source or input of T cells for stimulation with one or more T cell stimulatory agents(s), includingrecombinant IL-2 and optionally e.g. anti-CD3/anti-CD28. In some embodiments, the T cells are from a biological sample from a subject that is known or likely to contain tumor reactive T cells. The collected biological sample contains or is suspected to contain lymphocytes that have endogenous TCRs that are reactive to mutations present on a tumor.

In aspects of any of the provided embodiments, a suitable biological sample from a subject, such as from a patient of interest, i.e., a patient suspected of having or known to have cancer, is obtained. In some embodiments, the sample is one that is known or suspected of containing T cells, such as T cells that may be or may likely express an endogenous T cell receptor (TCR) that is specific to, binds or recognizes a tumor-associated antigen. The sample may be derived from any initial source that would contain or is suspected of containing such T cells. In some aspects, biological sample sources of interest include, but are not limited to, many different physiological sources, e.g. tissue derived samples, e.g. homogenates, and blood or derivatives thereof.

Any of a variety of samples can be used as a source of potentially reactive T cells. Although the tumor and downstream lymph nodes may have the highest frequency of reactive T cells (Powell et al., Clin. Cancer. Res., 2014), other sample sources also can be used. In some cases the sample is a tumor sample, a tertiary lymphoid site, a draining lymph node, peripheral blood or bone marrow. In some embodiments, the sample is a tumor sample. In some embodiments, the sample is a lymph sample. In some embodiments, the sample is a peripheral blood sample.

The samples include tissue, fluid, and other samples taken directly from the subject, as well as samples resulting from one or more processing steps, such as separation, e.g. selection or enrichment, centrifugation, washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In many embodiments, the sample may be derived from fluids in which the T cells of interest are at least suspected of being present. In many embodiments, a suitable initial source for the sample is blood. In some embodiments, the biological sample is a blood-derived sample. The blood-derived sample may be derived from whole blood or a fraction thereof, e.g. serum, plasma, etc., where in many embodiments the sample is derived from blood cells harvested from whole blood. In some aspects, the sample source contains mononuclear cells. For example, a biological sample is or contains peripheral blood mononuclear cells (PBMCs) or is derived from PBMCs.

In some embodiments in which the sample is a PBMC derived sample, the sample is generally a fluid PBMC derived sample. Any convenient methodology for producing a fluid PBMC sample may be employed. In many embodiments, the fluid PBMC derived sample is prepared by separating PBMCs from whole blood, i.e., collecting PBMCs, e.g., by centrifugation (such as by Ficoll-Hypaque density gradient centrifugation, where representative protocols for such separation procedures are disclosed in WO 98/15646 and U.S. Pat. No. 5,985,565).

In some embodiments, the sample is a tumor sample and thereby provides a source of tumor-infiltrating lymphocytes (TILs). In some aspects, TII,s are T cells that have left the bloodstream of a subject and migrated into or infiltrated a tumor. In particular aspects, TII,s are reactive to a tumor antigen.

A patient tumor sample may be obtained by any of a variety of methods in which the method obtains a sample that contains a mixture of tumor and TIL cells. In some embodiments, the tumor sample is obtained by surgical resection. In some embodiments, the tumor sample is obtained by needle biopsy. In general, the tumor sample may be from any solid tumor, including primary tumors, invasive tumors or metastatic tumors. The tumor sample may also be a liquid tumor, such as a tumor obtained from a hematological malignancy. The solid tumor may be of any cancer type, including, but not limited to, breast, pancreatic, prostate, colorectal, lung, brain, renal, stomach (gastrointestinal), and skin (including but not limited to squamous cell carcinoma, basal cell carcinoma, and melanoma). In particular embodiments, the tumor is any as described in Section III. In some embodiments, the tumor sample is from the same tumor source as was used to identify a neoantigen for preparing peptide neoepitopes.

In provided embodiments, the obtained tumor sample is fragmented into small pieces of between at or about 1 mm³ and at or about 8 mm³ in size, such as between at or about 1 mm³ and at or about 6 mm³, between at or about 1 mm³ and at or about 4 mm³, between at or about 1 mm³ and at or about 2 mm³. In some embodiments, the tumor fragment is from about 2-3 mm³. In some embodiments, the tumor fragment is from about 1-2 mm³. In some embodiments, the tumor fragment is obtained by physical fragmentation, such as by dissection. In some embodiments, the tumor fragment is obtained by sharp dissection.

In some of any of the provided embodiments, the obtained tumor sample is fragmented into small pieces of between at or about 1 mm and at or about 8 mm in diameter, such as between at or about 1 mm and at or about 6 mm in diameter, between at or about 1 mm and at or about 4 mm in diameter, between at or about 1 mm and at or about 2 mm in diameter. In some embodiments, the tumor fragment is from about 2-3 mm in diameter. In some embodiments, the tumor fragment is from about 1-2 mm in diameter. In some embodiments, the tumor fragment is obtained by physical fragmentation, such as by dissection. In some embodiments, the tumor fragment is obtained by sharp dissection.

In some embodiments, the tumor sample is cryopreserved prior to fragmentation. In some embodiments, the tumor fragments are cryopreserved.

In some embodiments, obtained tumor fragments are placed into culture media under conditions and with appropriate nutrients to mediate T cell activation and/or sustain T cell expansion, such as any of the conditions described in Subsection I.B.2 below for stimulation of T cells. In some embodiments 1 to 500 tumor fragments (e.g. each 1-8 mm in size) are placed in an appropriate culture vessel under conditions for expansion. In some embodiments, 10, 20, 30, 40, 50 or more fragments are cultured under conditions for expansion. The culture vessel can be a microwell, flask, tube, bag or other closed system device. In some embodiments the culture vessel is a closed container that provides a gas-permeable surface area, such as a a gas permeable flask. An exemplary culture vessel that provides a gas-permeable surface area include G-Rex plates or flasks. In some embodiments, 1 tumor fragment (about 1-8 mm in diameter) is placed for each about 2 cm² area of a culture vessel. The particular culture vessel can be chosen based on the number of tumor fragments available and/or the desired yield of cells. The choice of culture vessel (e.g. G-Rex) can be chosen by linearly scaling the number of fragments seeded to the surface area of the culture vessel. In some embodiments, the surface areas of the culture vessel is about 2 cm² (e.g. G-Rex 24 well plate) and about 1 tumor fragment (about 1-8 mm in diameter) is placed in the culture vessel. In some embodiments, the surface area of a culture vessel is about 10 cm² (e.g. G-Rex 10 or G-Rex 10M) and about 5 tumor fragments (each about 1-8 mm in diameter) are placed in the culture vessel. In some embodiments, the surface area of a culture vessel is about 100 cm² (e.g. G-Rex 100 M/100M-CS) and about 50 tumor fragments (each about 1-8 mm in diameter) are placed in the culture vessel. In some embodiments, the surface area of a culture vessel is about 500 cm² (e.g. G-Rex 500 M/500M-CS) and about 250 tumor fragments (each about 1-8 mm in diameter) are placed in the culture vessel. In aspects of the provided methods, increasing the size of the culture vessel, and hence the number of tumor fragments per vessel, may decrease variability as compared to methods involving smaller culture vessels and/or fewer fragments per vessel, such by pooling larger numbers of fragments to minimize inter-tumor variability among fragments.

In some embodiments, the tumor fragments are placed in culture media for stimulation of the cells using any of the conditions described in Subsection I.B.2 below. The culture media is a serum-free media containing recombinant cytokine, where the recombinant cytokine is or comprisesIL-2, and optionally further comprises IL-7, IL-15, and/or IL-21, such as recombinant IL-12 or recombinant IL-7 and IL-15. The concentration of recombinant cytokine can include any as described. The culture media is a serum-free media containing recombinant IL-2, such as from at or about 300 IU/mL to at or about 1000 IU/mL, for example at or about 300 IU/mL. Tthe culture media is a serum-free media containing IL-2, optionally further containing an anti-CD3 antibody and/or CD28 targeting agent (e.g. anti-CD28 antibody) and one or more additional recombinant cytokines.

In some embodiments, the provided methods involve obtaining cells from the tumor fragments, such as by enzymatic digestion of tumor fragments to obtain TILs. Enzymatic digestion can be carried out using a collagenase, such as a type IV collagenase or a type I/II collagenase. The enzyme, such as a collagenase, can be present in media for the enzymatic digestion at a concentration of from at or about 1 mg/mL to at or about 5 mg/mL, such as at or about 1 mg/mL, at or about 2 mg/mL, at or about 3 mg/mL, at or about 4 mg/mL or at or about 5 mg/mL, or any value between any of the foregoing. In some embodiments, the enzymatic digestion is with a media that includes type IV collagenase, such as from at or about 1 mg/mL to at or about 5 mg/mL. In some embodiments, the enzymatic digestion is with a media that includes type I/II collagenase, such as from at or about 1 mg/mL to at or about 5 mg/mL. In other embodiments, enzymes from the Miltenyi human tumor dissociation kit can be used (e.g. Cat. O. 130-095-929; Miltenyi Biotec). The enzymatic media containing the enzyme can be a serum-free media, such as any as described. In particular embodiments, enzymatic media includes collagenase, e.g., Roswell Park Memorial Institute (RPMI) 1640 buffer, 2 mM glutamate (e.g. GlutaMAX), 10 mg/mL gentamicin, 30 units/mL of DNase and 1.0 mg/mL of collagenase). In some embodiments, enzymatic media includes a serum free media (e.g. OpTmizer) containing 2 mM glutamate (e.g. GlutaMAX), 10 µg/mL gentamicin, an immune cell serum replacement (e.g. CTS Immune Cell Serum Replacement) and 1.0 mg/mL to 5.0 mg/mL of collagenase). In some embodiments, the collagenase is a type IV collagenase. In some embodiments, the collagenase is a type I/II collagenase.

The tumor fragment is then mechanically dissected to dissociate the TILs, e.g., using a tissue dissociator. An example of a tissue dissociator is GentleMACs^{™} (Miltenyi Biotec) to homogenize the tissue. Tumor digests may be produced by placing the tumor in enzymatic media and mechanically dissociating the tumor for approximately 1 minute, followed by incubation for 30 minutes at 37 °C in 5% CO₂, followed by repeated cycles of mechanical dissociation and incubation under the foregoing conditions until only small tissue pieces are present. At the end of this process, if the cell suspension contains a large number of red blood cells or dead cells, a density gradient separation using FICOLL can be performed to remove these cells. In some cases, separation can be achieved by centrifugation, in which case the cell pellet can be resuspended and strained through a e.g. 70 µm strainer to remove debris. Alternative methods known in the art may be used, such as those described in U.S. Patent Application Publication No. 2012/0244133 A1. Any of the foregoing methods may be used in any of the embodiments described herein for methods of obtaining TILs for use in the provided methods.

In some embodiments, digested cells from the tumor fragments are placed into culture media under conditions and with appropriate nutrients to mediate T cell activation and/or sustain T cell expansion, such as any of the conditions described in Subsection I.B.2 below for stimulation of T cells. The cells are seeded at a particular density suitable for the particular culture vessel. The culture vessel can be a microwell, flask, tube, bag or other closed system device. In some embodiments the culture vessel is a closed container that provides a gas-permeable surface area, such as a a gas permeable flask. An exemplary culture vessel that provides a gas-permeable surface area include G-Rex plates or flasks. In some embodiments approximately 5 x 10⁵ to 2 x 10⁶ cells of an enzymatically digested single cell suspension are seeded for each about 2 cm² area of a culture vessel. The particular culture vessel can be chosen based on the number of cells available and/or the desired yield of cells. The choice of culture vessel (e.g. G-Rex) can be chosen by linearly scaling the number of cells seeded to the surface area of the culture vessel. In some embodiments, the surface areas of the culture vessel is about 2 cm² (e.g. G-Rex 24 well plate) and about 5 x 10⁵ to 2 x 10⁶ cells of an enzymatically digested single cell suspension is placed in the culture vessel. In some embodiments, the surface area of a culture vessel is about 10 cm² (e.g. G-Rex 10 or G-Rex 10M) and about 2.5 x 10⁶ to 1 x 10⁷ cells of an enzymatically digested single cell suspension are placed in the culture vessel. In some embodiments, the surface area of a culture vessel is about 100 cm² (e.g. G-Rex 100 M/100M-CS) and about 2.5 x 10⁷ to 1 x 10⁸ cells of an enzymatically digested single cell suspension are placed in the culture vessel. In some embodiments, the surface area of a culture vessel is about 500 cm² (e.g. G-Rex 500 M/500M-CS) and about 1.25 x 10⁸ to 5 x 10⁸ cells of an enzymatically digested single cell suspension are placed in the culture vessel.

The culture media is a serum-free media containing recombinant IL-2. In some embodiments, one or more additional T cell stimulating agent can also be included. The culture media is a serum-free media containing recombinant IL-2 and optionally an anti-CD3 antibody and/or a CD28 targeting agent (e.g. anti-CD28 antibody) and one or more further recombinant cytokines (eIL-7, IL-15 and/or IL-21).

The sample may be obtained from a variety of different subjects/patients/hosts. Generally such hosts are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the hosts will be humans.

In some aspects, the subject is a human. Accordingly, the cells in some embodiments are primary cells, e.g., primary human cells. In some embodiments, the sample is autologous to a subject to be treated, such as a subject who is a patient in need of a particular therapeutic intervention, such as the adoptive cell therapy for which cells are being isolated, processed, and/or expanded in accord with the provided methods. In some embodiments, the sample is allogenic to a subject to be treated.

In some embodiments, the T cells for use in connection with the provided methods can be enriched or sorted a variety of ways including, but not limited to, magnetic bead separation, fluorescent cell sorting, and disposable closed cartridge based cell sorters. In particular aspects, one or more reagents specific to T cells or a subset thereof, such as reagents specific to T cell activation markers for selecting reactive cells, can be used including, but not limited to, florescent antibodies, nanoparticles or beads on cell selection equipment, but not limited to, the CliniMACS, Sony FX500 or the Tyto cell sorting systems (Miltenyi).

In some aspects, T cells can be selected from a biological sample, such as based on T cell markers CD3, CD4 or CD8. In some embodiments, selecting for a T cell that is surface positive for one or more cell surface marker includes any method for separation based on such markers.

In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. In some embodiments, the immunoaffinity-based selections include contacting a sample containing cells, such as a sample containing a bulk population of T cells, e.g. primary human T cells, containing CD3+ T cells or CD4+ and CD8+ cells, with an antibody or binding partner that specifically binds to the cell surface marker or markers. In some embodiments, the antibody or binding partner is bound to a solid support or matrix, such as a sphere or bead, for example a nanoparticle, microbeads, nanobeads, including agarose, magnetic bead or paramagnetic beads, to allow for separation of cells for positive and/or negative selection. In some embodiments, the spheres or beads can be packed into a column to effect immunoaffinity chromatography, in which a sample containing cells, such as primary human T cells containing CD3+ T cells or CD4+ and CD8+ cells, is contacted with the matrix of the column and subsequently eluted or released therefrom. In other embodiments, the antibody or binding partner is detectably labeled.

In some aspects, the sample or composition of cells to be separated is incubated with small, magnetizable or magnetically responsive material, such as magnetically responsive particles or microparticles, such as nanoparticles or paramagnetic beads. The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select. Such beads are known and are commercially available from a variety of sources including, in some aspects, Dynabeads^{®} (Life Technologies, Carlsbad, CA), MACS^{®} beads (Miltenyi Biotec, San Diego, CA) or Streptamer^{®} bead reagents (IBA, Germany). In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained.

In certain embodiments, the sample is contacted with a binding agent, e.g., a detectably labeled binding agent, that specifically binds to a cell surface marker. In certain embodiments, the detectably labeled binding agent(s) are fluorescently labeled. In certain embodiments, T cells labeled with binding agents specific to a cell surface marker are identified by flow cytometry. In certain embodiments, the method further includes separating any resultant T cells labeled with the binding agent(s) from other components of the sample to produce a composition enriched for T cells surface positive for the one or more cell surface marker. Cell selection sorting equipment can be used that has a sufficiently high-throughput to handle large volumes and cell numbers. Non-limiting cell sorting equipment includes, for example, Sony FX500 or the Tyto cell sorting systems (Miltenyi).

The incubation generally is carried out under conditions whereby the antibodies or binding partners, or molecules, such as secondary antibodies or other reagents, which specifically bind to such antibodies or binding partners, which are attached to the magnetic particle or bead and/or are detectably labeled, specifically bind to cell surface molecules if present on cells within the sample. In some aspects, cells bound to the antibodies can be recovered or separated from non-bound cells in the sample.

In some aspects, a combination of positive and negative selection is performed during the same selection step, where the positive and negative fractions are retained and further processed or subject to further separation steps. Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells. For example, in some aspects, a selection of one of the CD4+ or CD8+ population enriches for said population, either the CD4+ or CD8+ population, but also can contain some residual or small percentage of other non-selected cells, which can, in some cases, include the other of the CD4 or CD8 population still being present in the enriched population.

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In particular embodiments, a T cell population is one that includes both CD4+ and CD8+ T cells. Many cancers, including solid tumors, such as many common epithelial indications (e.g. GI), express class I and class II restricted mutations. In order for a T cell product to target such indications, e.g. common epithelial indications, it is contemplated that both CD8+ T cells to recognize class I MHC-restricted molecules and CD4+ T cells to recognize Class II MHC-restricted molecules are necessary.

In some embodiments, the methods include isolation, selection and/or enrichment of CD3+ cells. In some embodiments, the methods include isolation, selection and/or enrichment of CD4+ and CD8+ cells. In some aspects, a CD4⁺ or CD8⁺ selection step, such as positive selection for CD4 and positive selection for CD8, is used to separate CD4⁺ helper and CD8⁺ cytotoxic T cells. Such selections in some aspects are carried out simultaneously and in other aspects are carried out sequentially, in either order. In some embodiments, the methods include enriching for CD4+ and CD8+ T cells by selecting for T cells surface positive for CD3 or by sequential or simultaneous selection for T cells surface positive for CD4 and T cells surface positive for CD8. Such CD3+ T cells, or CD4⁺ and/or CD8⁺ populations, can be further sorted into sub-populations by positive or negative selection for markers expressed or expressed to a relatively higher degree on tumor-reactive T cells or on T cells having expression of T cell activation markers associated with tumor-reactive T cells, e.g. as described in Section I.D.

In some embodiments, the selected T cells can be further enriched for tumor-reactive T cells based on expression of a marker associated with activated T cells. Particular markers for use in selecting or enriching for such tumor-reactive T cells is described in Section I.D. below. In other cases, selection or enrichment of tumor-reactive T cells is carried out in one or more subsequent step of the process, such as after co-culture with one or more mutated peptide (peptide neoepitope).

In some embodiments, selecting the T cells from the biological sample further includes enriching or selecting for tumor-reactive T cells or T cells that express one or more activation markers associated with tumor-reactive T cells. The T cell activation markers include cell surface markers whose expression is upregulated or specific to T cells that have been exposed to antigen and activated. Exemplary markers are described in Section I.D below.

In some of any of the provided embodiments, the biological sample is a lymph sourced sample or a tumor sourced sample, and wherein: the number of cells at the initiation of the culturing is between at or about 10 × 10⁶ and 100 × 10⁶ total viable cells, 20 × 10⁶ and 100 × 10⁶ total viable cells, or 12 × 10⁶ and 43 × 10⁶ total viable cells; or is at or about 10 × 10⁶ total viable cells, at or about 12 × 10⁶ total viable cells, 20 × 10⁶ total viable cells, 40 × 10⁶ total viable cells, 60 × 10⁶ total viable cells, or 100 × 10⁶ total viable cells, , or any value between any of the foregoing. In some embodiments, the percentage of tumor reactive T cells at the initiation of the culturing is between at or about 1% and at or about 90%, at or about 1% and at or about 75%, at or about 1% and at or about 50%, at or about 1% and at or about 25% or at or about 1% and at or about 14%.

In some of any of the provided embodiments, the biological sample is a lymph sourced sample or a tumor sourced sample, and wherein the number of T cells surface positive for the T cell activation marker at the initiation of the culturing is between at or about 0.7 x 10⁶ and at or about 15 × 10⁶ T cells, 1 × 10⁶ and at or about 15 × 10⁶ T cells, or at or about 0.7 × 10⁶ and at or about 5.4 × 10⁶ T cells; or is at or about 0.7 × 10⁶ T cells, 1 × 10⁶ T cells, 5.4 × 10⁶ T cells, or 15 × 10⁶ T cells, or any value between any of the foregoing.

### 2. Stimulation of T cells for Initial Expansion

In aspects of the provided methods, the T cells from the biological sample (input or first population of T cells, such as present in a resected tumor fragment) are incubated or cultured in the presence of one or more T cell stimulatory agent(s) under conditions for stimulating the T cells, such as to expand the T cells wherein the stimulation is performed in a closed system using serum free medium, and wherein the one or more first T-cell stimulating agents is or comprises recombinant IL-2. In some embodiments, the incubation or culturing with one or more T cell stimulatory agent(s) results in expansion or outgrowth of selected T cells, or a desired subset or subtype thereof or for viable cells thereof, for use in subsequent steps of the provided methods. Non-limiting examples of T cell stimulatory agent(s) and conditions for incubation or culture are described herein.

In some embodiments, the T cell stimulatory agent(s) include a further recombinant T cell stimulating cytokine, such as IL-7, IL-15 and/or IL-21. The T cell stimulating cytokine includes IL-2, optionally alone or in combination with another cytokine from among IL-7, IL-15 and/or IL-21. In some embodiments, the T cell stimulating cytokine additionally includes IL-7 and IL-15.

In some of any of the provided embodiments, the additional T cell stimulatory agent(s) is selected from an agent that initiates TCR/CD3 intracellular signaling and an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the additional T cell stimulatory agent(s) is an anti-CD3 antibody and an anti-CD28 antibody that each are soluble.

Thus, among the provided methods are methods of culturing T cells for manufacture of tumor reactive T cells in which T cells are cultured or incubated in the presence of a T cell stimulatory agent under conditions to expand T cells, such as present in the co-culture. In some embodiments, the T cell stimulatory agent includes an anti-CD3 antibody and anti-CD28 antibody.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include theUHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the T cell stimulatory agent(s) can include adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

The T cell stimulatory agent(s) include one or more recombinant cytokines. In some embodiments, the cytokine is added or is exogenous to the culture media. Thus, in some embodiments, one or more further recombinant cytokine also is included during the culturing. The recombinant cytokine includes IL-2 and optionally one or more of IL-7, IL-15 or IL-21. In some embodiments, the culturing and incubation is carried out in the presence of recombinant IL-2, IL-15 and IL-7. The culturing is carried out in the presence of a recombinant IL-2. In some embodiments, the culturing is carried out in the further presence of IL-15 and IL-17. In particular embodiments, the recombinant cytokine(s) is human.

The recombinant cytokine generally is a recombinant human protein. In particular embodiments, the recombinant cytokine is present in the cell culture medium during the incubation at a concentration of at least or at least about 0.5 IU/mL, at least or at least about 1.0 IU/mL, at least or at least about 5 IU/mL, at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

Recombinant IL-2 is present in the cell culture medium. IL-2 is a cytokine that supports T cell recovery and proliferation. IL-2 also supports the homeostasis of T cells, thereby supporting their phenotype, differentiation status, and immune memory. In some cases, induction of regulatory T cells in the tumor microenvironment may lead to low bioavailability of IL-2. Recombinant IL-2 has been regularly used in broad expansion of T cells in various contexts. Recombinant IL-2 is commercially available. In particular embodiments, recombinant IL-2 is GMP grade (e.g. MACS GMP Recombinant Human IL-2, Miltenyi Biotec).

Recombinant IL-2 can be included in cell culture media during various stages of the provided process. Recombinant IL-2 is included in the initial T cell expansion (first expansion), such as to promote TIL outgrowth and allow their proliferation from solid tumor. IL-2 also can be included in antigen-presenting cell co-culture as described in Section I.C, such as to allow for peak activation of neo-antigen reactive T prior to their separation or selection. Recombinant IL-2 is also included in cultures to expand tumor-reactive T cells during the second expansion phase, such as described in Section I.D.

In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. IL-15 is a cytokine that is involved in memory T cell homeostasis and activation. In some cases, IL-15 can promote effector functions of antigen-experienced T cells in the absence of antigen and prevent their differentiation into an exhausted phenotype. IL-15 also plays a role in T cell proliferation. Recombinant IL-15 is commercially available. In particular embodiments, recombinant IL-15 is GMP grade (e.g. MACS GMP Recombinant Human IL-15, Miltenyi Biotec).

Recombinant IL-15 can be included in cell culture media during various stages of the provided process. In some cases, recombinant IL-15 can be included in the initial T cell expansion (first expansion), such as to promote TIL expansion to promote their outgrowth and allow their proliferation and/or stabilize phenotype from solid tumor. Recombinant IL-15 also can be included in antigen-presenting cell co-culture as described in Section I.C, such as to allow for peak activation of neo-antigen reactive T prior to their separation or selection. In some cases, recombinant IL-15 can also be included in cultures to expand tumor-reactive T cells during the second expansion phase, such as described in Section I.D. In some cases, recombinant IL-15 can be combined with recombinant IL-7 to provide for activation, survival and/or expansion of tumor-reactive T cells in the provided methods.

In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. IL-7 is a cytokine that is involved in promoting T cell maintenance and homeostasis. In some cases, IL-7 can boost memory T cell survival and proliferation, particularly the central memory compartment. Recombinant IL-7 is commercially available. In particular embodiments, recombinant IL-7 is GMP grade (e.g. MACS GMP Recombinant Human IL-7, Miltenyi Biotec).

Recombinant IL-7 can be included in cell culture media during various stages of the provided process. In some cases, recombinant IL-7 can be included in the initial T cell expansion (first expansion), such as to promote TIL expansion to promote their outgrowth and allow their proliferation and/or stabilize phenotype from solid tumor. IL-7 also can be included in antigen-presenting cell co-culture as described in Section I.C, such as to allow for peak activation of neo-antigen reactive T prior to their separation or selection. In some cases, recombinant IL-7 can also be included in cultures to expand tumor-reactive T cells during the second expansion phase, such as described in Section I.D. Inclusion of recombinant IL-7 in the process can maintain or support expansion of memory T cell subsets in the process. In some cases, recombinant IL-7 can be combined with recombinant IL-15 to provide for activation, survival and/or expansion of tumor-reactive T cells in the provided methods. In some such embodiments, the combination of recombinant IL-7 and IL-15 is an alternative to the use of recombinant IL-2 in the culture, and the culture media does not additionally contain recombinant IL-2.

In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. IL-21 is a cytokine that supports a broad range of T cell activation without increasing regulatory T cell signaling. In some cases, IL-21 can support memory cell stabilization, effector function, and proliferation of antigen-experienced T cells. IL-21 can induce upregulation of effector molecules in both CD4 and CD8 T cells. Recombinant IL-21 is commercially available. In particular embodiments, recombinant IL-21 is GMP grade (e.g. MACS GMP Recombinant Human IL-21, Miltenyi Biotec).

Recombinant IL-21 can be included in cell culture media during various stages of the provided process. In some cases, recombinant IL-21 can be included in the initial T cell expansion (first expansion), such as to promote TIL outgrowth from solid tumor, including by stabilizing memory T cell activation, function and/or proliferation. In some aspects, the presence of IL-21 allows for improved recovery of TIL. Recombinant IL-21 also can be included in antigen-presenting cell co-culture as described in Section I.C, such as due to the ability to stimulate expression of T cell activation markers, including expression of activation markers on neo-antigen reactive TIL. In some cases, recombinant IL-21 can also be included in cultures to expand tumor-reactive T cells during the second expansion phase as described in Section I.D, such as to support proliferation and stabilization of memory phenotype.

In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

The T cell stimulatory agent(s) present during the incubation, such as for expansion of cells, contain recombinant IL-2. In some embodiments, one or more other stimulating agent can be included such as one or more other recombinant cytokine from IL-7, IL-15, IL-21 or an anti-CD3 antibody (e.g. OKT-3). In some cases in which an anti-CD3 antibody (e.g. OKT-3) the T cell stimulating agent(s) also can include a costimulating agent, such as provided by antigen-presenting feeder cells, such as PBMCs, or a soluble anti-CD28 antibody.

In particular embodiments, T cell stimulatory agent(s) present during the incubation, such as for expansion of cells contains recombinant IL-2, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-15 and recombinant IL-7, an anti-CD3 antibody, e.g. OKT-3, and antigen-presenting feeder cells, such as PBMCs.

In some embodiments, the incubation with the T cell stimulatory agent(s) is carried out under conditions for initial expansion of T cells from the biological sample. In some embodiments, the cells are cultured at about 37 °C with about 5% CO₂.

In some embodiments, the incubation with the T cell stimulatory agent(s) is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation with the T cell stimulatory agent(s) is carried out for 7 to 21 days, such as 7 days, 8 days, 9 days, 10 days, 11 days, 12 days, 13 days, 14 days, 15 days, 16 days, 17 days, 18 days, 19 days, 20 days or 21 days, or any value between any of the foregoing. In some embodiments, the incubation is carried out for 7-14 days. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days.

The incubation, such as for initial expansion of T cells in the biological sample, can be carried out under GMP conditions. The incubation is in a closed system, which in some aspects may be a closed automated system. The culture media containing the T cell stimulatory agent(s) is a serum-free media. In some embodiments, the incubation is carried out in a closed automated system and with serum-free media.

In some embodiments, the initial expansion of cells under the one or more stimulatory conditions is in a culture vessel suitable for cell expansion. In some embodiments, the culture vessel is a gas permeable culture vessel, such as a G-Rex system (e.g. G-Rex 10, G-Rex 10M, G-Rex 100 M/100M-CS or G-Rex 500 M/500M-CS). In some embodiments the culture vessel is a microplate, flask, bar or other culture vessel suitable for expansion of cells in a closed system. In some embodiments, expansion can be carried out in a bioreactor. In some embodiments, the initial expansion can be carried out using a cell expansion system by transfer of the cells to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 × 10⁶ cells/mL to 1.5 × 10⁶ cells/mL. In some embodiments, the density is at or about 0.5 × 10⁶ cells/mL, 0.75 × 10⁶ cells/mL, 1 × 10⁶ cells/mL, 1.25 × 10⁶ cells/mL or 1.5 × 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

The expansion methods can be carried out under GMP conditions. The expansion is carried out in a closed automated system and using serum free medium. In some embodiments, one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, immediately after the incubation, the stimulated cells can be collected for subsequent co-culture with APCs, such as in accord with methods described in Section I.C below.

In some embodiments, the stimulated cells are collected and are cryofrozen. The provision of an intermediate hold step by cryopreservation after the initial expansion phase can be used to coordinate timing with the neoepitope identification and peptide generation, such as described in Section I.A and/or the generation of APCs as described in Section I.C. In some embodiments, for cryopreservation, the stimulated cells are formulated as a composition with a cryoprotectant. In some embodiments, the cryoprotectant is or comprises DMSO and/or is glycerol. In some embodiments, compositions formulated for cryopreservation can be stored at low temperatures, such as ultra low temperatures, for example, storage with temperature ranges from -40 °C to -150 °C, such as or about 80 °C ± 6.0 ° C.

In some embodiments, the cryopreserved cells are prepared for subsequent steps by thawing. In some cases, the cells can be ready for subsequent culturing with APCs and peptides immediately after thawing following one or more wash steps.

### C. Co-culture of T cells With APCs

In embodiments of the provided methods, once the neopitopes that encode for proteins are synthesized a plurality of the synthetic peptides are contacted with antigen presenting cells under conditions to present one or more non-native peptides in the context of an MHC molecule and incubated with the second population of T cells from a population of T cells for recognition of the peptides presented on the APCs, in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor. In some embodiments, the synthetic peptides are pulsed into autologous or allogeneic APCs that are then co-cultured with patient T cells. Antigen presenting cells are used to present these peptides. T cells that recognize these peptides on the surface of the APC can then be isolated, such as by methods described below. The incubated cells can be cultured under conditions that enrich for and expand tumor-reactive T cells, i.e. T cells containing endogenous TCR that are reactive to peptides present on the APCs, in the culture. In some embodiments, the methods include culturing the T cells under conditions for expansion until a threshold amount of T cells is obtained and/or until up to 20 days after initiation of incubation. In some embodiments, of the provided methods the method can include co-culturing the T cells with the APCs over the course of several hours to days and then separating antigen presenting cells from the population of T cells for the expansion of the T cells under conditions to enrich or expand tumor-reactive T cells. In some embodiments, of the provided methods the method can include co-culturing the T cells with the APCs over the course of 1-7 days, and then separating antigen presenting cells from the population of T cells for the expansion of the T cells under conditions to enrich or expand tumor-reactive T cells. In some embodiments, the separating can include isolating or selecting reactive T cells from culture based on one or more T cell activation markers on T cells.

Various methods can be used for culturing cells for antigen-specificity, see e.g. US published application No. US2017/0224800.

In some embodiments, the tumor reactive T cells are co-cultured with APCs that have been contacted or exposed to present a peptide, e.g. containing a mutated amino acid sequence, such as neoepitope peptides as described above. The method may comprise inducing autologous antigen presenting cells (APCs) of the patient to present the mutated amino acid sequence. The APCs may include any cells which present peptide fragments of proteins in association with major histocompatibility complex (MHC) molecules on their cell surface. The MHC molecule can be any MHC molecule expressed by the patient including, but not limited to, MHC Class I, MHC Class II, HLA-A, HLA-B, HLA-C, HLA-DM, HLA-DO, HLA-DP, HLA-DQ, and HLA-DR molecules. The APCs may include, for example, any one or more of macrophages, DCs, Langerhans cells, B-lymphocytes, and T-cells. In particular embodiments, the APCs are DCs. In some particular embodiments, the APCs are B cells. In some embodiments, the APCs are artificial APCs.

In particular embodiments, the APCs include cells that are able to present Class I and Class II restricted molecules. For example, B cells and DCs both have the ability to present MHC class I and MHC class II restricted molecules. In some embodiments, the APC cell sample includes B cells and DCs. In some embodiments, the APC cell sample is enriched for B cells, such as by selection or isolation from a primary cell sample. In some embodiments, the APC cell sample is enriched for DCs, such as by selection or isolation from a primary cell sample.

In some embodiments, the APCs express MHC class I and/or MHC class II molecules with a matched HLA from which the source of T cells has been obtained. In particular embodiments, both the APCs and T cells have been isolated from the same subject, i.e. are autologous to the cancer patient. In some embodiments, the method may comprise inducing autologous antigen presenting cells (APCs) of the patient to present the mutated amino acid sequence. By using autologous APCs from the patient, the methods may identify T cells that have antigenic specificity for a mutated amino acid sequence encoded by a cancer-specific mutation that is presented in the context of an MHC molecule expressed by the patient.

In some embodiments, the APCs are cells from a blood or apheresis sample from a subject, such as the patient. In some embodiments, the APCs include cells present in a peripheral blood mononuclear cell (PBMC) sample. Typically, APCs function in a PBMC culture primarily involves monocytes and B cells. In some embodiments, a population of isolated PBMCs can be used as APCs in the provided methods. PBMCs can be obtained using standard methods such as Ficoll-Paque gradient separation. In some cases, the APCs are or include B cells that are isolated from the blood or apheresis sample or from a PBMC sample. In other cases, the APCs are or include monocytes isolated from the blood or apheresis sample or from a PBMC sample. In some aspects, the monocytes can be used as a source for preparing monocyte-derived DCs for use as APCs. In some embodiments, a source of monocyte-derived DCs (e.g. CD11c^{high}MHCII^{high}CD14^{low} cells) can be generated ex vivo from isolated monocytes, by culture with GM-CSF and IL-4 for 4 to 6 days to produce monocyte-derived dendritic cells. In particular embodiments, the monocytes are isolated from PBMCs such as by CD14 selection, and then are cultured with GM-CSF and IL-4 for 4 to 6 days.

In some embodiments, the APCs are primary cells (e.g. B cells or monocyte-derived DCs) that are replication competent, for example, the cells are not subjected to irradiation, heat treatment or other method that would result in their inactivation. In particular embodiments, the provided methods do not use irradiated APCs. In some embodiments, the APCs are freshly isolated primary cells obtained from the subject, or are derived from primary cells obtained from the subject. In some embodiments, the APCs have been cryopreserved and subsequently thawed prior to the co-culture with the stimulated T cells in accord with provided methods.

In some particular embodiments, B cells are used as a source of APCs and are generated from a patient apheresis, such as an apheresis autologous to the subject from which the tumor fragments and/or T cells were obtained. In other particular embodiments, monocyte-derived dendritic cells are used as a source of APCs and are generated from monocytes from a patient apheresis, such as an apheresis autologous to the subject from which the tumor fragment and/or T cells are obtained.

In some embodiments, the isolated or generated APCs are collected and are cryofrozen. The provision of an intermediate hold step by cryopreservation after the isolation or generation of APCs can be used to coordinate timing with the neoepitope identification and peptide generation such as described in Section I.A and/or initial expansion of T cells, such as described in Section I.B. In some embodiments, for cryopreservation, the isolated or generated APCs are formulated as a composition with a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to -150°C, such as or about 80 °C ± 6.0 ° C.

In some embodiments, the cryopreserved cells are prepared for subsequent steps by thawing. In some cases, the cells can be ready for subsequent culturing with T cells and peptides immediately after thawing following one or more wash steps.

In particular embodiments, the methods for enriching or selecting tumor reactive cells are initiated by contacting PBMCs with the mutated amino acid sequence, such as one or more, such as a plurality of, neoepitope peptides. The PBMCs/peptides can then be cultured with stimulated T cells. The PBMCs and T cells can be obtained from the same subject.

In particular embodiments, the methods for enriching or selecting tumor reactive cells are initiated by contacting B cells with the mutated amino acid sequence, such as one or more, such as a plurality of, neoepitope peptides. The B cell/peptides can then be cultured with stimulated T cells. The B cells and T cells can be obtained from the same subj ect.

In particular embodiments, the methods for enriching or selecting tumor reactive cells are initiated by contacting monocyte-derived DCs with the mutated amino acid sequence, such as one or more, such as a plurality of, neoepitope peptides. The monocyte-derived DCs/peptides can then be cultured with stimulated T cells. The monocyte-derived DCs and T cells can be obtained or derived from the same subject.

In some embodiments, the APC is an artificial antigen presenting cell (aAPC). Typically, aAPCs include features of natural APCs, including expression of an MHC molecule, stimulatory and costimulatory molecule(s), Fc receptor, adhesion molecule(s) and/or the ability to produce or secrete cytokines (e.g. IL-2). Normally, an aAPC is a cell line that lacks expression of one or more of the above, and is generated by introduction (e.g. by transfection or transduction) of one or more of the missing elements from among an MHC molecule, a low affinity Fc receptor (CD32), a high affinity Fc receptor (CD64), one or more of a co-stimulatory signal (e.g. CD7, B7-1 (CD80), B7-2 (CD86), PD-L1, PD-L2, 4-1BBL, OX40L, ICOS-L, ICAM, CD30L, CD40, CD70, CD83, HLA-G, MICA, MICB, HVEM, lymphotoxin beta receptor, ILT3, ILT4, 3/TR6 or a ligand of B7-H3; or an antibody that specifically binds to CD27, CD28, 4-1BB, OX40, CD30, CD40, PD-1, ICOS, LFA-1, CD2, CD7, LIGHT, NKG2C, B7-H3, Toll ligand receptor or a ligand of CD83), a cell adhesion molecule (e.g. ICAM-1 or LFA-3) and/or a cytokine (e.g. IL-2, IL-4, IL-6, IL-7, IL-10, IL-12, IL-15, IL-21, interferon-alpha (IFNα), interferon-beta (IFNβ), interferon-gamma (IPNγ), tumor necrosis factor-alpha (TNFα), tumor necrosis factor-beta (TNFβ), granulocyte macrophage colony stimulating factor (GM-CSF), and granulocyte colony stimulating factor (GCSF)). In some cases, an aAPC does not normally express an MHC molecule, but can be engineered to express an MHC molecule or, in some cases, is or can be induced to express an MHC molecule, such as by stimulation with cytokines. In some cases, aAPCs also can be loaded with a stimulatory or co-stimulatory ligand, which can include, for example, an anti-CD3 antibody, an anti-CD28 antibody or an anti-CD2 antibody. Exemplary of a cell line that can be used as a backbone for generating an aAPC is a K562 cell line or fibroblast cell line. Various aAPCs are known in the art, see e.g., U.S. Patent No. 8,722,400, published application No. US2014/0212446; Butler and Hirano (2014) Immunol Rev., 257(1): 10. 1111/imr. 12129; Suhoshki et al. (2007) Mol. Ther., 15:981-988). In particular embodiments, the methods for enriching or selecting tumor reactive cells are initiated by contacting aAPCs with the mutated amino acid sequence, such as one or more, such as a plurality of, neoepitope peptides. The aAPC/peptides can then be cultured with stimulated T cells.

Inducing APCs (e.g. B cells or monocyte-derived DCs) to present the mutated amino acid sequence may be carried out using various suitable methods. In an embodiment, inducing APCs to present the mutated amino acid sequence (e.g. peptide neoepitope) comprises pulsing the APCs with synthetic peptides comprising the mutated amino acid sequence or a pool of peptides, each peptide in the pool comprising a different mutated amino acid sequence. In some cases, the APCs are pulsed with the peptides using electroporation into an antigen presenting cell. The synthetic peptides can then be presented by the antigen presenting cells to be recognized by CD8 cells (MHC class I) or CD4 cells (MHC class II). In certain particular embodiments, synthetic peptides are generated to be suitable for expression by MHC class I restricted molecules for recognition by CD8 cells. In other particular embodiments, synthetic peptides are generated to be suitable for expression by MHC class II restricted molecules for recognition by CD4 cells.

In some embodiments, the APCs (e.g. PBMCs, B cells or monocyte-derived DCs) are contacted with a single peptide or a pool of peptides. The pool of peptide can represent many different mutated amino acid sequences, such as 5, 10, 20, 25, 30, 40, 50, 60, 70, 80, 90, 100, 150, 200, 300, 400, 500, 600, 700, 800, 900 or 100 peptides, or any value between any of the foregoing.

The peptides or pool of peptides are loaded onto antigen presenting cells (e.g. dendritic cells), such as by peptide pulsing, at a concentrations suitable for their presentation on the surface of a major histocompatibility complex (MHC).

In some embodiments, the peptide concentration representing an individual or single peptide can range between at or about 0.00000 1 µg/mL and at or about 10 µg/mL. In some embodiments, the peptide concentration representing an individual or single peptide can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration representing an individual or single peptide can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the peptides are a pool of peptides representing many different mutated amino acid sequences and the concentration on average of individual or single peptides in the pool can range between at or about 0.00000 1 µg/mL and at or about 10 µg/mL. In some embodiments, the peptides are a pool of peptides representing many different mutated amino acid sequences and the concentration on average of individual or single peptides in the pool can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration on average of individual or single peptides in the pool can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the concentration of individual peptides of the one or more non-native peptide is, on average, less than 0.02 µg/mL. In some embodiments, the concentration of individual peptides of the one or more non-native peptides is, on average, from at or about 0.00001 µg/mL to at or about 0.01 µg/mL, such as at or about 0.00001 µg/mL to at or about 0.005 µg/mL, at or about 0.00001 µg/mL to at or about 0.002 µg/mL, at or about 0.00001 µg/mL to at or about 0.001 µg/mL, at or about 0.00001 µg/mL to at or about 0.0005 µg/mL, at or about 0.00001 µg/mL to at or about 0.0002 µg/mL, at or about 0.00001 µg/mL to at or about 0.0001 µg/mL, at or about 0.00001 µg/mL to at or about 0.00005 µg/mL, at or about 0.00001 µg/mL to at or about 0.00002 µg/mL, at or about 0.00002 µg/mL to at or about 0.005 µg/mL, at or about 0.00002 µg/mL to at or about 0.002 µg/mL, at or about 0.00002 µg/mL to at or about 0.001 µg/mL, at or about 0.00002 µg/mL to at or about 0.0005 µg/mL, at or about 0.00002 µg/mL to at or about 0.0002 µg/mL, at or about 0.00002 µg/mL to at or about 0.0001 µg/mL, at or about 0.00002 µg/mL to at or about 0.00005 µg/mL, at or about 0.00005 µg/mL to at or about 0.005 µg/mL, at or about 0.00005 µg/mL to at or about 0.002 µg/mL, at or about 0.00005 µg/mL to at or about 0.001 µg/mL, at or about 0.00005 µg/mL to at or about 0.0005 µg/mL, at or about 0.00005 µg/mL to at or about 0.0002 µg/mL, at or about 0.00005 µg/mL to at or about 0.0001 µg/mL, at or about 0.0001 µg/mL to at or about 0.005 µg/mL, at or about 0.0001 µg/mL to at or about 0.002 µg/mL, at or about 0.0001 µg/mL to at or about 0.001 µg/mL, at or about 0.0001 µg/mL to at or about 0.0005 µg/mL, at or about 0.0001 µg/mL to at or about 0.0002 µg/mL, at or about 0.0005 µg/mL to at or about 0.005 µg/mL, at or about 0.0005 µg/mL to at or about 0.002 µg/mL, at or about 0.0005 µg/mL to at or about 0.001 µg/mL, at or about 0.001 µg/mL to at or about 0.005 µg/mL, at or about 0.001 µg/mL to at or about 0.002 µg/mL, or at or about 0.002 µg/mL to at or about 0.005 µg/mL.

In some embodiments, the peptide concentration, representing the single peptide or pool of peptides, can range between at or about 0.0001 µg/mL and at or about 40 µg/mL. The peptide concentration, representing the single peptide or pool of peptides, can range between at or about 0.001 µg/mL and at or about 40 µg/mL, at or about 0.001 µg/mL and at or about 25 µg/mL, 0.001 µg/mL and at or about 10 µg/mL, 0.001 µg/mL and at or about 5 µg/mL, 0.001 µg/mL and at or about 1 µg/mL , 0.001 µg/mL and at or about 0.5 µg/mL, 0.001 µg/mL and at or about 0.1 µg/mL, 0.001 µg/mL and at or about 0.01 µg/mL, 0.01 µg/mL and at or about 40 µg/mL, such as at or about 0.01 µg/mL and at or about 25 µg/mL, at or about 0.01 µg/mL and at or about 10 µg/mL, at or about 0.01 µg/mL and at or about 5 µg/mL, at or about 0.01 µg/mL and at or about 1 µg/mL, at or about 0.01 µg/mL and at or about 0.5 µg/mL, at or about 0.01 µg/mL and at or about 0.1 µg/mL, at or about 0.01 µg/mL and at or about 0.05 µg/mL, 0.05 µg/mL and at or about 40 µg/mL, at or about 0.05 µg/mL and at or about 25 µg/mL, at or about 0.05 µg/mL and at or about 10 µg/mL, at or about 0.05 µg/mL and at or about 5 µg/mL, at or about 0.05 µg/mL and at or about 1 µg/mL, at or about 0.05 µg/mL and at or about 0.5 µg/mL, at or about 0.05 µg/mL and at or about 0.1 µg/mL, 0.1 µg/mL and at or about 40 µg/mL, such as at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL and at or about 40 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL and at or about 40 µg/mL, at or about 1 µg/mL and at or about 25 µg/mL, at or about 1 µg/mL and at or about 10 µg/mL, at or about 1 µg/mL and at or about 5 µg/mL, 5 µg/mL and at or about 40 µg/mL, at or about 5 µg/mL and at or about 25 µg/mL, at or about 5 µg/mL and at or about 10 µg/mL, 10 µg/mL and at or about 40 µg/mL, at or about 10 µg/mL and at or about 25 µg/mL, or at or about 25 µg/mL and at or about 40 µg/mL. In some embodiments, the peptide concentration, representing the single peptide or pool of peptides can be at or about 0.0001 µg/mL, at or about 0.001 µg/mL, at or about 0.01 µg/mL, at or about 0.1 µg/mL, at or about 1 µg/mL, at or about 10 µg/mL, at or about 20 µg/mL, at or about 30 µg/mL or at or about 40 µg/mL or any value between any of the foregoing. In some embodiments, the peptide concentrations is the concentration of a pool of peptides. In some embodiments, the peptide concentration is a concentration of a single or individual peptide.

In an embodiment, inducing APCs (e.g. B cells or monocyte-derived DCs) to present the mutated amino acid sequence comprises introducing a nucleotide sequence encoding the mutated amino acid sequence into the APCs. The nucleotide sequence is introduced into the APCs so that the APCs express and display the mutated amino acid sequence, bound to an MHC molecule, on the cell membrane. The nucleotide sequence encoding the mutated amino acid may be RNA or DNA. Introducing a nucleotide sequence into APCs may be carried out in any of a variety of different ways. Non-limiting examples of techniques that are useful for introducing a nucleotide sequence into APCs include transformation, transduction, transfection, and electroporation. In some cases, peptides for binding MHC class II restricted molecules are presented as a gene encoding DNA of the mutation and electroporated into the antigen presenting cell. This DNA will then be in-vitro transcribed into RNA encoding peptides on the surface for recognition by CD4+ cells. In some cases, Tandem Mini Gene methods can be employed to do this for MHC class II restricted molecules, see e.g. published PCT Patent Application Number WO2016/053338 and Parkhurst et al. (2016) Clin Cancer Res., 23:2491-505. In an embodiment in which more than one gene is identified, the method may comprise preparing more than one nucleotide sequence, each encoding a mutated amino acid sequence encoded by a different gene, and introducing each nucleotide sequence into a different population of APCs. In this regard, multiple populations of APCs, each population expressing and displaying a different mutated amino acid sequence, may be obtained. For example, in the case where tandem minigenes are used, APCs (e.g. B cells or monocyte-derived DCs) are electroporated with a mixture of DNA (plurality of DNA) encoding a different mutated amino acid sequences, which will then be in-vitro transcribed into RNA encoding peptides for surface recognition by CD4+ T cells. In some embodiments, APCs (e.g. B cells or monocyte-derived DCs) are electroporated using the Lonza 4D Nucleofector continuous electroporation system.

The methods include adding T cells (e.g. from patient having a tumor) with the culture of APCs presenting the peptides and co-culturing the APCs and T cells for a period of time to allow presentation and recognition of the peptide on the surface of APCs by one or more T cells in the population. In provided embodiments, the T cells include a population of the stimulated T cells.

In some embodiments, for peptide pulsing APCs (e.g. B cells or monocyte-derived DCs) are incubated with peptides for between at or about 2 hours and at or about 48 hours, such as between at or about 2 hours and at or about 36 hours, between at or about 2 hours and at or about 24 hours, between at or about 2 hours and at or about 24 hours, between at or about 2 hours and at or about 18 hours, between at or about 2 hours and at or about 12 hours, between at or about 2 hours and at or about 6 hours, between at or about 6 hours and at or about 48 hours, between at or about 6 hours and at or about 36 hours, between at or about 6 hours and at or about 24 hours, between at or about 6 hours and at or about 24 hours, between at or about 6 hours and at or about 18 hours, between at or about 6 hours and at or about 12 hours, between at or about 12 hours and at or about 48 hours, between at or about 12 hours and at or about 36 hours, between at or about 12 hours and at or about 24 hours, between at or about 12 hours and at or about 18 hours, between at or about 18 hours and at or about 48 hours, between at or about 18 hours and at or about 36 hours, between at or about 18 hours and at or about 24 hours, between at or about 24 hours and at or about 48 hours, between at or about 24 hours and at or about 36 hours, or between at or about 36 hours and at or about 48 hours. In some embodiments, the APCs (e.g. B cells or monocyte-derived DCs) are incubated with peptides for at or about 4 hours, at or about 6 hours, at or about 7 hours, at or about 8 hours, at or about 9 hours, at or about 10 hours, at or about 12 hours, at or about 14 hours, at or about 16 hours, at or about 18 hours, at or about 20 hours, at or about 22 hours, at or about 24 hours, or any value between any of the foregoing. In particular embodiments, the APCs (e.g. PBMCs, B cells or monocyte-derived DCs) are incubated with peptides overnight, such as for between at or about 8 to 12 hours. In some embodiments, the co-culture incubation is for at or about 6 hours.

The T cells (e.g. stimulated T cells) and APCs (e.g. B cells or monocyte-derived DCs) can be present in a culture at a ratio of T cells to APC of 1:100 to 100:1, such as 1:50 to 50:1, 1:25 to 25:1, 1:10 to 10:1, or 1:5 to 5:1. In some embodiments, the ratio of T cells (e.g. stimulated T cells) to APC is at or about 1:100, at or about 1:50, at or about 1:25, at or about 1:10, at or about 1:5, at or about 1:2.5, at or about 1:1, at or about 2:5:1, at or about 5:1, at or about 10:1, at or about 25:1, at or about 50:1 or at or about 100:1, or any value between any of the foregoing. In some embodiments, the ratio of T cells (e.g. stimulated T cells) to APC is between 20:1 and 1:1, between 15:1 and 1:1, between 10:1 and 1:1, between 5:1 and 1:1, or between 2.5:1 and 1:1. In some embodiments, the ratio of T cells (e.g. stimulated T cells) to APC is between 1:20 and 1:1, between 1:15 and 1:1, between 1:10 and 1:1, between 1:5 and 1:1, or between 1:2.5 and 1:1. In particular embodiments, coculture will be performed by mixing the T cells, e.g. population of stimulated T cells, and APC (e.g. B cells or monocyte-derived DC) at approximately a 3:1 ratio. In some embodiments, coculture will be performed by mixing the T cells, e.g. population of stimulated T cells, and APC (e.g. B cells or monocyte-derived DC) at approximately a 1:1 ratio.

In some embodiments, one or more recombinant cytokine for sustaining T cells is added to the co-culture. In some embodiments, the recombinant cytokine can include one or more of IL-2, IL-7, IL-15 or IL-21. In some embodiments, the co-culturing is carried out in the presence of recombinant IL-2, IL-15 and IL-7. In some embodiments, the co-culturing is carried out in the presence of a IL-2. In some embodiments, the co-culturing is carried out in the presence of IL-15 and IL-17, which, in some aspects does not additionally include IL-2.

The recombinant cytokine generally is a recombinant human protein. In particular embodiments, the recombinant cytokine is present in the cell culture medium during the co-culture at a concentration of at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

The co-culture of APCs and T cells can be incubated at a temperature suitable for the presentation of peptides on MHC and the activation of T cells in the culture, for example, at least about 25 degrees Celsius, generally at least about 30 degrees, and generally at or about 37 degrees Celsius. In some embodiments, the incubation is carried out for up to 96 hours. The incubation can be carried out for 24 hours to 96 hours, such as at or about 24 hours, at or about 36 hours, at or about 48 hours, at or about 60 hours, at or about 72 hours, at or about 84 hours or at or about 96 hours, or for a time between any of the foregoing. In particular embodiments, the co-culture is incubated for 24 to 48 hours.

At the end of the co-culturing tumor reactive T cells are separated from APCs present in the co-culture. In some embodiments, the separation can include methods that select away or remove the APCs. In some embodiments, the separation can include methods that positively select or retain the T cells present in the co-culture. In some embodiments, total T cells in the co-culture can be selected. In particular embodiments, tumor reactive T cells or T cells that express one or more upregulation marker, e.g. activation markers, associated with tumor-reactive T cells can be selected.

### D. Selection of Tumor Reactive T cells

In embodiments of the provided methods, the methods involve enrichment or selection of tumor reactive T cells or T cells that are likely or suspected of being tumor reactive T cells containing endogenous TCR that are reactive to peptides present on the APCs by selecting or isolating T cells that are surface positive for one or more T cell upregulation markers expressed on reactive or activated T cells (e.g. activation markers) associated with tumor reactive T cells. In some embodiments, T cells that are surface positive for one or more activation marker is further selected or enriched from the third population of T cells that have been isolated or selected from a biological sample, such as described in Section I.B. 1, in a closed system to produce a fourth population containing the selected T cells. In some embodiments, T cells that are surface positive for one or more activation marker is further selected or enriched from the population of stimulated T cells, such as described in Section I. B.2. In some embodiments, T cells that are surface positive for one or more activation marker is further selected or enriched from a population of T cells after their co-culture with APCs, such as described in Section I.C. In some embodiments, the methods can include a combination of any of the above selections for obtaining or enriching in tumor reactive T cells or T cells that are likely or suspected of being tumor reactive T cells. In some embodiments, the enriched population of cells is used in subsequent processing steps, such as subsequent processing steps involving incubation, stimulation or activation, and/or expansion in accord with one or more steps of any of the provided methods.

In aspects of any of the provided embodiments, tumor-reactive T cells and/or T cells expressing at least one T cell activation marker associated with tumor-reactive T cells are enriched from a biological sample in accord with any of the provided methods. In some aspects, prior to or concurrently with the enrichment, T cells can be enriched or selected from a biological sample, such as based on T cell markers CD3, CD4 or CD8. In some embodiments, enriching for a T cell that is surface positive for one or more cell surface marker includes any method for separation based on such markers. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Prior to the further expansion of T cells from the co-culture, the provided methods further involve enrichment or selection of tumor reactive T cells or T cells that are likely or suspected of being tumor reactive T cells. In some embodiments, such enrichment includes selecting or isolating T cells from the co-culture that are surface positive for one or more T cell activation markers associated with tumor reactive T cells. In some embodiments, T cells selected from the co-culture results in a population of T cells enriched for CD3+ T cells or CD4+ cells and CD8+ cells, that are further positive for one of more of such T cell activation marker. In some embodiments, such cells include or are enriched for tumor-reactive T cells or T cells associated with tumor-reactive T cells. For example, such CD3+ T cells, or CD4⁺ and/or CD8⁺ populations, can be further sorted into sub-populations by positive or negative selection for markers expressed or expressed to a relatively higher degree on tumor-reactive T cells or on T cells having expression of T cell activation markers associated with tumor-reactive T cells. In particular embodiments, the enriched population of cells is cultured under conditions for expansion, such as described in Section I.E.

In some aspects, positive selection is carried out for one or more T cell activation marker. When a T cell is activated by a target or mutant peptide it begins to express upregulation markers such as, but not limited to, CD107, CD107a, CD39, CD103, CD137 (4-1BB), CD59, CD90, CD38, CD30, CD154, CD252, CD134 (OX40), CD258, CD256, PD-1, TIM-3 and/or LAG-3. In particular embodiments, the upregulation marker is CD107, CD107a, CD39, CD137, CD59, CD90, CD38, or CD103. These markers can then be used to select reactive cells. In particular, among T cell activation markers are those that are upregulated and/or whose expression is specifically detected following antigen stimulation of T cells, such that antigen specific effectors can be identified as a surrogate of an antigen that is activating or stimulating the cells. For example, following antigen-induced stimulation, human T-cells undergo dynamic functional and phenotypic changes, including upregulated surface expression of multiple activation-associated molecules, such as CD25, CD69, CD38 and others. The upregulation of surface molecules provides the opportunity to identify and isolate antigen-specific T-cells, such as tumor-reactive T cells, through antibody binding of the upregulated determinant and subsequent enrichment by flow cytometry, including by methods involving magnetic separation and fluorescence-activated cell sorting (FACS).

In some embodiments, the T cell activation marker is selected for any one or more CD107, CD107a, CD39, CD103, CD137 (4-1BB), CD59, CD90, CD38, CD30, CD154, CD252, CD134, CD258, CD256, PD-1, TIM-3 and/or LAG-3. In some embodiments, the T cell activation marker is selected from any one or more of CD107, CD107a, CD39, CD103, CD59, CD90, CD38, CD30, CD154, CD252, CD134, CD258 and/or CD256. In some embodiments, the T cell activation marker is selected from any one or more of CD107a, CD39, CD103, CD59, CD90 and/or CD38.

In some embodiments, the T cell activation marker is or includes CD107a. CD107a is a lysosomal associated protein that is normally found on the T cell Surface. Upon TCR triggering, degranulation of CD8 T cells can occur rapidly, and CD107 and other lysosomal proteins can be transported to the cell membrane to facilitate the release of perforin and granzyme. For example, in some cases CD107 expression can be detected on antigen specific CD8 T cells, such as as early as 30 minutes post-stimulation. (Betts et al. (2003) J. Immunol. Methods 281:6578).

In some embodiments, the T cell activation marker is or includes CD39. In some embodiments, the T cell activation marker is or includes CD103. In some embodiments, the T cell activation marker is or includes CD59. In some embodiments, the T cell activation marker is or includes CD90. In some embodiments, the T cell activation marker is or includes CD38.

In some embodiments, the T cell activation marker is or includes CD137 (41BB). In some embodiments, the T cell activation marker is or includes CD134 (OX40).

In some embodiments, tumor-reactive T cells or T cells associated with tumor-reactive T cells are selected, enriched or isolated based on positive surface expression of at least two or more T cell activation markers, such as at least 3, 4, 5 or 6 T cell activation markers. In some embodiments, the tumor-reactive T cells or T cells associated with tumor-reactive T cells are selected, enriched or isolated based on positive surface expression of two or more of PD-1, TIM-3, LAG-3, CD137, CD107, CD107a, CD39, CD103, CD59, CD90, CD38, CD30, CD154, CD252, CD134, CD258 and/or CD256.

In some embodiments, the at least two T cell activation markers are selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38.

In some embodiments, the tumor-reactive T cells or T cells associated with tumor-reactive T cells are selected, enriched or isolated based on positive surface expression of PD-1, TIM-2, LAG-3 and/or CD137 and at least one other T cell activation marker. In some embodiments, the tumor-reactive T cells or T cells associated with tumor-reactive T cells are selected, enriched or isolated based on positive surface expression of CD137 and at least one other T cell activation marker. In some embodiments, the at least one other T cell activation marker is selected from one or more of PD-1, TIM-3, LAG-3, CD107, CD107a, CD137, CD39, CD103, CD59, CD90, CD38, CD30, CD154, CD252, CD134, CD258 and CD256. In some embodiments, the at least one other T cell activation marker is selected from one or more of CD107, CD107a, CD39, CD103, CD59, CD90, CD38, CD30, CD154, CD252, CD134, CD258 and CD256. In some embodiments, the at least one other T cell activation marker is selected from one or more of CD107a, CD39, CD103, , CD59, CD90 and CD38. In some embodiments, the tumor-reactive T cells or T cells associated with tumor-reactive T cells are selected, enriched or isolated based on positive surface expression of CD107a and CD137, CD38 and CD137, CD103 and CD137, CD59 and CD137, CD90 and CD137 and CD38 and CD137.

In some embodiments, the T cell activation marker includes CD137 (41BB) and CD134 (OX40).

In some embodiments, tumor-reactive T cells are selected using an MHC tetramer bound to a mutation-associated or tumor-associated peptide. In some embodiments, the tetramers are prepared using MHC class I or MHC class II algorithms. In some embodiments, the tetramer is detectably labeled, such as fluorescently labeled. In some embodiments, the tetramer is HLA-matched to the subject from which the source of biological cells is obtained. In some embodiments, selection of cells using an MHC tetramer is directly from a cell source, e.g. peripheral blood, for a sample from a subject. In some embodiments, selection of cells using an MHC tetramer is after selecting or enriching T cells that are surface positive for a T cell activation marker.

Methods of isolating, selecting and/or enriching for cells can be by any of a variety of methods, such as by positive or negative selection based, such as by using any methods as described in Section I.B above. In some embodiments, methods can include immunoaffinity-based selections. In some embodiments, the T cells can be enriched or sorted a variety of ways including, but not limited to, magnetic bead separation, fluorescent cell sorting, and disposable closed cartridge based cell sorters. In particular aspects, one or more T cell activation markers can be used to select reactive cells using, but not limited to, florescent antibodies, nanoparticles or beads on cell selection equipment, but not limited to, the CliniMACS, Sony FX500 or the Tyto cell sorting systems (Miltenyi).

In some embodiments, the selections produces an enriched population of cells, such as a population of cells enriched for CD3+ T cells or CD4+ cells and CD8+ cells, that are further positive for one of more of such T cell activation marker. In some embodiments, such cells include or are enriched for tumor-reactive T cells or T cells associated with tumor-reactive T cells. In some embodiments, the enriched population of cells is used in subsequent processing steps, such as subsequent processing steps involving incubation, stimulation or activation, and/or expansion in accord with one or more steps of any of the provided methods.

In some embodiments, the enriched population of cells are enriched cells from a starting sample as describe above, in which the percentage of cells of a particular phenotype, e.g. tumor-reactive CD3+ T cells or CD3+ T cells surface positive for one or more T cell activation marker, in the enriched population of cells in increased by at least 10%, 20%, 30%, 40%, 50%, 60%, 70%, 80%, 90%, 100%, 500%, 1000%, 5000% or more greater than the percentage of such cells in the starting sample. In some embodiments, the purity of tumor-reactive CD3+ T cells or CD3+ T cells surface positive for one or more T cell activation marker in the enriched composition, i.e. the percentage of cells positive for the selected cell surface marker versus total cells in the population of enriched cells, is at least 90%, 91%, 92%, 93%, 94%, and is generally at least 95%, 96%, 97%, 98%, 99% or greater.

### E. Further Expansion and Harvesting

The T cells from the co-culture, or selected T cells therefrom, are further incubated under conditions to expand the cells *ex vivo* following the co-culture. The incubation is carried out in the presence of one or more T cell stimulatory agent(s) under conditions for stimulating the T cells, such as to expand the T cells in a closed system using serum free medium to produce a composition of expanded T cells for use as a therapeutic cell composition, wherein the one or more second T-cell stimulating agents is or comprises recombinant IL-2. The T cell stimulatory agent(s) can include any as described in Section B.2 above.

In some of any of the provided embodiments, the T cell stimulatory agent(s) is or comprises recombinant IL-2, optionally wherein a further T cell stimulatory agent(s) is selected from an agent that initiates TCR/CD3 intracellular signaling and/or an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include theUHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the T cell stimulatory agent(s) can include adding adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

In general, the culturing and incubations can occur in the presence of recombinant cytokines. In some embodiments, the cytokine is added or is exogenous to the culture media. The culturing is carried out in the presence of recombinant IL-2 and optionally a recombinant cytokine selected from the group consisting of IL-15, IL-7 and IL-21. In some embodiments, the culturing and incubation is carried out in the presence of recombinant IL-2, IL-15 and IL-7. The culturing is carried out in the presence of a IL-2. In some embodiments, the culturing is carried out in the presence of IL-15 and IL-17.

The recombinant cytokine generally is a recombinant human protein. In particular embodiments, the recombinant cytokine is present in the cell culture medium during the incubation at a concentration of at least or at least about 0.5 IU/mL, at least or at least about 1.0 IU/mL, at least or at least about 5 IU/mL, at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

Recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at at or about 8000 IU/mL, between at or about 5000 IU/mL and at or about 7000 IU/mL, between at or about 5000 IU/mL and at or about 6000 IU/mL, between at or about 6000 IU/mL at at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

The sorted or selected T cells can be expanded under the one or more stimulatory conditions in a culture vessel suitable for cell expansion. In some embodiments, the culture vessel is a gas permeable culture vessel, such as a G-Rex system (e.g. G-Rex 10, G-Rex 10M, G-Rex 100 M/100M-CS or G-Rex 500 M/500M-CS). In some embodiments the culture vessel is a microplate, flask, bar or other culture vessel suitable for expansion of cells in a closed system. In some embodiments, expansion can be carried out in a bioreactor. In some embodiments the composition of expanded T cells is removed from a closed system and placed in and/or connected to a bioreactor for expansion. The sorted or selected T cells can be expanded using a cell expansion system by transfer to the cell to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 × 10⁶ cells/mL to 1.5 × 10⁶ cells/mL. In some embodiments, the density is at or about 0.5 × 10⁶ cells/mL, 0.75 × 10⁶ cells/mL, 1 × 10⁶ cells/mL, 1.25 × 10⁶ cells/mL or 1.5 × 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

In some embodiments, expansion is carried out using a Xuri cell expansion system bioreactor. The cells can be seeded at 0.5-1.5 million cells per mL. The cells can be cultured under static or rocking conditions. The medium can be added in bolus or on a perfusion schedule. In embodiments, the bioreactor maintains the temperature at or near 37°C and CO2 levels at or near 5%. The volume of the culture can be maintained at approximately 0.5 L to 1.0 L. In some embodiments, the expansion is carried out for 7-14 days such as 7-10 days. In some aspects, expansion results in a 100 million to 50 billion cells after the expansion and/or in a fold-expansion of 10 to 1000-fold expansion.

In some embodiments, expansion is carried out using a Miltenyi Prodigy bioreactor. The cells can be seeded at 0.5-1.5 million cells per mL. The cells can be cultured under static or shaking conditions. The medium can be added in bolus or on a perfusion schedule. In embodiments, the bioreactor maintains the temperature at or near 37°C and CO2 levels at or near 5%. The volume of the culture can be maintained at approximately 70 mL to 400 mL. In some embodiments, the expansion is carried out for 7-14 days such as 7-10 days. In some aspects, expansion results in a 100 million to 3 billion cells after the expansion and/or in a 10 to 1000-fold expansion.

In some embodiments, expansion is carried out using a gas-permeable bag. The cells can be seeded at 0.5-1.5 million cells per mL. The cells can be cultured under static conditions. In embodiments, the bioreactor maintains the temperature at or near 37°C and CO2 levels at or near 5%. In such aspects, when the cell concentration exceeds 2.0 million cells per mL, medium can be added to bring the cell concentration to between 0.5 and 1.0 million cells per mL. If the volume reaches the maximum volume of the bag the cells would be added to a larger bag or multiple bags for culture under the same conditions. In some embodiments, the expansion is carried out for 7-14 days such as 7-10 days.

The expansion methods are carried out under GMP conditions, including in a closed automated system and using serum free medium. One or more of the steps of the method are be carried out in a closed system or under GMP conditions. In certain embodiments, all process operations are performed in a GMP suite. A closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, the incubation with the T cell stimulatory agent(s) for expansion of tumor-reactive cells is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation with the T cell stimulatory agent(s) for expansion of tumor-reactive cells is carried out for 7-21 days, such as 7 days, 8 days, 9 days, 10 days, 11 days, 12 days, 13 days, 14, days, 15 days, 16 days, 17 days, 18 days, 19 days, 20 days or 21 days, or any value between any of the foregoing. In some embodiments, the incubation is carried out for 7-14 days. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days. In some cases, media can be exchanged daily, every other day, every third day, every 5^{th} day or once a week during the time of the culture or incubation. In some embodiments, the stimulating agents (e.g. cytokines, anti-CD3) are replenished at each media exchange.

In some embodiments, the methods of culturing for expanding cells in accord with any of the provided methods is carried out until a threshold amount of cells, such as tumor-reactive cells or cells positive for one or more T cell activation marker, is obtained. In some embodiments, the method of culturing for expanding cells in accord with any of the provided method is carried out until up to 30 days from the time of enriching the lymphocytes. In some embodiments, the method of culturing for expanding cells in accord with any of the provided method is carried out until up to 20 days after the initiation of the first expansion. In some embodiments, the method of culturing for expanding cells in accord with any of the provided methods is carried out until up to 20 days after initiation of the co-culturing. In some of any of the provided embodiments, harvesting is carried out within 20 days after initiation of the culturing and/or the enriching of T cells comprising tumor-reactive cells. In some of any of the provided embodiments, the cells are harvested 7 to 20 days, 7 to 14 days, 7 to 10 days, 10 to 20 days, 10 to 14 days or 14 to 20 days after the initiation of the culturing. It is understood that reference to the number of days is with reference to days in which the cells are present in a culture and do not include time in which the cells from any one or more of the steps may be stored under conditions for cryopreservation.

In some of any of the provided embodiments, the culturing is carried out until a threshold amount of cells is achieved that is between at or about 0.5 × 10⁸ and at or about 50 × 10⁹ total cells or total viable cells, between at or about 0.5 × 10⁸ and at or about 30 × 10⁹ total cells or total viable cells, between 0.5 × 10⁸ and at or about 12 × 10⁹ total cells or total viable cells, between at or about 0.5 × 10⁸ and at or about 60 × 10⁸ total cells or total viable cells, between at or about 0.5 × 10⁸ and at or about 15 × 10⁸ total cells or total viable cells, between at or about 0.5 × 10⁸ and at or about 8 × 10⁸ total cells or total viable cells, between at or about 0.5 × 10⁸ and at or about 3.5x 10⁸ total cells or total viable cells, between at or about 0.5 × 10⁸ and at or about 1 × 10⁸ total cells or total viable cells, between 1 × 10⁸ and at or about 50 × 10⁹ total cells or total viable cells, between at or about 1 × 10⁸ and at or about 30 × 10⁹ total cells or total viable cells, between 1 × 10⁸ and at or about 12 × 10⁹ total cells or total viable cells, between at or about 1 × 10⁸ and at or about 60 × 10⁸ total cells or total viable cells, between at or about 1 × 10⁸ and at or about 15 × 10⁸ total cells or total viable cells, between at or about 1 × 10⁸ and at or about 8 × 10⁸ total cells or total viable cells, between at or about 1 × 10⁸ and at or about 3.5x 10⁸ total cells or total viable cells, between at or about 3.5 × 10⁸ and at or about 50 × 10⁹ total cells or total viable cells, between at or about 3.5 × 10⁸ and at or about 30 × 10⁹ total cells or total viable cells, between at or about 3.5 × 10⁸ and at or about 12 × 10⁹ total cells or total viable cells, between at or about 3.5 × 10⁸ and at or about 60 × 10⁸ total cells or total viable cells, between at or about 3.5 × 10⁸ and at or about 15 × 10⁸ total cells or total viable cells, between at or about 3.5 × 10⁸ and at or about 8 × 10⁸ total cells or total viable cells, between at or about 8 × 10⁸ and at or about 50 × 10⁹ total cells or total viable cells, between at or about 8 × 10⁸ and at or about 30 × 10⁹ total cells or total viable cells, between at or about 8 × 10⁸ and at or about 12 × 10⁹ total cells or total viable cells, between at or about 8 × 10⁸ and at or about 60 × 10⁸ total cells or total viable cells, between at or about 8 × 10⁸ and at or about 15 × 10⁸ total cells or total viable cells, between at or about 15 × 10⁸ and at or about 50 × 10⁹ total cells or total viable cells, between at or about 15 × 10⁸ and at or about 30 × 10⁹ total cells or total viable cells, between at or about 15 × 10⁸ and at or about 12 × 10⁹ total cells or total viable cells, between at or about 15 × 10⁸ and at or about 60 × 10⁸ total cells or total viable cells, between at or about 60 × 10⁸ and at or about 50 × 10⁹ total cells or total viable cells, between at or about 60 × 10⁸ and at or about 30 × 10⁹ total cells or total viable cells, between at or about 60 × 10⁸ and at or about 12 × 10⁹ total cells or total viable cells, between at or about 12 × 10⁹ and at or about 50 × 10⁹ total cells or total viable cells, between at or about 12 × 10⁹ and at or about 30 × 10⁹ total cells or total viable cells, or between at or about 30 × 10⁹ and at or about 60 × 10⁹ total cells or total viable cells, each inclusive.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more.

Upon reaching a therapeutic dose after expansion the product can be concentrated and frozen in cryopreservation medium. Also provided herein are populations of T cells produced by methods described herein and pharmaceutical compositions thereof.

### Compositions and pharmaceutical formulations

We also describecompositions containing expanded T cells such as produced by any of the provided methods. In some disclosures, the compositions contain tumor reactive T cells or T cells containing an endogenous TCR specific to a tumor-associated antigen, e.g. neoantigen. In particular, among the provided compositions are compositions of cells that are enriched for tumor reactive T cells or T cells containing an endogenous TCR specific to a tumor-associated antigen, e.g. neoantigen.

In some disclosures, the composition comprises about 5-99% tumor-reactive T cells or T cells surface positive for the one or more T cell activation marker, or any percentage of such cells between 5 and 99% inclusive. In some disclosures, the composition can include an increased or greater percentages of tumor-reactive CD3+ T cells or of CD3+ T cells surface positive for the one or more T cell activation marker relative to total CD3+ T cells or total cells in the composition compared to the percentage of such tumor-reactive CD3+ T cells or of CD3+ T cells surface positive for the one or more T cell activation marker relative to total CD3+ T cells or total cells naturally present in the subject or biological sample from which the cells were isolated. In some disclosures, the percentage is increased at least or at least about 2-fold, 3-fold, 4-fold, 5-fold, 10-fold, 20-fold, 30-fold, 40-fold, 50-fold, 60-fold, 70-fold, 80-fold, 90-fold, 100-fold, 150-fold, 200-fold or more. In such disclosures, the one or more T cell activation marker can be any as described, such as any one or more of CD107a, CD39, CD103, CD59, CD90 and/or CD38.

In some disclosures, the composition can include at least at or about 20%, at least at or about 30%, at least at or about 40%, at least at or about 50%, at least at or about 60%, at least at or about 65%, at least at or about 70%, at least at or about 75%, at least at or about 80%, at least at or about 81%, at least at or about 82%, at least at or about 83%, at least at or about 84%, at least at or about 85%, at least at or about 86%, at least at or about 87%, at least at or about 88%, at least at or about 89%, at least at or about 90%, at least at or about 91%, at least at or about 92%, at least at or about 93%, at least at or about 94%, at least at or about 95%, at least at or about 96%, at least at or about 97%, at least at or about 98%, at least at or about 99%, or substantially 100% tumor-reactive CD3+ T cells or CD3+ T cells surface positive for one or more activation marker. In some disclosures, the composition comprises more than 30% tumor reactive CD3+ T cells or CD3+ T cells surface positive for one or more activation marker. In some disclosures, the composition comprises more than 40% tumor reactive CD3+ T cells or CD3+ T cells surface positive for one or more activation marker. In some disclosures, the composition comprises more than 50% tumor reactive CD3+ T cells or CD3+ T cells surface positive for one or more activation marker. In some disclosures, the composition comprises more than 60% tumor reactive CD3+ T cells or CD3+ T cells surface positive for one or more activation marker. In some disclosures, the composition comprises more than 70% tumor reactive CD3+ T cells or CD3+ T cells surface positive for one or more activation marker. In some disclosures, the composition comprises more than 80% tumor reactive CD3+ T cells or CD3+ T cells surface positive for one or more activation marker. In some disclosures, the composition comprises more than 90% tumor reactive CD3+ T cells or CD3+ T cells surface positive for one or more activation marker. In such disclosures, the one or more T cell activation marker can be any as described, such as any one or more of CD107a, CD39, CD103, CD59, CD90 and/or CD38. In such disclosures, the one or more T cell activation marker can be CD134 and/or CD137.

In some disclosures, the tumor reactive CD3+ T cells or CD3+ T cells surface positive for one or more activation marker can be present in the composition in a therapeutically effective amount. An effective amount of cells can vary depending on the patient, as well as the type, severity and extent of disease. Thus, a physician can determine what an effective amount is after considering the health of the subject, the extent and severity of disease, and other variables.

In certain disclosures, the number of such cells in the composition is a therapeutically effective amount. In some disclosures, the amount is an amount that reduces the severity, the duration and/or the symptoms associated with cancer, viral infection, microbial infection, or septic shock in an animal. In some disclosures, a therapeutically effective amount is a dose of cells that results in a reduction of the growth or spread of cancer by at least 2.5%, at least 5%, at least 10%, at least 15%, at least 25%, at least 35%, at least 45%, at least 50%, at least 75%, at least 85%, by at least 90%, at least 95%, or at least 99% in a patient or an animal administered a composition described herein relative to the growth or spread of cancer in a patient (or an animal) or a group of patients (or animals) not administered the composition. In some disclosures, a therapeutically effective amount is an amount to result in cytotoxic activity resulting in activity to inhibit or reduce the growth of cancer, viral and microbial cells.

In some disclosures, the composition comprises an amount of tumor reactive CD3+ T cells or CD3+ T cells surface positive for one or more activation marker that is from at or about 10⁵ and at or about 10¹² tumor reactive CD3+ T cells or CD3+ T cells surface positive for one or more activation marker, or from at or about 10⁵ to at or about 10⁸ tumor reactive CD3+ T cells or CD3+ T cells surface positive for one or more activation marker, or from at or about 10⁶ and at or about 10¹² tumor reactive CD3+ T cells or CD3+ T cells surface positive for one or more activation marker, or from at or about 10⁸ and at or about 10¹¹ tumor reactive CD3+ T cells or CD3+ T cells surface positive for one or more activation marker, or from at or about 10⁹ and at or about 10¹⁰ tumor reactive CD3+ T cells or CD3+ T cells surface positive for one or more activation marker. In some disclosures, the composition comprises greater than or greater than at or about 10⁵ tumor reactive CD3+ T cells or CD3+ T cells surface positive for one or more activation marker, at or about 10⁶ tumor reactive CD3+ T cells or CD3+ T cells surface positive for one or more activation marker, at or about 10⁷ tumor reactive CD3+ T cells or CD3+ T cells surface positive for one or more activation marker, at or about 10⁸ tumor reactive CD3+ T cells or CD3+ T cells surface positive for one or more activation marker, at or about 10⁹ tumor reactive CD3+ T cells or CD3+ T cells surface positive for one or more activation marker, at or about10¹⁰ tumor reactive CD3+ T cells or CD3+ T cells surface positive for one or more activation marker, at or about 10¹¹ tumor reactive CD3+ T cells or CD3+ T cells surface positive for one or more activation marker, or at or about 10¹² tumor reactive CD3+ T cells or CD3+ T cells surface positive for one or more activation marker. In some disclosures, such an amount can be administered to a subject having a disease or condition, such as to a cancer patient. In such disclosures, the one or more T cell activation marker can be any as described, such as any one or more of CD107a, CD39, CD103, CD59, CD90 and/or CD38. In some disclosures, the one or more T cell activation marker is CD134 and/or CD137.

In some disclosures, the composition comprises CD3+ T cells as a percentage of total cells in the population that is greater than or greater than about 60%, greater than or greater than about 70%, greater than or greater than about 80%, greater than or greater than about 90% or greater than or greater than about 95%. In some disclosures, the composition contains CD4+ T cells and CD8+ T cells as a percentage of total cells in the population that is greater than or greater than about 60%, greater than or greater than about 70%, greater than or greater than about 80%, greater than or greater than about 90% or greater than or greater than about 95%. In particular disclosures, the composition contains a ratio of CD8+ T cells to CD4+ T cells that is between at or about 1:100 and at or about 100:1, between at or about 1:50 and at or about 50:1, between at or about 1:25 and at or about 25: 1, between at or about 1: 10 and at or about 10:1, between at or about 1:5 and at or about 5:1, or between at or about 1:2.5 and at or about 2.5:1.

In some disclosures, the volume of the composition is at least or at least about 10 mL, 50 mL, 100 mL, 200 mL, 300 mL, 400 mL or 500 mL, such as is from or from about 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL, each inclusive. In some disclosures, the composition has a cell density of at least or at least about 1 × 10⁵ cells/mL, 5 × 10⁵ cells/mL, 1 × 10⁶ cells/mL, 5 × 10⁶ cells/mL, 1 × 10⁷ cells/mL, 5 × 10⁷ cells/mL or 1 × 10⁸ cells/ mL. In some disclosures, the cell density of the composition is between or between about 1 × 10⁵ cells/mL to 1 × 10⁸ cells/mL, 1 × 10⁵ cells/mL to 1 × 10⁷ cells/mL, 1 × 10⁵ cells/mL to 1 × 10⁶ cells/mL, 1 × 10⁶ cells/mL to 1 × 10⁷ cells/mL, 1 × 10⁶ cells/mL to 1 × 10⁸ cells/mL, 1 × 10⁶ cells/mL to 1 × 10⁷ cells/mL or 1 × 10⁷ cells/mL to 1 × 10⁸ cells/mL, each inclusive.

Among the compositions are pharmaceutical compositions and formulations for administration, such as for adoptive cell therapy. In some disclosures, the engineered cells are formulated with a pharmaceutically acceptable carrier.

A pharmaceutically acceptable carrier can include all solvents, dispersion media, coatings, antibacterial and antifungal agents, isotonic and absorption delaying agents, and the like, compatible with pharmaceutical administration (Gennaro, 2000, Remington: The science and practice of pharmacy, Lippincott, Williams & Wilkins, Philadelphia, PA). Examples of such carriers or diluents include, but are not limited to, water, saline, Ringer's solutions, dextrose solution, and 5% human serum albumin. Liposomes and non-aqueous vehicles such as fixed oils may also be used. Supplementary active compounds can also be incorporated into the compositions. The pharmaceutical carrier should be one that is suitable for NK cells, such as a saline solution, a dextrose solution or a solution comprising human serum albumin.

In some disclosures, the pharmaceutically acceptable carrier or vehicle for such compositions is any non-toxic aqueous solution in which the NK cells can be maintained, or remain viable, for a time sufficient to allow administration of live NK cells. For example, the pharmaceutically acceptable carrier or vehicle can be a saline solution or buffered saline solution. The pharmaceutically acceptable carrier or vehicle can also include various bio materials that may increase the efficiency of NK cells. Cell vehicles and carriers can, for example, include polysaccharides such as methylcellulose (M. C. Tate, D. A. Shear, S. W. Hoffman, D. G. Stein, M. C. LaPlaca, Biomaterials 22, 1113, 2001), chitosan (Suh J K F, Matthew H W T. Biomaterials, 21, 2589, 2000; Lahiji A, Sohrabi A, Hungerford D S, et al., J Biomed Mater Res, 51, 586, 2000), N-isopropylacrylamide copolymer P(NIPAM-co-AA) (Y. H. Bae, B. Vernon, C. K. Han, S. W. Kim, J. Control. Release 53, 249, 1998; H. Gappa, M. Baudys, J. J. Koh, S. W. Kim, Y. H. Bae, Tissue Eng. 7, 35, 2001), as well as Poly(oxyethylene)/poly(D,L-lactic acid-co-glycolic acid) (B. Jeong, K. M. Lee, A. Gutowska, Y. H. An, Biomacromolecules 3, 865, 2002), P(PF-co-EG) (Suggs L J, Mikos A G. Cell Trans, 8, 345, 1999), PEO/PEG (Mann B K, Gobin A S, Tsai A T, Schmedlen R H, West J L., Biomaterials, 22, 3045, 2001; Bryant S J, Anseth K S. Biomaterials, 22, 619, 2001), PVA (Chih-Ta Lee, Po-Han Kung and Yu-Der Lee, Carbohydrate Polymers, 61, 348, 2005), collagen (Lee C R, Grodzinsky A J, Spector M., Biomaterials 22, 3145, 2001), alginate (Bouhadir K H, Lee K Y, Alsberg E, Damm K L, Anderson K W, Mooney D J. Biotech Prog 17, 945, 2001; Smidsrd O, Skjak-Braek G., Trends Biotech, 8, 71, 1990).

In some disclosures, the composition is sterile. In some disclosures, isolation or enrichment of the cells is carried out in a closed or sterile environment, for example, to minimize error, user handling and/or contamination. In some disclosures, sterility may be readily accomplished, e.g., by filtration through sterile filtration membranes.

We also describe compositions that are suitable for cryopreserving the provided T cells, including tumor-reactive T cells or T cells surface positive for one or more activation marker. In some disclosures, the composition comprises a cryoprotectant. In some disclosures, the cryoprotectant is or comprises DMSO and/or s glycerol. In some disclosures, compositions formulated for cryopreservation can be stored at low temperatures, such as ultra low temperatures, for example, storage with temperature ranges from -40 °C to -150 °C, such as or about 80 °C ± 6.0 ° C.

In some disclosures, the cryopreserved cells are prepared for administration by thawing. In some cases, the cells can be administered to a subject immediately after thawing. In such a disclosures, the composition is ready-to-use without any further processing. In other cases, the cells are further processed after thawing, such as by resuspension with a pharmaceutically acceptable carrier, incubation with an activating or stimulating agent, or are activated washed and resuspended in a pharmaceutically acceptable buffer prior to administration to a subject.

### Methods of treatment and therapeutic applications

We also describe compositions and methods relating to the provided therapeutic cell compositions described herein for use in treating diseases or conditions in a subject such as a cancer. Such methods and uses include therapeutic methods and uses, for example, involving administration of the therapeutic cells, or compositions containing the same, to a subject having a disease, condition, or disorder. In some cases, the disease or disorder is a tumor or cancer. In some disclosures, the cells or pharmaceutical composition thereof is administered in an effective amount to effect treatment of the disease or disorder. Uses include uses of the cells or pharmaceutical compositions thereof in such methods and treatments, and in the preparation of a medicament in order to carry out such therapeutic methods. In some disclosures, the methods thereby treat the disease or condition or disorder in the subject.

In some disclosures, the methods of treatment comprise administering an effective amount of a composition containing tumor reactive CD3+ T cells or CD3+ T cells surface, which may include T cells surface positive for one or more activation marker. Such compositions can include any as described herein, including compositions produced by the provided methods.

In some disclosures, a subject (e.g. autologous) is administered from at or about 10⁵ to at or about 10¹² CD3+ T cells produced by any of the provided methods, or from at or about 10⁵ to at or about 10⁸ CD3+ T cells produced by any of the provide methods, or from at or about 10⁶ and at or about 10¹² CD3+ T cells produced by any of the provided methods, or from at or about 10⁸ and at or about 10¹¹ CD3+ T cells produced by any of the provided methods, or from at or about 10⁹ and at or about 10¹⁰ CD3+ T cells produced by any of the provided methods. In some disclosures, the therapeutically effective amount for administration comprises greater than or greater than at or about 10⁵ CD3+ T cells produced by any of the provided methods, at or about 10⁶ CD3+ T cells produced by any of the provided methods, at or about 10⁷ CD3+ T cells produced by any of the provided methods, at or about 10⁸ CD3+ T cells produced by any of the provided methods, at or about 10⁹ CD3+ T cells produced by any of the provided methods, at or about 10¹⁰ CD3+ T cells produced by any of the provided methods, at or about 10¹¹ CD3+ T cells produced by any of the provided methods, or at or about 10¹² CD3+ T cells produced by any of the provided methods. In some disclosures, such an amount can be administered to a subject having a disease or condition, such as to a cancer patient. In some disclosures, the number of T cells are administered are viable T cells.

In some disclosures, the methods of treatment comprise administering an effective amount of a composition containing tumor reactive CD3+ T cells or CD3+ T cells surface positive for one or more activation marker. Such compositions can include any as described herein, including compositions produced by the provided methods. In some disclosuresfrom at or about 10⁵ to at or about 10¹² tumor reactive CD3+ T cells or CD3+ T cells surface positive for one or more activation marker, such as any as described, or from at or about 10⁵ to at or about 10⁸ tumor reactive CD3+ T cells or CD3+ T cells surface positive for one or more activation marker, or from at or about 10⁶ and at or about 10¹² tumor reactive CD3+ T cells or CD3+ T cells surface positive for one or more activation marker, or from at or about 10⁸ and at or about 10¹¹ tumor reactive CD3+ T cells or CD3+ T cells surface positive for one or more activation marker, or from at or about 10⁹ and at or about 10¹⁰ tumor reactive CD3+ T cells or CD3+ T cells surface positive for one or more activation marker are administered to the individual. In some disclosures, the therapeutically effective amount for administration comprises greater than or greater than at or about 10⁵ tumor reactive CD3+ T cells or CD3+ T cells surface positive for one or more activation marker, at or about 10⁶ tumor reactive CD3+ T cells or CD3+ T cells surface positive for one or more activation marker, at or about 10⁷ tumor reactive CD3+ T cells or CD3+ T cells surface positive for one or more activation marker, at or about 10⁸ tumor reactive CD3+ T cells or CD3+ T cells surface positive for one or more activation marker, at or about 10⁹ tumor reactive CD3+ T cells or CD3+ T cells surface positive for one or more activation marker, at or about 10¹⁰ tumor reactive CD3+ T cells or CD3+ T cells surface positive for one or more activation marker, at or about 10¹¹ tumor reactive CD3+ T cells or CD3+ T cells surface positive for one or more activation marker, or at or about 10¹² tumor reactive CD3+ T cells or CD3+ T cells surface positive for one or more activation marker. In some disclosures, such an amount can be administered to a subject having a disease or condition, such as to a cancer patient. In some disclosures, the number of T cells are administered are viable T cells.

In some disclosures, the amount is administered as a flat dose. In other disclosures, the amount is administered per kilogram body weight of the subject.

In some disclosures, the composition, such as produced by any of the provided methods or containing tumor-reactive T cells or T cells surface positive for a T cell activation marker, are administered to an individual soon after expansion according to the provided methods. In other disclosures, the expanded T cells, such as expanded tumor-reactive T cells or T cells surface positive for a T cell activation marker, are cryopreserved prior to administration, such as by methods described above. For example, the T cells, such as tumor-reactive T cells or T cells surface positive for a T cell activation marker, can be stored for greater than 6, 12, 18, or 24 months prior to administration to the individual. Such cryopreserved cells can be thawed prior to the administration.

In some disclosures, the provided compositions, such as provided by any of the provided methods or containing tumor-reactive T cells or T cells surface positive for a T cell activation marker, can be administered to a subject by any convenient route including parenteral routes such as subcutaneous, intramuscular, intravenous, and/or epidural routes of administration.

In some disclosures, the compositions, such as provided by any of the provided methods or containing tumor-reactive T cells or T cells surface positive for a T cell activation marker may be administered in a single dose. Such administration may be by injection, e.g., intravenous injection. In some disclosures, tumor-reactive T cells or T cells surface positive for a T cell activation marker may be administered in multiple doses. Dosing may be once, twice, three times, four times, five times, six times, or more than six times per year. Dosing may be once a month, once every two weeks, once a week, or once every other day. Administration of such compositions and cells may continue as long as necessary.

In some disclosures, the subject is administered a lymphodepleting therapy prior to the administration of the dose of cells from a provided compositions, such as produced by any of the provided methods or containing tumor-reactive T cells or T cells surface positive for a T cell activation marker. The lymphodepleting therapy can include administration of fludarabine and/or cyclophosphamide (the active form being referred to as mafosfamide) and combinations thereof. Such methods are described in Gassner et al. (Cancer Immunol Immunother . 201 1 , 60(l):75-85, Muranski, et al, Nat Clin Pract Oncol, 2006 3(12):668-681, Dudley, et al., J Clin Oncol 2008, 26:5233-5239, and Dudley, et al., J Clin Oncol. 2005, 23(10):2346-2357. In some disclosures, the fludarabine is administered at a dosage of 10 mg/kg/day, 15 mg/kg/day, 20 mg/kg/day, 25 mg/kg/day, 30 mg/kg/day, 35 mg/kg/day, 40 mg/kg/day, or 45 mg/kg/day, or a dosage amount between a range of any of the foregoing. In some disclosures, the fludarabine is for 2-7 days, such as for 3-5 days, such as at or about 3 days, at or about 4 days or at or about 5 days. In some disclosures, the cyclophosphamide is administered at a dosage of 100 mg/m2/day, 150 mg/m2/day, 175 mg/m2/day, 200 mg/m2/day, 225 mg/m2/day, 250 mg/m2/day, 275 mg/m2/day, or 300 mg/m2/day. In some disclosures, the cyclophosphamide is administered intravenously (i.e., i.v.). In some disclosures, the cyclophosphamide treatment is for 2-7 days, such as 3-5 days, at or about 3 days, at or about 4 days or at or about 5 days. The lymphodepleting therapy is administered prior to the provided cell compositions. In some disclosures, the lymphodepleting therapy is carried out within a week of the administration of the provided cell compositions, such as 5-7 days prior to the administration of the dose of cells.

The compositions described herein can be used in a method for treating hyperproliferative disorders. In a preferred disclosure, they are for use in treating cancers. In some aspects, the cancer can be a melanoma, ovarian cancer, cervical cancer, lung cancer, bladder cancer, breast cancer, head and neck cancer, renal cell carcinoma, acute myeloid leukemia, colorectal cancer, and sarcoma. In some disclosures, the cancer is a cancer with a high mutational burden. In some disclosures, the cancer is melanoma, lung squamous, lung adenocarcinoma, bladder cancer, lung small cell cancer, esophageal cancer, colorectal cancer, cervical cancer, head and neck cancer, stomach cancer or uterine cancer.

In some disclosures, the cancer is an epithelial cancer. In some disclosures, the cancer is selected from non-small cell lung cancer (NSCLC), CRC, ovarian cancer, breast cancer, esophageal cancer, gastric cancer, pancreatic cancer, cholangiocarcinoma cancer, endometrial cancer. In some disclosures, the breast cancer is HR+/Her2- breast cancer. In some disclosures, the breast cancer is a triple negative breast cancer (TNBC). In some disclosures, the breast cancer is a HER2+ breast cancer.

In some disclosures, the subject has a cancer that is is a hematological tumor. Non- limiting examples of hematological tumors include leukemias, including acute leukemias (such as 1 lq23- positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some disclosures, the subject has a solid tumor cancer. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some disclosures, the cancer is a skin cancer. In particular disclosures, the cancer is a melanoma, such as a cutaneous melanoma. In some disclosures, the cancer is a merkel cell or metastatic cutaneous squamous cell carcinoma (CSCC).

In some disclosures, the tumor is a carcinoma, which is a cancer that develops from epithelial cells or is a cancer of epithelial origin. In some disclosures, the cancer arises from epithelial cells which include, but are not limited to, breast cancer, basal cell carcinoma, adenocarcinoma, gastrointestinal cancer, lip cancer, mouth cancer, esophageal cancer, small bowel cancer and stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that effect epithelial cells throughout the body.

In some disclosures, the subject has a cancer that is a gastrointestinal cancer involving a cancer of the gastrointestinal tract (GI tract), including cancers or the upper or lower digestive tract, or an accessory organ of digestion, such as esophagus, stomach, biliary system, pancreas, small intestine, large intestine, rectum or anus. In some disclosures, the cancer is an espphageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular disclosures, the cancer is a colorectal cancer.

In some disclosures, the cancer is a colorectal cancer. Colorectal cancer (CRC) is a common tumor of increasing incidence, which, in many cases, does not response to checkpoint inhibition or other immunotherapy. This is the case even though such cancers have properties that are associated with response, e.g. a reasonably high mutation rate and well established association of prognosis with level of T cell infiltration.

In some disclosures, the cancer is an ovarian cancer. In some disclosures, the cancer is a triple-negative breast cancer (TNBC).

In some disclosures, the cancer is lung cancer. In some disclosures, the cancer is a breast cancer. In some disclosures, the cancer is a colorectal cancer. In some disclosures, the cancer is pancreatic cancer. In some disclosures, the cancer is a merkel cell cancer. In some disclosures, the cancer is a metastatic cutaneous squamous cell carcinoma (CSCC). In some disclosures, the cancer is a melanoma.

In some disclosures, the subject is one whose cancer is refractory to, and or who has relapsed following treatment with, a checkpoint blockade, such as an anti-PD1 or anti-PD-L1 therapy.

In some disclosures, the subject is the same subject from with the biological sample was obtained for producing the therapeutic cell composition. In some such disclosures, the provided methods of treatment is an adoptive cell therapy with a therapeutic composition containing T cells autologous to the subject.

In some disclosures, the cell compositions provided herein are autologous to the subject to be treated. In such disclosures, the starting cells for expansion are isolated directly from a biological sample from the subject as described herein, in some cases including with enrichment for T cells surface positive for one or more T cell activation marker as described, and cultured under conditions for expansion as provided herein. In some aspects, the biological sample from the subject is or includes a tumor or lymph node sample and such sample tumor and an amount of such tissue is obtained, such as by resection or biospsy (e.g. core needle biopsy or fine-needle aspiration). In some disclosures, following the culturing under conditions for expansion in accord with the provided methods the cells are formulated and optionally cryopreserved for subsequent administration to the same subject for treating the cancer.

In some disclosures, the cell compositions provided herein are allogenic to the subject to be treated. In some aspects, the subject from which the cells are derived or isolated is a healthy subject or is not known to have a disease or conditions, such as a cancer. In such disclosures, the starting cells for expansion are isolated directly from a biological sample from such a subject as described herein, in some cases including with enrichment for T cells surface positive for one or more T cell activation marker as described, and cultured under conditions for expansion as provided herein. In some aspects, the biological sample from the subject is or includes a tumor or lymph node sample and such sample tumor and an amount of such tissue is obtained, such as by resection or biospsy (e.g. core needle biopsy or fine-needle aspiration). In some disclosures, following the culturing under conditions for expansion the cells are formulated and optionally cryopreserved for subsequent administration to the a different subject for treating a cancer in such different subject.

In some disclosures, the provided methods can be carried out with one or more other immunotherapies. In some disclosures, the immunotherapy is an immune modulating agent that is an immune checkpoint inhibitor. In some disclosures, the immune checkpoint inhibitor specifically binds a molecule selected from among CD25, PD-1, PD-L1, PD-L2, CTLA-4, LAG-3, TIM-3, 4-1BB, GITR, CD40, CD40L, OX40, OX40L, CXCR2, B7-H3, B7-H4, BTLA, HVEM, CD28, TIGIT and VISTA. In some disclosures, the immune checkpoint inhibitor is and antibody or antigen-binding fragment, a small molecule or a polypeptide. In some disclosures, the immune checkpoint inhibitor is selected from among nivolumab, pembrolizumab, pidilizumab, MK-3475, BMS-936559, MPDL3280A, ipilimumab, tremelimumab, IMP31, BMS-986016, urelumab, TRX518, dacetuzumab, lucatumumab, SEQ-CD40, CP-870, CP-893, MED16469, MEDI4736, MOXR0916, AMP-224, and MSB001078C, or is an antigen-binding fragment thereof.

In some disclosures, the provided methods include combination therapy of a cell therapy as described and PD-1 or PD-L1 inhibitors. A PD-1 or PD-L1 inhibitor can include binding antibodies, antagonists, or inhibitors (i.e., blockers).

In a disclosure, the PD-I inhibitor is nivolumab (commercially available as OPDIVO from Bristol-Myers Squibb Co.), or biosimilars, antigen-binding fragments, conjugates, or variants thereof. Nivolumab is a fully human IgG4 antibody blocking the PD-I receptor. In a disclosure, the anti-PD-I antibody is an immunoglobulin G4 kappa, anti-(human CD274) antibody. Nivolumab is assigned Chemical Abstracts Service (CAS) registry number 946414-94-4 and is also known as 5C4, BMS-936558, l\tIDX-1106, and ONO-4538. The preparation and properties of nivolumab are described in U.S. Patent No. 8,008,449 and International Patent Publication No. WO 2006/121168.

In another disclosure, the PD-1 inhibitor comprises pembrolizumab ( commercially available as KEYTRUDA from Merck & Co., Inc., Kenilworth, NJ, USA), or antigen-binding fragments, conjugates, or variants thereof. Pembrolizumab is assigned CAS registry number 1374853-91-4 and is also known as lambrolizumab, MK-3475, and SCH-900475. The properties, uses, and preparation of pembrolizumab are described in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2.

In a disclosure, the PD-LI inhibitor is durvalumab, also known as MEDI4736 (which is commercially available from Medimmune, LLC, Gaithersburg, JVIaryland, a subsidiary f AstraZeneca plc.), or antigen-binding fragments, conjugates, or variants thereof. In a disclosure, the PD-LI inhibitor is an antibody disclosed in U.S. Patent No. 8,779,108 or U.S. Patent Application Publication No. 2013/0034559.

In a disclosure, the PD-LI inhibitor is avelumab, also known as MSB0010718C (commercially available from Merck KGaA/EMD Serono), or antigen-binding fragments, conjugates, or variants thereof. The preparation and properties of avelumab are described in U.S. Patent Application Publication No. US 2014/0341917 A1.

In a disclosure, the PD-LI inhibitor is atezolizumab, also known as MPDL3280A or RG7446 (commercially available as TECENTRIQ from Genentech, Inc., a subsidiary of Roche Holding AG, Basel, Switzerland), or antigen-binding fragments, conjugates, or variants thereof. In a disclosure, the PD-LI inhibitor is an antibody disclosed in U.S. Patent No. 8,217,149. In a disclosure, the PD-LI inhibitor is an antibody disclosed in U.S. Patent Application Publication Nos. 2010/0203056 A1, 2013/0045200 A1, 2013/0045201 A1, 2013/0045202 A1, or 2014/0065135 A1. The preparation and properties of atezolizumab are described in U.S. Patent No. 8,217,149.

### II. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

The term "epitope" means a short peptide derived from a protein antigen, wherein the peptide binds to a major histo compatibility complex (MHC) molecule and is recognized in the MHC-bound context by a T cell. The epitope may bind an MHC class I molecule (e.g., HLA-A1 HLA-A2 or HLA-A3) or an MHC class II molecule.

The term "allogeneic" as used herein means a cell or tissue that is removed from one organism and then infused or adoptively transferred into a genetically dissimilar organism of the same species.

The term "autologous" as used herein means a cell or tissue that is removed from the same organism to which it is later infused or adoptively transferred.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD. Among the provided antibodies are antibody fragments.

An "antibody fragment" or "antigen-binding fragment" refers to a molecule other than a conventional or intact antibody that comprises a portion of an conventional or intact antibody containing at least a variable region that binds an antigen. Examples of antibody fragments include but are not limited to Fv, single chain Fvs (sdFvs), Fab, Fab', Fab'-SH, F(ab')₂; diabodies; linear antibodies; an single-domain antibodies comprising only the V_{H} region (VHH).

As used herein, "bind," "bound" or grammatical variations thereof refers to the participation of a molecule in any attractive interaction with another molecule, resulting in a stable association in which the two molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs.

The term "biological sample" means a quantity of a substance from a living thing or formerly living thing. Such substances include, but are not limited to, blood, (for example, whole blood), plasma, serum, urine, amniotic fluid, synovial fluid, endothelial cells, leukocytes, monocytes, other cells, organs, tissues, bone marrow, lymph nodes and spleen.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

The term "concurrently" is used herein to refer to a procedure, such as an incubation, selection, enrichment or administration, involving two or more agents, where at least part of the particular procedure with one agent overlaps in time with at least a second agent.

The term "intermittently" is used herein to refer to a procedure, such as an incubation, selection, enrichment or administration, involving two or more agents, where the particular procedure involving each agent do not occur at regular intervals or are not continuous or stop and start repeatedly with periods in between.

The term "sequentially" is used herein to refer to a procedure, such as an incubation, selection, enrichment or administration, involving two or more agents, where the particular procedure involving each agent do not overlap in time.

As used herein, "isolated" or "purified with reference to a peptide, protein or polypeptide refers to a molecule which is substantially free of all other polypeptides, contaminants, starting reagents or other materials, or substantially free from chemical precursors or other chemicals when chemically synthesized. Preparations can be determined to be substantially free if they appear free of readily detectable impurities as determined by standard methods of analysis, such as high-performance liquid chromatography (HPLC), thin-layer chromatography (TLC) or capillary electrophoresis (CE), used by those of skill in the art to assess such purity, or sufficiently pure such that further purification would not detectably alter the physical and chemical properties of the substance.

As used herein, the term "recombinant" refers to a cell, microorganism, nucleic acid molecule, or vector that has been modified by introduction of an exogenous, such as heterologous, nucleic acid molecule, or refers to a cell or microorganism that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent molecule. The term "recombinant" also can refer to a protein product expressed from such a nucleic acid molecule or vector or from such cell or microorganism to which is introduced or modified with an exogenous nucleic acid.

As used herein, a composition refers to any mixture of two or more products, substances, or compounds, including cells. It may be a solution, a suspension, liquid, powder, a paste, aqueous, non-aqueous or any combination thereof.

As used herein, "optional" or "optionally" means that the subsequently described event or circumstance does or does not occur, and that the description includes instances where said event or circumstance occurs and instances where it does not. For example, an optionally substituted group means that the group is unsubstituted or is substituted.

The term "pharmaceutical composition" refers to a composition suitable for pharmaceutical use in a mammalian subject, often a human. A pharmaceutical composition typically comprises an effective amount of an active agent (e.g., cells, such as expanded in accord with the provided methods) and a carrier, excipient, or diluent. The carrier, excipient, or diluent is typically a pharmaceutically acceptable carrier, excipient or diluent, respectively.

A "pharmaceutically acceptable carrier" refers to a non-toxic solid, semi solid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and are compatible with other ingredients of the formulation. The pharmaceutically acceptable carrier is appropriate for the formulation employed.

Reference to "population of cells" herein is meant to refer to a number of cells that share a common trait. A population of cells generally contains a plurality of cells, such as greater than at or about 100 cells, at or about 1000 cells, and typically range from 1 × 10⁴ to 1 × 10¹⁰ in number.

The term "soluble" as used herein in reference to proteins, means that the protein is not bound, immobilized or attached to a particle, such as a cell or solid support, e.g. a bead. For example, a soluble protein includes a protein that is not bound as a transmembrane protein to the cell membrane of a cell. In some cases, solubility of a protein can be improved by linkage or attachment, directly or indirectly via a linker, to another molecule such as an Fc domain, which, in some cases, also can improve the stability and/or half-life of the protein. In some aspects, a soluble protein is an Fc fusion protein.

The term "specifically binds" as used herein means the ability of a protein, under specific binding conditions, to bind to a target protein such that its affinity or avidity is at least 10 times as great, but optionally 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule but may specifically bind to more than one target molecule. In some cases, a specifically binding protein may bind to a protein that has similarity in structural conformation with the target protein (e.g., paralogs or orthologs). Those of skill will recognize that specific binding to a molecule having the same function in a different species of animal (i.e., ortholog) or to a molecule having a substantially similar epitope as the target molecule (e.g., paralog) is possible and does not detract from the specificity of binding which is determined relative to a statistically valid collection of unique non-targets (e.g., random polypeptides). Solid-phase ELISA immunoassays, ForteBio Octet or Biacore measurements can be used to determine specific binding between two proteins. Generally, interactions between two binding proteins have dissociation constants (Kd) less than about 1×10⁻⁵ M, and often as low as about 1 × 10⁻¹² M. In certain aspects of the present disclosure, interactions between two binding proteins have dissociation constants of less than about 1×10⁻⁶ M, 1×10⁻⁷ M, 1×10⁻⁸ M, 1×10⁻⁹ M, 1×10⁻¹⁰ M, or 1×10⁻¹¹ M or less.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, a "subject" is a mammal, such as a human or other animal, and typically is human. The subject can be male or female and can be any suitable age, including infant, juvenile, adolescent, adult, and geriatric subjects.

The terms "effective amount" or "therapeutically effective amount" refer to a quantity and/or concentration of a therapeutic composition, such as containing cells, e.g. expanded in accord with the provide methods, that when administered to a patient yields any manner in which the symptoms of a condition, disorder or disease or other indication, are ameliorated or otherwise beneficially altered. An effective amount for treating a disease or disorder may be an amount that relieves, lessens, or alleviates at least one symptom or biological response or effect associated with the disease or disorder, prevents progression of the disease or disorder, or improves physical functioning of the patient. In particular aspects, there is a statistically significant inhibition of disease progression as, for example, by ameliorating or eliminating symptoms and/or the cause of the disease. In the case of cell therapy, the effective amount is an effective dose or number of cells administered to a patient. In some embodiments the patient is a human patient.

As used herein, "disease," disorder" or "condition" refers to a pathological condition in an organism resulting from cause or condition including, but not limited to, infections, acquired conditions, genetic conditions, and characterized by identifiable symptoms. In particular, it is a condition where treatment is needed and/or desired.

The terms "treating," "treatment," or "therapy" of a disease or disorder as used herein mean slowing, stopping or reversing the disease or disorders progression, as evidenced by decreasing, cessation or elimination of either clinical or diagnostic symptoms, by administration of an immunomodulatory protein or engineered cells disclosed herein either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. "Preventing," "prophylaxis," or "prevention" of a disease or disorder as used in the context of this disclosure refers to the administration of an immunomodulatory protein or engineered cells expressing an immunomodulatory protein of the present disclosure, either alone or in combination with another compound, to prevent the occurrence or onset of a disease or disorder or some or all of the symptoms of a disease or disorder or to lessen the likelihood of the onset of a disease or disorder. For example, in the context of cancer, the terms "treatment" or, "inhibit," "inhibiting" or "inhibition" of cancer refers to at least one of: a statistically significant decrease in the rate of tumor growth, a cessation of tumor growth, or a reduction in the size, mass, metabolic activity, or volume of the tumor, as measured by standard criteria such as, but not limited to, the Response Evaluation Criteria for Solid Tumors (RECIST), or a statistically significant increase in progression free survival (PFS) or overall survival (OS).

The term "antigen" refers to a molecule that can induce an immune response. Typically, an antigen is a molecule that is capable of being bound by a recognition site on an immune molecule, such as an antibody or T cell receptor if presented by major histocompatibility complex (MHC) molecules. An antigen can have one or more epitopes in which each epitope that is part of the antigen can be bound by a recognition site of an antibody or TCR/MHC complex. In some embodiments, an antigen is capable of inducing a humoral immune response or a cellular immune response leading to the activation of B lymphocytes and/or T lymphocytes.

As used herein, a "tumor-associated antigen" or "tumor-specific antigen" refers to a protein or other molecule that is found only on cancer cells and not on normal cells.

As used herein, "neoantigen" refers to an antigen to which the immune system has not previously been exposed, such as one that arises by alteration of host antigens by viral infection, neoplastic transformation, drug metabolism or other manner. In particular aspects, a neoantigen is an antigen encoded by tumor-specific mutated genes or that is a new antigen that develops in a tumor cell.

The term "in vivo" refers to an event that takes plane in a mammalian subject's body.

The term "ex vivo" refers to an event that takes place on or in a tissue or cells from a mammalian subject but outside of the mammalian subject's body. Typically the event is carried out in an external environment. In particular aspects, an ex vivo procedure includes any in which an organ, cell or tissue is taken from a subject, typically a living body, for a treatment or procedure and then returned to the subject.

The term "in vitro" refers to an event that takes place in a test system, such as in a laboratory.

As used herein, a kit is a packaged combination that optionally includes other elements, such as additional reagents and instructions for use of the combination or elements thereof.

The term "package insert" is used to refer to instructions customarily included in commercial packages of therapeutic products, that contain information about the indications, usage, dosage, administration, combination therapy, contraindications and/or warnings concerning the use of such therapeutic products.

As used herein, an "article of manufacture" is a product that is made and, in some cases, that can be sold. In some embodiments, the term can refer to compositions contained in articles of packaging, such as in a container.

It is understood that aspects and embodiments of the invention described herein include "comprising," "consisting," and "consisting essentially of" aspects and embodiments.

### III. EXAMPLES

The following examples are included for illustrative purposes only and are not intended to limit the scope of the invention.

### Example 1 Assessment of Tumor Processing Methodologies in Obtaining a Population of Tumor-derived T-Cells

Tumors from patients with colorectal cancer (CRC) or melanoma were processed as described below and resultant infiltrating T-cell populations were analyzed for cell count viability.

### A. Colorectal Cancer

Tumors were sourced from primary tumors in patients with colorectal cancer and shipped overnight in HypoThermosol at 4 °C. Tumors were either processed as fragment or single cell suspension (SCS) cultures.

For fragment cultures, tumors were minced into fragments 1 - 8 mm in diameter, and each 1 - 8 mm fragment was placed into a well of culture vessel, either a gas-permeable 24-well culture plate or conventional 6-well plate, in the presence of Roswell Park Memorial Institute (RPMI) containing 5% human serum or serum free OpTmizer medium (ThermoFisher). Media was supplemented with either 300 or 6000 IU/mL recombinant IL-2, and also contained gentamicin at 10 µg/ml, Immune Cell Serum Replacement (ThermoFisher) at between 2 and 5% according to manufacturer's recommendations, and a final concentration of 2.0 mM of a L-alanyl-L-glutamine dipeptide form of glutamine (GlutaMAX Supplement; Thermofisher). Fragment cultures were maintained and monitored visually until a cell count was performed between approximately day 5 and 11 of culture.

For SCS cultures, tumors were also minced into fragments 1 - 8 mm in diameter. Fragments were then homogenized in a closed system using the Miltenyi GentleMACS in the presence or absence of an enzyme to digest the tumor, either an enzyme cocktail from the Miltenyi Tumor Dissociation Kit, human (part 130-095-929) used according to the manufacturer's recommendation, Collagenase I/II blend (Nordmark, Collagenase NB 4G Proved Grade, part: S 1746503) or Collagenase IV (Worthington Biomedical part: LS004130) at 1 mg/ml or 5 mg/ml. Fragments designated for SCS with homogenization and enzyme digestion were incubated with the enzyme cocktail or collagenase for a total of 60 minutes. Immediately following the generation of SCS, cell counts and viability assessments were performed on the NC-200 Automated Cell Counter (ChemoMetec).

As shown in **FIG. 3A**, SCS cultures with or without enzymatic digestion yielded more total viable cells (TVC) than was obtained following culture from CRC tumor fragments. Depicted in **FIG. 3B** the percent of viable cells was similar in cultures generated from fragments or SCS generated by homogenization in the presence of enzymes.

### B. Melanoma

Tumors were sourced from primary tumors in patients with melanoma and shipped overnight in HypoThermosol at 4 °C. Cells were cultured similarly as described above.

Briefly, for fragment cultures tumors were minced into fragments 1 - 8 mm in diameter, and each 1 - 8 mm fragment was cultured in a well of a gas-permeable 24-well culture plate or conventional 6-well plate in the presence of RPMI containing 5% human serum or serum free OpTmizer medium supplemented with recombinant IL-2 at a concentration of 300 lU/ml or 6000 IU/ml. The media also contained gentamicin at 10 µg/ml, and a final concentration of 2.0 mM of a L-alanyl-L-glutamine dipeptide form of glutamine (GlutaMAX Supplement; Thermofisher). Fragment cultures were maintained and monitored visually until a cell count was performed between day 5 and 9 of culture.

To generate SCS cultures, tumor fragments were homogenized using the Miltenyi GentleMACS in the presence or absence of an enzyme, which included Collagenase IV at a concentration of 1 mg/mL or 5 mg/mL or Collagenase I/II blend at 1 mg/mL or 5 mg/mL (Nordmark, Collagenase NB 4G Proved Grade, part: S1746503).. As above, cell count and viability assessments were performed immediately following the generation of SCS using the NC-200 Automated Cell Counter (Chemometec).

Depicted in **FIG. 4A**, cultures generated from melanoma tumor fragments yielded more total viable cells than SCS generated by homogenization and dissociation with enzymes. As shown in **FIG. 4B**, fragment cultures also had a higher percent viable cells than cells in SCS cultures, irrespective of enzymatic homogenization.

### Example 2 Assessment of T Cell Expansion Kinetics of Tumor-Derived Cells

Tumors were processed as described in Example 1 to generate 1 - 8 mm in diameter fragments or SCS cultures, which were then incubated under conditions to expand T cell populations present within the tumor. Cultures were grown under various tested conditions in the presence of recombinant IL-2 as described below in order to assess cellular expansion. Among the conditions that were tested were the effect of the type of culture plate, culture media, and concentration of IL-2 on cell expansion.

### A. Culture Conditions

Single cell suspensions (SCSs) obtained by homogenization and enzyme digestion of primary tumors from donor patients with CRC or melanoma, as described in Example 1, were cultured in a conventional 6-well plate or a gas-permeable 24-well culture plate. Where possible, multiple conditions from each donor were initiated and averaged (error bars represent ± standard deviation). For 6-well plates, cells were seeded between 250,000 and 1,000,000 cells/mL and for gas-permeable 24-well plates cells were seeded between 5,000 and 750,000 cells/mL. In both cases, the cells were seeded in either RPMI containing 5% human serum or serum free OpTmizer medium with recombinant IL-2 supplemented at a concentration of IL-2 of 300 IU/mL or 6000 IU/mL. The media also contained gentamicin at 10 µg/ml and a final concentration of 2.0 mM of a L-alanyl-L-glutamine dipeptide form of glutamine (GlutaMAX Supplement; Thermofisher). The cells were incubated for up to 31 days, typically 14 to 21 days, wherein 50% of cellular media was exchanged every other day beginning on the 5th day of culture.

For expansion from tumor fragments, an individual 1 - 8 mm tumor fragment, obtained as described in Example 1 from primary tumors of donor patients with CRC or melanoma, was placed in a well of a gas-permeable 24-well culture plate or a 6-well plate, and cultured in either RPMI containing 5% human serum or serum free OpTmizer medium with recombinant IL-2 supplemented at a concentration of 300 IU/mL or 6000 IU/mL. The media also contained gentamicin at 10 µg/ml, and a final concentration of 2.0 mM of a L-alanyl-L-glutamine dipeptide form of glutamine (GlutaMAX Supplement; Thermofisher). The cells were incubated for up to 31 days, such as typically 14 to 21 days, wherein 50% of cellular media was exchanged every other day beginning on the 5th day of culture.

For all conditions, cell counts were performed approximately every other day using the NC-200 Automated Cell Counter (Chemometec) and samples were collected for fluorescence-activated cell sorting (FACS). After completion of the expansion phase (e.g. day 14 - 31), cells were washed in PBS then cryopreserved in the presence of a cryoprotectant. Cryopreservation was carried out using CoolCell devices (Corning) or the VIA Freeze (GE Healthcare).

### B. Results

### 1. Growth Curves

Growth curves of expansion of SCS obtained from tumor fragments from CRC donor patients following expansion culture in different culture vessels are shown in **FIGS. 5A and 5B****.** The results shown are from cultures incubated with recombinant IL-2 at both 300 IU/mL or 6000 IU/mL concentrations, and in either media type, but are separated based on the source of starting cells. As shown, it was possible to expand tumor-derived cells from SCS from tumors of CRC donors under these conditions. In some donors greater than 2-fold and even as high as 10-fold or more expansion was observed in this initial expansion phase of cells obtained directly from CRC tumors.

Expansion achieved from SCS compared to tumor fragments from CRC tumor biopsy products were assessed. As shown in **FIGS. 5C and 5D**, it was possible to expand cells from CRC tumors under these conditions whether extracted and cultured as fragments or as SCS. However, in general, a greater expansion was achieved in CRC cultures extracted as SCS, as evidenced by higher total cell numbers (**FIG. 5C**) and fold expansion (**FIG. 5D**) compared to culturing cells extracted via fragments.

Growth curves of expansion of cells cultured as extracted tumor fragments, or as SCSs from tumor fragments, from different melanoma donors following expansion culture in different culture vessels are shown in **FIGS. 6A and 6B****.** The results shown are from cultures incubated with recombinant IL-2 at both 300 IU/mL or 6000 IU/mL concentrations, and in either media type, but are separated based on the source of starting cells. As shown substantial expansion was observed in melanoma cultures extracted as tumor fragments in either culture vessel, whereas less expansion was observed for melanoma cells cultured as SCS.

Consistent with previous observations, tumor cells from certain donors were not amenable to expansion, irrespective of tumor type, indicative of inherent variability in expansion potential between donors and furthermore between tumor fragments of the same donor tumor. Larger scale methods in which tumor fragments from a donor patient are pooled during culture would be expected to mitigate against intra-tumor variability by combining tumor fragments from the same donor tumor.

### 2. Growth Assessment by Cellular Media

Expanded cultures generated as described above in RPMI media containing either 5% human serum or a serum replacement formulation (OpTmizer media) were compared after expansion for between 14 and 21 days. The results shown are from cultures incubated with recombinant IL-2 at both 300 IU/mL or 6000 IU/mL concentrations, and in either type of culture vessel, but are separated based on the type of media. The results for CRC tumors are from culture of SCS obtained from tumor fragments (**FIG. 7A and 7B**), while the results from melanoma tumors are from culture of tumor fragments (**FIG. 8A and 8B**).

For both tumor types, an increase in total cell number (**FIG. 7A** **and** **FIG. 8A**) and fold-expansion (**FIG. 7B** **and** **FIG. 8B**) was observed by culture in either the 5% human serum or serum replacement media for both tumor types. In the samples tested, there was a trend to improved expansion using OpTmizer media, as evidenced by higher overall cell number at the end of the initial expansion phase (**FIG. 7A** **and** **FIG. 8A**).

### 3. IL-2 Concentration

The effect of different IL-2 concentrations during the expansions from the different tumor types was compared. The cultures were expanded as described above in RPMI media containing either 5% human serum or a serum replacement formulation (OpTmizer media) for between 14 and 21 days, in either 300 IU/mL or 6000 IU/mL recombinant IL-2. The results shown are from cultures incubated in the presence of either media type, and in either type of culture vessel, but are separated based on the IL-2 concentration. The results for CRC tumors are from culture of SCS obtained from tumor fragments (**FIG. 9A and 9B**), while the results from melanoma tumors are from culture of tumor fragments (**FIG. 10A and 10B**).

For both tumor types, the results showed similar expansion of cells grown in either a high or low concentration of IL-2, as evidenced by similar total cell numbers after expansion (**FIG. 9A** **and** **FIG. 10A**) as well as fold expansion (**FIG. 9B** **and** **FIG. 10B**). These data support the observation that doses of IL-2 of about 300 IU/mL support expansion, and that a high dose of IL-2, such as 6000 IU/mL, is not necessary for cellular expansion for either CRC or melanoma cultures.

Together, the results show that while expansion can be donor and moreover, tumor sample dependent, CRC tumor infiltrating T-cells were grown successfully from SCS cultures and melanoma infiltrating T-cells from fragment cultures across multiple donors. It was similarly observed that for both melanoma and CRC derived T-cell cultures, the addition of high concentrations of IL-2 did not result in an appreciably distinct expansion response when compared with a lower dose.

### Example 3 Assessment of Anti-CD3 Stimulation on Expansion of Tumor-Derived Cells

Cells processed from melanoma tumor fragments as described in Examples 1 and 2, were cultured in the presence or absence of 50 ng/mL OKT3, a human anti-CD3 monoclonal antibody. Cell cultures were carried out to between 14 and 31 days in either a conventional 6-well plate or a gas permeable culture plate with RPMI or OpTmizer media. The cultures also were supplemented with 300 or 6000 IU/mL recombinant IL-2, 10 µg/mL gentamicin and a final concentration of 2.0 mM of a L-alanyl-L-glutamine dipeptide form of glutamine (GlutaMAX Supplement; Thermofisher). About 50% of cellular media was exchanged every other day beginning on the 5th day of culture as described previously. Cells were then counted using the NC-200 Automated Cell Counter (Chemometec).

The results shown are from cultures incubated with recombinant IL-2 at both 300 IU/mL or 6000 IU/mL concentrations, different medias, and in either type of culture vessel, but are separated based on the presence of absence of anti-CD3 stimulation. Cells from donor 6 were tested in both the presence or absence of anti-CD3 stimulation, and demonstrated 2-4 fold expansion in all conditions, with a 13-fold expansion observed in OpTmizer media supplemented with 300 IU/mL IL-2 with incubation in the absence of anti-CD3 stimulation (-OKT3). The results shown in **FIGS. 11A-11B** demonstrate that CD3 stimulation via OKT3 supported T cell expansion, but did not substantially impact total cell number (**FIG. 11A**) or fold expansion (**FIG. 11B**). These data are consistent with a finding that anti-CD3 stimulation (e.g. via OKT3) may not be necessary for the expansion of cells from tumor cultures.

### Example 4 Assessment of Post-Stimulation CD4+ and CD8+ Activation Markers

T-cells from three healthy donors were thawed, rested overnight in OpTmizer media supplemented with 300 IU/mL recombinant IL-2, and then activated using 50 ng/mL OKT3, a human anti-CD3 monoclonal antibody. Specific markers of activation on the CD4+ and CD8+ cell populations were measured using flow cytometry over a time course of 3 - 48 hours. Specifically, the following markers were assessed: CD38 and CD39 (**FIG. 12A** **and** **FIG. 13A**), CD134 and CD137 (**FIG. 12B** **and** **FIG. 13B**), and CD69 and CD90 (**FIG. 12C** **and** **FIG. 13C**).

Results for expression of activation markers on the surface of CD8 cells are shown in **FIGS. 12A-12C**, which demonstrates the kinetics of upregulation of markers on CD8+ T cells in the 48 hours following stimulation with OKT3 compared to culture in the absence of OKT3. In some cases, some basal level of the markers can be seen on day 0 before stimulation. As shown, all assessed markers were upregulated to some extent during this time course, with the highest percentage of cells being upregulated for markers CD38 (**FIG. 12A**), CD134 (**FIG. 12B**) and CD69 (**FIG. 12C**) during this study.

Results for expression of activation markers on the surface of CD4 cells are shown in **FIGS. 13A-13C****,** which demonstrates the kinetics of upregulation of markers on CD4+ T cells in the first 48 hours following stimulation with OKT3 compared to the culture in the absence of OKT3 . As shown, all assessed markers were upregulated to some extent during this time course, with the highest percentage of cells being upregulated for markers CD38 (**FIG. 13A**), CD137 (**FIG. 13B**) and CD69 (**FIG. 13C**) during this study.

Taken together, these data support that expression of the above markers can be used as upregulation markers to select for T cells that have been activated, including under conditions of activation that would be expected to stimulate signaling via the TCR-CD3 complex as would occur following co-culture with antigen presenting cells presenting neoantigenic peptides.

### Example 5 Determination of Donor Cell Phenotype and Cellular Viability

T cells were sourced from primary tumors in patients with melanoma or CRC as described in Example 1. Cells from the tumors were extracted either as tumor fragments or as a SCS as described in Example 1, and then were assessed for T cell phenotype by flow cytometry.

For tumor fragments, each 1 - 8 mm fragment was placed into a well of culture vessel, either a gas-permeable 24-well culture plate or conventional 6-well plate, and incubated for between 5 and 11 days in the presence of RPMI containing 5% human serum or serum free OpTmizer medium (ThermoFisher). Media was supplemented with either 300 or 6000 IU/mL recombinant IL-2, and also contained gentamicin at 10 µg/ml, and a final concentration of 2.0 mM of a L-alanyl-L-glutamine dipeptide form of glutamine (GlutaMAX Supplement; Thermofisher). The incubation also was carried out with or without 50 ng/mL of anti-CD3 antibody OKT3. Fragment cultures were monitored visually until it was determined that a cell count could be performed (typically between day 5 and 9 of culture), and then cells were stained ad analyzed by flow cytometry for T cell markers.

Alternatively, for SCS cultures, tumors were minced into fragments 1 - 8 mm in diameter, then homogenized in the presence or absence of Collagenase IV (Worthington Biomedical part: LS004130) at 1 mg/ml or 5 mg/ml or Collagenase NB4G Proved Grade (Nordmark Biomedicals; Catalog No. S1746503) at 1 mg/mL. After incubation with enzyme for about 90 minutes, cells were immediately stained and analyzed by flow cytometry for T cell markers.

The gating hierarchies were designed as follows: first, the percentage of CD3+ cells from a parent population of total cell events were recorded, followed by the percentage of viable CD4+ cells from the CD3+ parent populations and next the percentage of viable CD8+ cells from the same parent CD3+ population. The memory T-cell populations (Tern) were then calculated based on their respective CD4+ and CD8+ parent populations. Thus, CD4/Tem was determined from the parent population of viable CD4+ cells, while CD8/Tem was determined from the parent populations of viable CD8+ cells. Thus, the results, as depicted in **FIG. 12**, are the percentage of CD3+ cells from a parent population of total cell events recorded, which were sorted in a hierarchy into subpopulations as a percentage of the respective parent population in the hierarchy. **FIG. 14** depicts the percentage of viable cells positive for select T cell markers in single cell suspensions immediately after extraction of tumor fragments by homogenization and enzyme digestion from an exemplary CRC donor (donor 1).

The percentage of CD3+ cells was compared in SCS samples that had been extracted by homogenization only (no collagenase) or by homogenization following digestion with a low concentration of collagenase (1 mg/mL) or a high concentration of collagenase (5 mg/mL). Results from a second CRC and a melanoma patient are shown in **FIG. 15A** and **FIG. 15B****,** respectively. As shown in **FIG. 15A****,** the results demonstrate an increased recovery of CD3+ T cells in SCSs from a CRC donor following homogenization and digestion with a low concentration of collagenase. Although the percent of CD3+ cells in SCSs from a melanoma door was lower, the results also demonstrate that homogenization and digestion with a low concentration of collagenase yielded the highest percentage of CD3+ T cells **(****FIG. 15B**). Taken together, these observations demonstrate relative high purity of cells from SCS from melanoma tumors can be achieved and may support that SCS is a viable source of melanoma derived CD3+ cells.

The percentage of CD3+ cells in SCSs extracted from tumors of an additional exemplary CRC donor also was assessed. In addition, in this same donor, the percentage of CD3+ T cells in the SCSs immediately following homogenization and digestion was compared to (1) the percentage of CD3+ cells after culture of SCSs with 300 IU/mL IL-2 (low) or 6000 IU/mL IL-2 (high) for 6 days, or (2) the percentage of CD3+ cells following culture of individual tumor fragments for up to 6 days with 300 IU/mL IL-2 (low) or 6000 IU/mL (high) in the presence of absence of CD3 stimulation (OKT3). As shown in **FIG. 15C**, the percent of CD3 cells in baseline (day 0) SCS was substantially higher than the percent of CD3+ cells in cultures obtained following culture of tumor fragments with IL-2 or OKT3 for 6 days. Similar results from culture of tumor fragments from two additional donors was observed, in which the percentage of CD3+ cells in cultures obtained following culture of CRC-derived tumor fragments with IL-2 and/or OKT3 for 11 days (**FIG. 15D**) or 9 days (**FIG. 15E**) also generally showed a low yield when extracting tumor cells from CRC tumor fragments under various assessed conditions. These results are consistent with a finding that SCSs from tumor biopsies of CRC patients may be more capable of providing an increased number of T cells for expansion than cells obtained from culture of tumor fragments.

In contrast to the results from culture of tumor fragments for CRC patients, **FIG. 16** shows that a high percentage of CD3+ T cells can be obtained from culture of melanoma tumor fragments under various conditions, such as presence of low (300 IU/mL) or high (6000 IU/mL) concentrations of IL-2, presence of absence of CD3 stimulation (OKT3) or different media. The results depicted in **FIG. 16** are from a Day 0 culture. These results are consistent with a finding that culture of tumor fragments from melanoma patients may be more capable of providing an increased number of T cells for expansion than cells obtained from SCSs of tumor biopsies.

### Example 6 Quantification of Activation of Tumor Derived T Cells Following Co-Culture with Antigen Presenting Cells

T cells were sourced from primary tumors in patients with melanoma or CRC as tumor fragments, as described in Example 1. Following 5 days of culture in serum free OpTmizer medium (ThermoFisher) supplemented with 300 IU/mL recombinant IL-2, gentamicin at 10 µg/ml, Immune Cell Serum Replacement (ThermoFisher) at between 2 and 5% according to manufacturer's recommendations, and a final concentration of 2.0 mM of a L-alanyl-L-glutamine dipeptide form of glutamine (GlutaMAX Supplement; Thermofisher), tumor derived cells were washed with OpTmizer medium before being centrifuged at 300 x g for 5 minutes and suspended at 2 × 10⁶ cells/mL. Cells were then seeded into a conventional 6-well culture plate at 10,000,000 cells/well.

In a parallel culture, antigen presenting Dendritic Cells (DCs) were differentiated from PBMCs obtained from the same patient (autologous) as sourced T cells. Cryovials of frozen PBMC isolated from apheresed donors were thawed from liquid nitrogen storage. Cells were thawed in a ten-fold volume of 1X DPBS (Gibco) and counted (NucleoCounter NC200). After washing, cells were immediately used for CD14 microbead positive selection (MACS Miltenyi) according to manufacturer kit instructions. Purified CD14 (monocyte) cells were counted, the cells were resuspended in DendriMACs (MACS Miltenyi) and seeded at a density of 0.5-2 × 10⁶ cells per mL in the appropriate culture flask. GM-CSF (100 ng/mL) and IL-4 (20 ng/mL) were added to cultures to promote differentiation into immature dendritic cells. Monocytes were cultured and differentiated for a total of 5 days, with a 50% addition of medium equal to 50% of the starting amount of medium on day 2.

Coding transcripts of tumor specific peptides were identified autologously for each patient from whole exome sequencing and RNA sequencing as described in Parkhurst, Maria R., et al. "Unique neoantigens arise from somatic mutations in patients with gastrointestinal cancers." Cancer discovery 9.8 (2019): 1022-1035. Whole-exome sequencing (WES) of patient samples was performed on snap-frozen, unfixed, tumor tissue and normal peripheral blood cells (normal source). Alignments of sequences from tumor vs. normal samples were performed using novoalign MPI from novocraft (http://www.novocraft.com/) to human genome build hg19. Duplicates were marked using Picard's MarkDuplicates tool. Insertion deletion realignment and base recalibration was carried out according to the GATK best practices workflow (https://www.broadinstitute.org/gatk/). Post cleanup of data, pileup files were created using samtools mpileup (http://samtools.sourceforge.net) and Varscan2, (http://varscan.sourceforge.net), SomaticSniper (http://gmt.genome.wustl.edu/packages/somatic-sniper/), Strelka (https://sites.google.com/site/strelkasomaticvariantcaller/), and Mutect (https://www.broadinstitute.org/gatk/). VCF files were merged using GATK CombineVariants tools and annotated using Annovar (http://annovar.openbioinformatics.org). Variants (mutations) present in patient tumors were then annotated using Annovar (http://annovar.openbioinformatics.org).

The following filters were used to generate an initial list of putative mutations for evaluation: (1) a tumor and normal coverage of greater than 10, (2) a variant allele frequency (VAF) of 7% or above, (3) variant read counts of 4 or above, (4) and two of the four callers identifying mutations. For insertions and deletions, the same cutoffs were used except only a single caller identifying the mutation was required to pass filters, as these were only called by varscan and strelka. Tables of amino acid sequences corresponding the mutant residue joined to the 12 amino acids encoded by regions upstream and downstream of single nucleotide variants (SNVs) (Nmers) were generated for those variants which passed the four filters. For frame-shifted transcripts, sequences were translated until a stop codon was generated in either the normal coding region or in the 3' un-translated region. The Integrative Genomics Viewer (IGV, Broad Institute), which allows mapped alignments to be visualized, was then used to carry out manual curation of the variant calls. Changes were made to the sequences of Nmers when manual curation revealed non-synonymous changes resulting from additional somatic variants or germline variants present within transcripts encoding the Nmers. Variants inferred from reads containing multiple mis-matched nucleotides, insertion/deletions mapping to different locations in different reads, and variants corresponding to frequent SNPs were flagged for removal.

Variants that were detected in more than one patients tumor but in fewer than 2.5% of total tumors were flagged but included in the list of variants that passed. Variant transcripts only annotated in the ENSEMBL database generally represent unverified coding regions and were also removed. Variants flagged as being known single nucleotide polymorphisms or were present in multiple tumors were not automatically removed but were further evaluated using IGV, as removing potential false positives, which are unlikely to encode products recognized by T cells, was less critical than removing candidates that could represent false negatives.

These sequencing data were then used to generate a peptide pool representing mutated peptide associated with the tumor and wild-type peptide associated with non-diseased peripheral blood cells.

Synthetic peptides were synthesized via Fmoc chemistry. For indels, 25 amino acid peptides were synthesized overlapping by 10 amino acids based on the translation of the frame-shifted sequence until the next stop codon. In some cases, peptides of minimal epitopes were synthesized. Peptides were dissolved in DMSO and mixed in equal volumes.

Differentiated DCs were loaded with a varying number and concentration of peptides from a peptide pool identified as described above before they were added to the tumor derived culture at several ratios of Tumor Cells: DC. DCs and tumor derived cells were then co-cultured at 37°C for 6 hours at 5% CO₂ before the culture was gently agitated and cells in suspension recovered. Recovered cells were then sorted via flow cytometry for activated T-cells using markers of T cell activation 4-1BB and OX40.

**FIG. 17A and 17B** show tumor derived T cell activation over a peptide range of 20 to 0.1 ng/mL. As shown in **FIG. 17A**, T cell co-culture with DCs loaded with each of three peptide concentrations tested resulted in readily detectable levels of 4-1BB/OX40+ T cells, including as high as approximately 80% at 1 ng/mL peptide. Increase in T cell activation marker expression as compared to cells cultured with unloaded DCs is shown in **FIG. 17B**, where 0.1 ng/mL resulted in the largest delta but all three concentrations of peptide resulted in a positive fold change. These data demonstrate that lower peptide concentrations less than 20 ng/mL may result in increased upregulation of T cell activation markers (upregulation markers) following co-culture.

**FIG. 16** similarly depicts tumor derived T cell activation as a function of 41BB/OX40 expression in studies in which DCs were pulsed with one or two peptides for surface presentation during co-culture. Shown in **FIG. 18A** and again in **FIG. 18B** as fold change, DCs which were loaded with only one peptide were more markedly more efficient at activating T cells in co-culture.

As shown in **FIG. 19**, markers of T cell activation 41BB and OX40 were substantially upregulated when tumor derived T cells were co-cultured with DCs at a ratio of 1:2 (T cells: DC) as compared to 1:1.

### Example 7 Enrichment and Recovery of Activated T-Cells via Cell Sorting

T-cells from a healthy donor were isolated by immunoaffinity-based selection and then cryopreserved. The T cells were thawed and rested overnight, and then were activated with 50 ng/mL OKT3 for 24 - 48 hours prior to staining with anti-CD4 FITC (BD), anti-CDS PerCPCy.5.5 (BD), anti-CD134 (Beckman Couleter), and anti-CD137 (MACs Miltenyi). Cells were brought to a concentration of approximately 20 × 10⁶ cells/mL and sorted using the BD FACSAriaII at a sort rate of approximately 15,000 events per second. A gate was drawn around cells expressing CD134, CD137, or both CD134 and CD137 and sorted into a single population. This was the positive sorted population. Cells lacking both CD134 and CD137 expression were sorted into a separate population. This was the negative sorted population. After sorting, cells from the positive and negative sorted populations and the unsorted population were analyzed on an alternative flow cytometer to verify purity and assess recovery rates.

As shown in **FIG. 20**, the unsorted tumor derived T cell population (pre-sort) were compared to the positive sorted population which was collected after co-culture with mutant peptide loaded autologous dendritic cells as described previously in Example 6 and sorted into 41BB/OX40 positive populations. It was observed that this gating strategy resulted in an increased enrichment for percent reactive TCR for three donors (**FIG. 20A**) and average Class I reactivity **(****FIG. 20B**).

Total cell recovery from cell sorting is shown with respect to total cell input in **FIG. 21A**. Similarly seen in **FIG. 21B**, percent recovery from two independent runs was approximately 80%. The results demonstrate that it is possible to obtain a high recovery of cells after selection and sorting of cells positive for upregulation markers.

**FIG. 22** depicts CD4+ population purity via flow cytometry of healthy donor T cells activated with OKT3 and stained as described above. Cells were first gated on CD4+, then the population expressing the highest intensity of CD134+ was next gated and the outputs displayed showing CD4+ vs. CD8+ and CD137+ vs. CD134+. These data support the use of these markers for gating a high purity population of tumor infiltrating T cells.

### Example 8 Post-Sort Expansion of Activated Tumor Derived T Cells

T cells sourced from a primary CRC tumor were processed as described in Example 1, and then were co-cultured with peptide presenting dendritic cells, using methods as described in Example 6. Briefly, isolated tumor infiltrating lymphocytes were cultured with autologous DCs that were loaded to express peptide associated with healthy tissue (Wild-type, WT), peptide associated with tumor tissue (Mutant), or were not loaded with peptide at all (No peptide). A control subpopulation of T cells were cultured without DCs (Unactivated). After the co-culture, the cells were sorted via the fluorescence enabled Sony FX500 based on surface expression of activation markers 4-1BB and OX40.

Cells were then seeded into a gas permeable 24 well culture plate at 250,000-1,000,000 cells/cm2 in serum free OpTmizer medium supplemented with recombinant IL-2 at a concentration of 300 IU/mL, gentamicin at 10 µg/mL, and 2.0 mM of a L-alanyl-L-glutamine dipeptide form of glutamine (GlutaMAX Supplement; Thermofisher). Cells were incubated for a total of 7 days with 50% of the media exchanged every other day beginning on culture day 5. Cell counts were performed using NC-200 Automated Cell Counter (Chemometec). on each culture day.

As shown in **FIG. 23A** and again in **FIG. 23B** as fold expansion, each tumor infiltrating lymphocyte (TIL) T cell population tested underwent measurable expansion between culture days 3 and 5 and continued to trend upwards at the 7 day conclusion of the culture period. Tumor infiltrating T cells cultured with DCs loaded with mutant tumor-associated peptide reached the highest total cell number over the course of the experiment.

Using the data above, a theoretical mathematical model shown in **FIG. 23C** was created to predict the relationship between the number of cells recovered after sorting and the expected number of cells present in culture following the expansion phase.

### Example 9 Monte Carlo Modeling Ex Vivo Expansion of Tumor Derived T Cells

In complement to the deterministic point analysis in Example 8, a first probabilistic Monte Carlo simulation was designed to forecast the number of tumor infiltrating lymphocytes resultant from a first expansion as described in Example 2. Monte Carlo simulations of likely total viable and total reactive T cell numbers post extraction and a first expansion were run by substituting a probability distribution for two factors of inherent uncertainty, recovery efficiency and fold expansion capacity. The results were iteratively calculated tens of thousands of times as a normal distribution wherein a mean value of cells recovered was defined for low and mid recovery, and a mean value for fold change in expansion was defined for low, mid, and high expansion potentials. Distributions were then calculated for total viable T cell number as well as total reactive T cells.

For the initial Monte Carlo simulations, wherein probable T cell outputs are calculated for a first expansion, test cases were run to model low recovery/low expansion, mid recovery/low expansion, mid recovery/mid expansion, and mid recovery/high expansion conditions. Values for the mean and standard deviation for both recovery and expansion variables were assigned as follows: (1) low recovery was defined as culturing a total of 20 million viable cells from the processed tumor at a standard deviation of 6 million, (2) mid recovery was defined as 50 or 60 million cells with a standard deviation of 15 million, (3) a low first fold expansion was defined as 50 fold with a standard deviation of 11, (4) mid expansion was defined as 75 fold with a standard deviation of 15, and (5) high expansion was defined as 500 fold with a standard deviation of 160.

Data for each test case from the first Monte Carlo simulations A are displayed in **Table E1** below.

| **Table E1: Monte Carlo Simulations for a first expansion** | | | | |
|---|---|---|---|---|
| | | ***Total Viable Tumor Infiltrating T Cells*** | | ***Total Reactive Tumor Infiltrating T Cells*** |
| **Test Conditions** | **Assumptions** | **Statistics** | **Forecast Values** | **Forecast Values** |
| Low Recovery/ Low Expansion | Mean = 20 | Mean | 1.99 ×10⁷ | 1.59 ×10⁶ |
| | Std. Dev = 6 | Median | 1.99 ×10⁷ | 1.51 ×10⁶ |
| | Mean = 50 | Probability Distribution (10-90^{th} Percentile) | 1.22 ×10⁷ - 2.76 ×10⁷ | 7.48 ×10⁵ -2.53 ×10⁶ |
| | Std Dev = 11 | | | |
| Mid Recovery/Low Expansion | Mean = 50 | Mean | 4.99 ×10⁷ | 3.99 ×10⁶ |
| | Std. Dev = 15 | Median | 5.00 ×10⁷ | 3.81 ×10⁶ |
| | Mean = 50 | Probability Distribution (10-90^{th} Percentile) | 3.05 ×10⁷ - 6.93 ×10⁷ | 1.85 ×10⁶ -6.34 ×10⁶ |
| | Std Dev = 11 | | | |
| Mid Recovery/Mid Expansion | Mean = 60 | Mean | 6.00 ×10⁷ | 4.81 ×10⁶ |
| | Std. Dev = 15 | Median | 5.99 ×10⁷ | 4.63 ×10⁶ |
| | Mean = 75 | Probability Distribution (10-90^{th} Percentile) | 4.07 ×10⁷ - 7.91 ×10⁷ | 4.07 ×10⁷ - 7.91 ×10⁷ |
| | Std Dev = 15 | | | |
| Mid Recovery/High Expansion | Mean = 60 | Mean | 4.78 ×10⁷ | 5.99 ×10⁷ |
| | Std. Dev = 15 | Median | 4.60 ×10⁷ | 6.00 ×10⁷ |
| | Mean = 500 | Probability Distribution (10-90^{th} Percentile) | 4.07 ×10⁷ - 7.94 ×10⁷ | 2.43 ×10⁶ - 7.40 ×10⁶ |
| | Std Dev = 160 | | | |

Using these data, a second set of Monte Carlo simulations were designed to predict the final number of reactive tumor infiltrating lymphocytes following co-culture with APCs, sorting via flow cytometry, and a second expansion as described in Example 8. A fixed value for percentage of reactive T cells present in the population of total T cells cultured from either tumor fragments or SCS was assigned to a mean of 8% and a standard variation of 2.50. Following tens of thousands of iterative calculations, data for each test case from the second Monte Carlo simulations are depicted in Table E2 below.

| **Table E2: Monte Carlo Simulations for a second and final expansion** | | | |
|---|---|---|---|
| | | ***Total Reactive Tumor Infiltrating T Cells*** | |
| **Test Conditions** | **Assumptions** | **Statistics** | **Forecast Values** |
| Low Recovery/ Low Expansion | Mean = 20 | Mean | 7.96 ×10⁷ |
| | Std. Dev = 6 | Median | 7.34 ×10⁷ |
| | Mean = 50 | Probability Distribution (10-90^{th} Percentile) | 3.41 ×10⁷ - 1.34 ×10⁸ |
| | Std Dev = 11 | | |
| Mid Recovery/Low Expansion | Mean = 50 | Mean | 2.00 ×10⁸ |
| | Std. Dev = 15 | Median | 1.85 ×10⁸ |
| | Mean = 50 | Probability Distribution (10-90^{th} Percentile) | 8.44 ×10⁷ - 3.33 ×10⁸ |
| | Std Dev = 11 | | |
| Mid Recovery/Mid Expansion | Mean = 60 | Mean | 3.61 ×10⁸ |
| | Std. Dev = 15 | Median | 3.39 ×10⁸ |
| | Mean = 75 | Probability Distribution (10-90^{th} Percentile) | 1.68 ×10⁸ -5.80 ×10⁸ |
| | Std Dev = 15 | | |
| Mid Recovery/High Expansion | Mean = 60 | Mean | 2.39 ×10⁹ |
| | Std. Dev = 15 | Median | 2.19 ×10⁹ |
| | Mean = 500 | Probability Distribution (10-90^{th} Percentile) | 9.57 ×10⁸ - 4.06 ×10⁹ |
| | Std Dev = 160 | | |

Recovery and expansion potential of tumor infiltrating reactive T cells from a first expansion following tumor processing or a second expansion following downstream co-culture with APCs are factors which are inherently variable across donors and within a tumor cell population. The expected range of T cell numbers generated by the process described here is contained within the 10^{th} and 90^{th} percentiles. Cell numbers below the 10^{th} percentile are unlikely to be generated and will likely not result in a usable drug product. Therefore, the observations from the Monte Carlo simulations in **Tables E1 and E2** support that in all scenarios between the 10^{th} and 90^{th} percentiles, given a range of levels of variability for expansion potentials, the method described herein will likely provide a robust T cell output that is approximate to the number of cells which would be required for therapeutic dosing.

### Example 10 Example 10: Assessment of Tumor-reactive TCR Enrichment by IFN-gamma production and TCR clonality

T cells sourced from primary tumor of patients with ovarian cancer (Sample A), CRC (Sample B) or melanoma (Sample C) were processed from tumor fragments as described in Example 1. Following the initial expansion, T cells were then co-cultured with peptide presenting, autologous dendritic cells for 6 hours using methods substantially as described in Example 6. For the co-culture, autologous DCs were loaded with either a mutant single long peptide (e.g. 25 mer) unique to the patient tumor or a wild-type single long peptide that was not-mutated compared to normal sample from the patient. After the co-culture, tumor-reactive T cells were enriched by staining the cells for expression of 4-1BB (CD137) and/or OX40 (CD134) and sorting the cells by fluorescence-activated cell sorting (FACS). Cells that were positive for either or both of 41BB and OX40 were collected as the "positive" population (also called "mutant enriched" population) and cells that were double negative for 41BB and OX40 were collected as the "negative" population (also called "wild-type unenriched" population).

The mutant and wild-type T cell populations were then cultured for 16 hours in media alone or under conditions to stimulate IFN-gamma secretion. Unsorted, unenriched T cells (bulk T cells) from the co-culture that had not been sorted based on 41BB and OX40 expression were included as a pre-selection control and were similarly stimulated. Culture supernatant was collected and IFN-gamma secretion levels were determined by ELISA.

The percentage of T cells in the sorted population expressing a TCR reactive to the peptide neo-epitope was determined by single cell TCR sequencing . TCR clonality in the T cell populations also was determined by single cell RNA- sequencing for the TCR-beta and TCR-alpha chains.

### 1. Sample A (Ovarian Cancer)

The mutant and wild-type enriched T cell populations, or control bulk T cells, produced from Sample A tumor cells were cultured for 16 hours in media alone or were stimulated by culture with anti-CD28 and anti-CD49d antibodies along with either the minimal peptide epitope (8mer) corresponding to the mutant peptide (neo-epitope) or the wild-type peptide from the respective patient tumor. As shown in **FIG. 24A****,** bulk T cells exhibited improved reactivity, as evidenced by increased IFN-gamma secretion, following culture with the neo-epitope compared to culture media alone. The ability to produce IFN-gamma was further increased in the mutant enriched T cell population that was stimulated with the neo-epitope, but no difference was observed in the wild-type enriched T cell population following stimulation in media alone versus stimulation with the neo-epitope conditions. Additionally, the wild-type unenriched T cell population still included some degree of neo-antigen reactive T cells, as evidenced by their upregulation of IFN-gamma secretion compared to media alone. This data indicates that the bulk T cells following co-culture contain a neoantigen reactive population, which is enriched by sorting based on expression of 41BB and OX40. Further, the results also demonstrate the specificity of neo-antigen enrichment.

Analysis of neoepitope-specific TCRs by RNA sequencing and flow cytometry showed an enrichment of TCR "A" neoantigen-specific TCRs in the mutant enriched T cell populations with 17% neoantigen-specific TCRs compared to 2% in the initial bulk T cell population or 0.1% in the wild-type enriched T cell population (**FIG**. **24B**). The TCR clonality of T cells in the unselected population (wild-type enriched T cell population) compared to selected population (mutant enriched T cell population) is shown in **FIG. 24C**, which shows that the incoming TCR diversity is high the unsorted T cell population and that enrichment of unique TCR clones is achieved in the selected population. **FIG. 24D** demonstrates that the Pre- (bulk) and post-sort cell populations contain CD4 and CD8 cells, indicating that class I and class II reactive cells are present in the enriched population,

### 2. Sample B (CRC Patient)

The mutant and wild-type enriched T cell populations, or control bulk T cells, produced from Sample B tumor cells were cultured for 16 hours in media alone or were stimulated in response to a general TCR stimulation using an anti-CD3 antibody (OKT3). As shown in **FIG. 25A**, all T cell populations displayed functionality (i.e. IFNg production) after coculture and sorting in response to the general TCR stimulation.

Analysis of neoepitope-specific TCRs showed an enrichment of neoantigen "B"-specific TCRs in the mutant enriched T cell populations with 71% neoantigen-specific TCRs compared to 42% in the initial bulk T cell population or 17% in the wild-type enriched T cell population (**FIG. 25B**). Compared to the bulk T cells after co-culture, this represents an approximate 1.7-fold enrichment in the tumor-reactive T cells in the sorted T cell population, and an approximate 2.5-fold reduction in tumor-reactive T cells in the non-sorted T cell population. The TCR clonality of T cells in the unselected population (wild-type enriched T cell population) compared to selected population (mutant enriched T cell population) is shown in **FIG. 25C**, which shows that the incoming TCR diversity is high in the unsorted T cell population (807 unique TCR clones) and that enrichment of unique TCR clone is achieved in the selected population (64 unique TCR clones). **FIG. 25D** demonstrates that the pre- (bulk) and post-sort cell populations contain CD4 and CD8 cells, indicating that class I and class II reactive cells are present in the enriched population.

### 3. Sample C (Melanoma Patient)

T cells in the mutant and wild-type enriched T cell populations, or control bulk T cells, produced from Sample C tumor cells were assessed for neoepitope-specific TCRs by RNA sequencing and flow cytometry and TCR clonality. The results showed an enrichment of neoantigen "C"-specific TCRs in the mutant enriched T cell populations with 33% neoantigen-specific TCRs compared to 5% in the initial bulk T cell population or 4% in the wild-type enriched T cell population (**FIG. 26A**). Compared to the bulk T cells after co-culture, this represents an approximate 7-fold enrichment in the tumor-reactive T cells in the sorted T cell population, and no enrichment in tumor-reactive T cells in the non-sorted T cell population. The TCR clonality of T cells in the unselected population (wild-type enriched T cell population) compared to selected population (mutant enriched T cell population) is shown in **FIG. 26B**, which shows that the incoming TCR diversity is high in the unsorted T cell population (182 unique TCR clones) and that enrichment of unique TCR clone is achieved in the selected population (15 unique TCR clones). **FIG. 26C** demonstrates that the pre- (bulk) and post-sort cell populations contain CD4 and CD8 cells, indicating that class I and class II reactive cells are present in the enriched population.

### 4. Conclusion

Together, the results show that the incoming TCR diversity is high in the unsorted T cell population (e.g. 100-900 TCRs). This unsorted population produces a low level of IFNgamma (e.g. 5-25 pg/mL). After sorting of the TCR population based on activation markers, e.g. OX40/41BB, the TCR population is enriched in a reactive population of TCR's (e.g. 15-64 TCRs) that produce higher IFNgamma (e.g. 65.3-98.6 pg/mL) than the unsorted and negative sorted population (5 pg/mL) of TCRs. The results indicate that this is specific activation consistent with enrichment of tumor-reactive T cells as it is not seen in the wild-type, unsorted co-cultures.

## Claims

1. A method for manufacturing T cells for use in a therapeutic cell composition, wherein the T cells express a T cell receptor (TCR) that recognizes an antigen on the surface of a target cell, the method comprising:
a. processing a biological sample containing a population of T lymphocyte cells obtained from a donor subject that has a tumor to produce a first population of T lymphocyte cells;
b. stimulating the first population with one or more first T-cell stimulating agents of lymphocytes under conditions for expansion of T cells in the population to produce a second population of activated T cells wherein the stimulation is performed in a closed system using serum free medium, wherein the one or more first T-cell stimulating agents is or comprises recombinant IL-2;
c. co-culturing the second population of T cells in the presence of antigen presenting cells that present one or more non-native peptide on a major histocompatibility complex (MHC) in a closed system using serum free medium, said one or more non-native peptides are peptides corresponding to nonsynonymous somatic mutations associated in the tumor of the subject, to produce a third population of cells containing T cells comprising endogenous T cell receptors reactive to mutation encoding peptides of the tumor;
d. selecting, from the third population of cells, the population of T cells containing endogenous TCR that are reactive to peptides present on the APCs based on one or more upregulation markers expressed on reactive or activated T cells in a closed system to produce a fourth population containing the selected T cells; and
e. expanding the fourth population of selected cells in the presence of one or more second T-cell stimulating agents of lymphocytes under conditions to expand T cells in the population in a closed system using serum free medium to produce a composition of expanded T cells for use as a therapeutic cell composition, wherein the one or more second T-cell stimulating agents is or comprises recombinant IL-2.

2. The method of claim 1, wherein:
(a) the antigen presenting cells are nucleated cells such as dendritic cells, mononuclear phagocytes, B lymphocytes, endothelial cells or thymic epithelium, optionally wherein the antigen presenting cells are dendritic cells; and/or
(b) the antigen presenting cells and/or the T cells are autologous to the subject.

3. The method of claim 1 or claim 2, wherein the T cells are CD4+ cells, CD8+ cells, or CD4+ cells and CD8+ cells.

4. The method of any of claims 1-3, wherein:
(A) the one or more first T cell stimulating agents comprises recombinant IL-2 and wherein the concentration of recombinant IL-2 is:
(i) from 100 IU/mL to 6000 IU/mL;
(ii) from 300 IU/mL to 1000 IU/mL; or
(iii) at or about 300 IU/mL; and/or
(B) the one or more second T cell stimulating agents comprises recombinant IL-2 and wherein the concentration of recombinant IL-2 is:
(i) from 100 IU/mL to 6000 IU/mL;
(ii) from 300 IU/mL to 1000 IU/mL; or
(iii) at or about 300 IU/mL.

5. The method of any of claims 1-3, wherein:
(i) the one or more first T cell stimulating agents comprises recombinant IL-2 and the recombinant IL-2 is present in an amount that is or is about 6000 IU/mL; and/or
(ii) the one or more second T cell stimulating agents comprises recombinant IL-2 and the recombinant IL-2 is present in an amount that is or is about 6000 IU/mL.

6. The method of any of claims 1-5, wherein the one or more second T cell stimulating agent further comprises an anti-CD3 antibody, optionally OKT3, optionally wherein the concentration of the anti-CD3 antibody is at or about 50 ng/mL.

7. The method of any of claims 1-6, wherein the one or more second T cell stimulating agent further comprises feeder cells that are non-dividing peripheral blood mononuclear cells (PBMC), optionally wherein the feeder cells are gamma irradiated.

8. The method of any of claims 1-7, where the one or more non-native peptides are loaded on antigen presenting cells by peptide pulse, optionally wherein:
(i) the peptide pulse is carried out by electroporation;
(ii) the one or more non-native peptide is 5-30 amino acids, optionally 12-25 amino acids, optionally at or about 25 amino acids in length; and/or
(iii) the one or more non-native peptides:
(a) are a pool of peptides and the concentration of peptides in the pool of peptidess for the peptide pulse is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL; or
(b) is an individual peptide and the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL, optionally wherein the concentration of individual peptides of the one or more non-native peptide, on average, is from at or about 0.00001 µg/mL to at or about 0.01 µg/mL or the concentration of individual peptide of the one or more non-native peptide, on average, is from at or about 0.0001 µg/mL and at or about 0.001 µg/mL.

9. The method of any of claims 1-8, wherein the co-culture ratio of antigen presenting cells to T Cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1, optionally wherein the co-culture ratio of antigen presenting cells to T cells is or is about 1:1.

10. The method of any of claims 1-9, wherein the co-culturing is for 2 hours to 24 hours, optionally wherein the co-culturing is for at or about 6 hours.

11. The method of any of claims 1-10, where the selection is performed using a florescence based cell sorter, optionally wherein:
(i) the fluorescence based cell sorter is an automated high-throughput flow cytometry sorter, optionally FX500 cell sorter or Miltenyi Tyto cell sorter; and/or
(ii) the selection is by 1 run, 2 runs, 3 runs or 4 runs by the fluorescence based cell sorter; and/or
(iii) the selection is performed at rate between 10,000 and 100,000 cells / second using a florescent based disposable fluidics cell sorter.

12. The method of any of claims 1-11, wherein the one or more upregulation markers are selected from the group consisting of CD107, CD107a, CD39, CD103, CD137 (4-1BB), CD59, CD69, CD90, CD38, CD30, CD154, CD252, CD134 (OX40), CD258, CD256, PD-1, TIM-3 and LAG-3, optionally wherein the one or more upregulation marker is selected from the group consisting of CD107, CD107a, CD39, CD103, CD59, CD90, CD38, CD30, CD154, CD252, CD134, CD258 and CD256.

13. The method of any of claims 1-12, wherein the one or more upregulation maker:
(i) is CD134 and/or CD137; or
(ii) comprises at least two markers selected from CD107a and CD39, CD107a and CD103, CD107a and CD59, CD107a and CD90, CD107a and CD38, CD39 and CD103, CD39 and CD59, CD39 and CD90, CD39 and CD38, CD103 and CD59, CD103 and CD90, CD103 and CD38, CD59 and CD90, CD59 and CD38 and CD90 and CD38, optionally wherein the one or more T cell upregulation marker further comprises CD137, optionally wherein the one or more T cell upregulation marker comprises at least two markers selected from CD107a and CD137, CD38 and CD137, CD103 and CD137, CD59 and CD137, CD90 and CD137 and CD38 and CD137.

14. The method of any of claims 1-13, wherein:
(i) the stimulating the first population is for 7 to 21 days, optionally 7 to 14 days; and/or
(ii) the stimulating the first population in a closed system is using a gas permeable culture vessel; and/or
(iii) the stimulating the first population in a closed system is performed using a bioreactor.

15. The method of any of claims 1-14, wherein:
(i) the expanding the fourth population is for 7 to 21 days, optionally 7 to 14 days; and/or
(ii) the expanding the fourth population is performed in a gas permeable culture vessel; and/or
(iii) the expanding the fourth population is performed using a bioreactor.

16. The method of any of claims 1-15, wherein the time from processing the biological samples containing the population of lymphocytes to producing the expanded T cells is less than 30 days.

17. The method of any of claims 1-16, wherein:
(i) the tumor is a tumor of an epithelial cancer;
(ii) the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer; or
(iii) the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

18. The method of any of claims 1-17, wherein the biological sample is a tumor and the population of lymphocytes from the biological sample are from tumor infiltrating lymphocytes.

19. The method of any of claims 1-18, wherein:
A) the first population of lymphocytes are processed as tumor fragments from a resected tumor, optionally wherein the first population of lymphocytes are one or more tumor fragments from the resected tumor, more optionally wherein the first population of lymphocytes are seeded for stimulation in the closed system at about 1 tumor fragment per 2 cm²; or
B) the first population of lymphocytes are processed as a single cell suspension by homogenization and/or enzymatic digestion of one or more tumor fragments from a resected tumor, optionally wherein the enzymatic digestion is by incubation with a collagenase, optionally collagenase IV or collagenase I/II, more optionally wherein the first population of lymphocytes are seeded for stimulation in the closed system at about 5 × 10⁵ to at or about 2 × 10⁶ total cells per 2 cm².

20. The method of any of claims 1-19, further comprising harvesting cells produced by the method, optionally comprising formulating the harvested cells with a cryoprotectant.

21. The method of any of claims 1-20, wherein the population of T cells of the therapeutic composition are able to produce IFNgamma at a concentration of greater than at or about 30 pg/mL, optionally greater than at or about 60 pg/mL, following antigen-specific stimulation.

## Patentansprüche

1. Verfahren zur Herstellung von T-Zellen zur Verwendung in einer therapeutischen Zellzusammensetzung, wobei die T-Zellen einen T-Zell-Rezeptor (TCR) exprimieren, der ein Antigen auf der Oberfläche einer Zielzelle erkennt, wobei das Verfahren Folgendes umfasst:
a. Verarbeiten einer biologischen Probe, die eine Population von T-Lymphozytenzellen enthält, die von einem Spendersubjekt erhalten wird, das einen Tumor hat, um eine erste Population von T-Lymphozytenzellen zu erzeugen;
b. Stimulieren der ersten Population mit einem oder mehreren ersten T-Zell-stimulierenden Mitteln von Lymphozyten unter Bedingungen zur Expansion von T-Zellen in der Population, um eine zweite Population von aktivierten T-Zellen zu erzeugen, wobei die Stimulation in einem geschlossenen System unter Verwendung von serumfreiem Medium durchgeführt wird, wobei das eine oder die mehreren ersten T-Zell-stimulierenden Mittel rekombinantes IL-2 ist oder umfasst;
c. Co-Kultivieren der zweiten Population von T-Zellen in Anwesenheit von antigenpräsentierenden Zellen, die ein oder mehrere nicht native Peptide an einem Haupthistokompatibilitätskomplex (MHC) in einem geschlossenen System unter Verwendung von serumfreiem Medium präsentieren, wobei das eine oder die mehreren nicht nativen Peptide Peptide sind, die nichtsynonymen somatischen Mutationen im Zusammenhang in dem Tumor des Subjekts entsprechen, um eine dritte Population von Zellen zu erzeugen, die T-Zellen enthalten, die endogene T-Zell-Rezeptoren umfassen, die reaktiv auf Mutations-kodierende Peptide des Tumors sind;
d. Auswählen, aus der dritten Population von Zellen, der Population von T-Zellen, die endogene TCR enthalten, die auf Peptide reaktiv sind, die an den APCs vorhanden sind, basierend auf einem oder mehreren Hochregulierungsmarkern, die an reaktiven oder aktivierten T-Zellen in einem geschlossenen System exprimiert sind, um eine vierte Population zu erzeugen, die die ausgewählten T-Zellen enthält; und
e. Expandieren der vierten Population von ausgewählten Zellen in Anwesenheit von einem oder mehreren zweiten T-Zell-stimulierenden Mitteln von Lymphozyten unter Bedingungen, um T-Zellen in der Population in einem geschlossenen System unter Verwendung von serumfreiem Medium zu expandieren, um eine Zusammensetzung aus expandierten T-Zellen zur Verwendung in einer therapeutischen Zellzusammensetzung zu erzeugen, wobei das eine oder die mehreren zweiten T-Zell-stimulierenden Mittel rekombinantes IL-2 ist oder umfasst.

2. Verfahren nach Anspruch 1, wobei:
(a) die antigenpräsentierenden Zellen kernhaltige Zellen sind, wie z. B. dendritische Zellen, mononukleäre Phagozyten, B-Lymphozyten, Endothelzellen oder Thymusepithel, wobei optional die antigenpräsentierenden Zellen dendritische Zellen sind; und/oder
(b) die antigenpräsentierenden Zellen und/oder die T-Zellen autolog zu dem Subjekt sind.

3. Verfahren nach Anspruch 1 oder Anspruch 2, wobei die T-Zellen CD4+-Zellen, CD8+-Zellen oder CD4+-Zellen und CD8+-Zellen sind.

4. Verfahren nach einem der Ansprüche 1-3, wobei:
(A) das eine oder die mehreren ersten T-Zell-stimulierenden Mittel rekombinantes IL-2 umfasst, und wobei die Konzentration von rekombinantem IL-2 Folgendes ist:
(i) von 100 IU/ml bis 6000 IU/ml;
(ii) von 300 IU/ml bis 1000 lU/ml; oder
(iii) bei oder etwa 300 lU/ml; und/oder
(B) das eine oder die mehreren zweiten T-Zell-stimulierenden Mittel rekombinantes IL-2 umfasst, und wobei die Konzentration von rekombinantem IL-2 Folgendes ist:
(i) von 100 IU/ml bis 6000 IU/ml;
(ii) von 300 IU/ml bis 1000 lU/ml; oder
(iii) bei oder etwa 300 IU/ml.

5. Verfahren nach einem der Ansprüche 1-3, wobei:
(i) das eine oder die mehreren ersten T-Zell-stimulierenden Mittel rekombinantes IL-2 umfasst und das rekombinante IL-2 in einer Menge vorhanden ist, die 6000 IU/ml ist oder etwa ist; und/oder
(i) das eine oder die mehreren zweiten T-Zell-stimulierenden Mittel rekombinantes IL-2 umfasst und das rekombinante IL-2 in einer Menge vorhanden ist, die 6000 IU/ml ist oder etwa ist.

6. Verfahren nach einem der Ansprüche 1-5, wobei das eine oder die mehreren zweiten T-Zell-stimulierenden Mittel ferner einen Anti-CD3-Antikörper umfasst, optional OKT3, wobei optional die Konzentration des Anti-CD3-Antikörpers bei oder etwa 50 ng/ml ist.

7. Verfahren nach einem der Ansprüche 1-6, wobei das eine oder die mehreren zweiten T-Zell-stimulierenden Mittel ferner Feeder-Zellen umfasst, die nicht teilende periphere mononukleäre Blutzellen (PBMC) sind, wobei optional die Feeder-Zellen gammabestrahlt sind.

8. Verfahren nach einem der Ansprüche 1-7, wobei das eine oder die mehreren nicht nativen Peptide auf antigenpräsentierende Zellen durch Peptidpuls geladen werden, wobei optional:
(i) der Peptidpuls durch Elektroporation ausgeführt wird;
(ii) das eine oder die mehreren nicht nativen Peptide 5-30 Aminosäuren sind, optional 12-25 Aminosäuren, optional bei oder etwa 25 Aminosäuren in der Länge; und/oder
(iii) das eine oder die mehreren nicht nativen Peptide:
(a) ein Peptidpool aus Peptiden sind und die Konzentration von Peptiden in dem Peptidpool für den Peptidpuls zwischen bei oder etwa 0,001 µg/ml und bei oder etwa 40 µg/ml, 0,01 µg/ml und bei oder etwa 40 µg/ml, bei oder etwa 0,1 µg/ml und bei oder etwa 40 µg/ml, bei oder etwa 1 µg/ml und bei oder etwa 40 µg/ml, bei oder etwa 0,01 µg/ml und bei oder etwa 10 µg/ml oder bei oder etwa 1 µg/ml und bei oder etwa 10 µg/ml ist; oder
(b) ein individuelles Peptid ist und die Konzentration von individuellen Peptiden für den Peptidpuls zwischen bei oder etwa 0,00001 µg/ml und bei oder etwa 1 µg/ml, bei oder etwa 0,00001 µg/ml und bei oder etwa 0,1 µg/ml, bei oder etwa 0,00001 µg/ml und bei oder etwa 0,01 µg/ml, bei oder etwa 0,0001 µg/ml und bei oder etwa 1 µg/ml, bei oder etwa 0,0001 µg/ml und bei oder etwa 0,1 µg/ml, bei oder etwa 0,0001 µg/ml und bei oder etwa 0,1 µg/ml oder bei oder etwa 0,0001 µg/ml und bei oder etwa 0,01 µg/ml ist, wobei optional die Konzentration von individuellen Peptiden aus dem einen oder den mehreren nicht nativen Peptiden, im Durchschnitt, von bei oder etwa 0,00001 µg/ml bis bei oder etwa 0,01 µg/ml ist oder die Konzentration von individuellem Peptid aus dem einen oder den mehreren nicht nativen Peptid, im Durchschnitt, von bei oder etwa 0,0001 µg/ml bis bei oder etwa 0,001 µg/ml ist.

9. Verfahren nach einem der Ansprüche 1-8, wobei das Co-Kultivierungs-Verhältnis von antigenpräsentierenden Zellen zu T-Zellen zwischen 20:1 und 1:1, zwischen 15:1 und 1:1, zwischen 10:1 und 1:1, zwischen 5:1 und 1:1, zwischen 2,5:1 und 1:1, zwischen 1:20 und 1:1, zwischen 1:15 und 1:1, zwischen 1:10 und 1:1, zwischen 1:5 und 1:1 oder zwischen 1:2,5 und 1:1 ist, wobei optional das Co-Kultivierungs-Verhältnis von antigenpräsentierenden Zellen zu T-Zellen 1:1 ist oder etwa ist.

10. Verfahren nach einem der Ansprüche 1-9, wobei das Co-Kultivieren 2 Stunden bis 24 Stunden dauert, wobei optional das Co-Kultivieren 6 Stunden dauert oder etwa dauert.

11. Verfahren nach einem der Ansprüche 1-10, wobei die Auswahl unter Verwendung eines Fluoreszenz-basierten Zellsortierers durchgeführt wird, wobei optional:
(i) der Fluoreszenz-basierte Zellsortierer ein automatisierter Durchflusszytometriesortierer mit hohem Durchsatz ist, optional FX500-Zellsortierer oder Miltenyi-Tyto-Zellsortierer; und/oder
(ii) die Auswahl durch 1 Durchlauf, 2 Durchläufe, 3 Durchläufe oder 4 Durchläufe durch den Fluoreszenz-basierten Zellsortierer erfolgt; und/oder
(iii) die Auswahl bei einer Geschwindigkeit zwischen 10.000 und 100.000 Zellen/Sekunde unter Verwendung eines Fluoreszenz-basierten Einweg-Fluidik-Zellsortierers durchgeführt wird.

12. Verfahren nach einem der Ansprüche 1-11, wobei der eine oder die mehreren Hochregulierungsmarker ausgewählt sind aus der Gruppe bestehend aus CD107, CD107a, CD39, CD103, CD137 (4-1BB), CD59, CD69, CD90, CD38, CD30, CD154, CD252, CD134 (OX40), CD258, CD256, PD-1, TIM-3 und LAG-3, wobei optional der eine oder die mehreren Hochregulierungsmarker ausgewählt ist aus der Gruppe bestehend aus CD107, CD107a, CD39, CD103, CD59, CD90, CD38, CD30, CD154, CD252, CD134, CD258 und CD256.

13. Verfahren nach einem der Ansprüche 1-12, wobei der eine oder die mehreren Hochreguli erungsmarker:
(i) CD134 und/oder CD137 ist; oder
(ii) mindestens zwei Marker umfasst, ausgewählt aus CD107a und CD39, CD107a und CD103, CD107a und CD59, CD107a und CD90, CD107a und CD38, CD39 und CD103, CD39 und CD59, CD39 und CD90, CD39 und CD38, CD103 und CD59, CD103 und CD90, CD103 und CD38, CD59 und CD90, CD59 und CD38 und CD90 und CD38, wobei optional der eine oder die mehreren T-Zell-Hochregulierungsmarker ferner CD137 umfasst, wobei optional der eine oder die mehrere T-Zell-Hochregulierungsmarker mindestens zwei Marker umfasst, ausgewählt aus CD107a und CD137, CD38 und CD137, CD103 und CD137, CD59 und CD137, CD90 und CD137 und CD38 und CD137.

14. Verfahren nach einem der Ansprüche 1-13, wobei:
(i) das Stimulieren der ersten Population 7 bis 21 Tage dauert, optional 7 bis 14 Tage; und/oder
(ii) das Stimulieren der ersten Population in einem geschlossenen System ein gasdurchlässiges Kulturgefäß verwendet; und/oder
(iii) das Stimulieren der ersten Population in einem geschlossenen System unter Verwendung eines Bioreaktors durchgeführt wird.

15. Verfahren nach einem der Ansprüche 1-14, wobei:
(i) das Expandieren der vierten Population 7 bis 21 Tage dauert, optional 7 bis 14 Tage; und/oder
(ii) das Expandieren der vierten Population in einem gasdurchlässigen Kulturgefäß durchgeführt wird; und/oder
(iii) das Expandieren der vierten Population unter Verwendung eines Bioreaktors durchgeführt wird.

16. Verfahren nach einem der Ansprüche 1-15, wobei die Zeit vom Verarbeiten der biologischen Proben, die die Population von Lymphozyten enthalten, bis zum Erzeugen der expandierten T-Zellen weniger als 30 Tage beträgt.

17. Verfahren nach einem der Ansprüche 1-16, wobei:
(i) der Tumor ein Tumor von einem Epithelkrebs ist;
(ii) der Tumor ein Tumor von Melanom, squamösem Krebs der Lunge, Lungenadenokarzinom, Blasenkrebs, kleinzelligem Lungenkrebs, Speiseröhrenkrebs, Darmkrebs (CRC), Gebärmutterhalskrebs, Kopf-Hals-Krebs, Magenkrebs oder Uteruskrebs ist; oder
(iii) der Tumor ein Tumor von nicht-kleinzelligem Lungenkrebs (NSCLC), CRC, Eierstockkrebs, Brustkrebs, Speiseröhrenkrebs, Magenkrebs, Bauchspeicheldrüsenkrebs, Cholangiokarzinom-Krebs, Endometriumkrebs ist, wobei optional der Brustkrebs HR+/Her2-Brustkrebs, triple-negativer Brustkrebs (TNBC) oder HER2+-Brustkrebs ist.

18. Verfahren nach einem der Ansprüche 1-17, wobei die biologische Probe ein Tumor ist und die Population von Lymphozyten aus der biologischen Probe von tumorinfiltrierenden Lymphozyten sind.

19. Verfahren nach einem der Ansprüche 1-18, wobei:
A) die erste Population von Lymphozyten als Tumorfragmente von einem resezierten Tumor verarbeitet werden, wobei optional die erste Population von Lymphozyten ein oder mehrere Tumorfragmente von dem resezierten Tumor sind, wobei optionaler die erste Population von Lymphozyten zur Stimulation in dem geschlossenen System bei etwa 1 Tumorfragment pro 2 cm² geimpft werden; oder
B) die erste Population von Lymphozyten als eine Einzelzellsuspension durch Homogenisierung und/oder enzymatische Verdauung von einem oder mehreren Tumorfragmenten von einem resezierten Tumor verarbeitet werden, wobei optional die enzymatische Verdauung durch Inkubation mit einer Kollagenase erfolgt, optional Kollagenase IV oder Kollagenase I/II, wobei optionaler die erste Population von Lymphozyten zur Stimulation in dem geschlossenen System bei etwa 5 × 10⁵ bis bei oder etwa 2 × 10⁶ Gesamtzellen pro 2 cm² geimpft werden.

20. Verfahren nach einem der Ansprüche 1-19, ferner umfassend das Ernten von Zellen, die durch das Verfahren erzeugt werden, optional umfassend das Formulieren der geernteten Zellen mit einem Kryoprotektivum.

21. Verfahren nach einem der Ansprüche 1-20, wobei die Population von T-Zellen der therapeutischen Zusammensetzung in der Lage sind, IFN-gamma bei einer Konzentration von größer als bei oder etwa 30 pg/ml zu erzeugen, optional größer als bei oder etwa 60 pg/ml, anschließend an antigenspezifische Stimulation.

## Revendications

1. Procédé de fabrication de lymphocytes T destinés à être utilisés dans une composition cellulaire thérapeutique, dans lequel les lymphocytes T expriment un récepteur de lymphocytes T (TCR) qui reconnaît un antigène à la surface d'une cellule cible, le procédé comprenant :
a. le traitement d'un échantillon biologique contenant une population de cellules lymphocytaires T obtenues à partir d'un sujet donneur atteint d'une tumeur, afin de produire une première population de cellules lymphocytaires T ;
b. la stimulation de la première population avec un ou plusieurs premiers agents de stimulation de lymphocytes T dans des conditions d'expansion des lymphocytes T de la population afin de produire une deuxième population de lymphocytes T activés, dans lequel la stimulation est réalisée dans un système fermé utilisant un milieu sans sérum, dans lequel les un ou plusieurs premiers agents de stimulation de lymphocytes T sont ou comprennent de l'IL-2 recombinante ;
c. la co-culture de la deuxième population de lymphocytes T en présence de cellules présentatrices d'antigène qui présentent un ou plusieurs peptides non natifs sur un complexe majeur d'histocompatibilité (MHC) dans un système fermé utilisant un milieu sans sérum, lesdits un ou plusieurs peptides non natifs étant des peptides correspondant à des mutations somatiques non synonymes associées à la tumeur du sujet, afin de produire une troisième population de cellules contenant des lymphocytes T comprenant des récepteurs endogènes de lymphocytes T réactifs à des peptides de la tumeur codant pour des mutations ;
d. la sélection, à partir de la troisième population de cellules, de la population de lymphocytes T contenant des TCR endogènes qui sont réactifs aux peptides présents sur les APC, sur la base d'un ou de plusieurs marqueurs de régulation positive exprimés sur des lymphocytes T réactifs ou activés dans un système fermé, afin de produire une quatrième population contenant les lymphocytes T sélectionnés ; et
e. l'expansion de la quatrième population de cellules sélectionnées en présence d'un ou de plusieurs deuxièmes agents de stimulation des lymphocytes T dans des conditions permettant l'expansion des lymphocytes T de la population dans un système fermé utilisant un milieu sans sérum pour produire une composition de lymphocytes T expansés à utiliser comme composition cellulaire thérapeutique, dans lequel les un ou plusieurs deuxièmes agents de stimulation de lymphocytes T sont ou comprennent de l'IL-2 recombinante.

2. Procédé selon la revendication 1, dans lequel :
(a) les cellules présentatrices d'antigène sont des cellules nucléées telles que des cellules dendritiques, des phagocytes mononucléaires, des lymphocytes B, des cellules endothéliales ou l'épithélium thymique, éventuellement dans lequel les cellules présentatrices d'antigène sont des cellules dendritiques ; et/ou
(b) les cellules présentatrices d'antigènes et/ou les lymphocytes T sont autologues du sujet.

3. Procédé selon la revendication 1 ou la revendication 2, dans lequel les lymphocytes T sont des cellules CD4+, des cellules CD8+, ou des cellules CD4+ et des cellules CD8+.

4. Procédé selon l'une quelconque des revendications 1 à 3, dans lequel :
(A) les un ou plusieurs premiers agents de stimulation de lymphocytes T comprennent de l'IL-2 recombinante et dans lequel la concentration de l'IL-2 recombinante est :
(i) de 100 UI/mL à 6000 UI/mL ;
(ii) de 300 UI/mL à 1000 UI/mL ; ou
(iii) de ou d'environ 300 UI/mL ; et/ou
(B) les un ou plusieurs deuxièmes agents de stimulation de lymphocytes T comprennent de l'IL-2 recombinante et dans lequel la concentration de l'IL-2 recombinante est :
(i) de 100 UI/mL à 6000 UI/mL ;
(ii) de 300 UI/mL à 1000 UI/mL ; ou
(iii) de ou d'environ 300 UI/mL.

5. Procédé selon l'une quelconque des revendications 1 à 3, dans lequel :
(i) les un ou plusieurs premiers agents de stimulation de lymphocytes T comprennent de l'IL-2 recombinante et l'IL-2 recombinante est présente dans une quantité qui est de ou d'environ 6000 UI/mL ; et/ou
(ii) les un ou plusieurs deuxièmes agents de stimulation de lymphocytes T comprennent de l'IL-2 recombinants et l'IL-2 recombinants est présents dans une quantité qui est de ou d'environ 6000 UI/mL.

6. Procédé selon l'une quelconque des revendications 1 à 5, dans lequel les un ou plusieurs deuxièmes agents de stimulation de lymphocytes T comprennent en outre un anticorps anti-CD3, éventuellement OKT3, éventuellement dans lequel la concentration de l'anticorps anti-CD3 est d'environ 50 ng/mL.

7. Procédé selon l'une quelconque des revendications 1 à 6, dans lequel les un ou plusieurs deuxièmes agents de stimulation de lymphocytes T comprennent en outre des cellules nourricières qui sont des cellules mononucléaires de sang périphérique (PBMC) non mitotiques, éventuellement dans lequel les cellules nourricières sont irradiées aux rayons gamma.

8. Procédé selon l'une quelconque des revendications 1 à 7, où les un ou plusieurs peptides non natifs sont chargés sur des cellules présentatrices d'antigène par une impulsion peptidique, éventuellement dans lequel :
(i) l'impulsion peptidique est réalisée par électroporation ;
(ii) les un ou plusieurs peptides non natifs ont une longueur de 5 à 30 acides aminés, éventuellement de 12 à 25 acides aminés, éventuellement de ou d'environ 25 acides aminés ; et/ou
(iii) les un ou plusieurs peptides non natifs :
(a) constituent une réserve de peptides et la concentration de peptides dans la réserve de peptides pour l'impulsion peptidique est entre à ou environ 0,001 µg/ml et à ou environ 40 µg/ml, à ou environ 0,01 µg/ml et à ou environ 40 µg/ml, à ou environ 0,1 µg/mL et à ou environ 40 µg/mL, à ou environ 1 µg/mL et à ou environ 40 µg/mL, à ou environ 0,01 µg/mL et à ou environ 10 µg/mL ou à ou environ 1 µg/mL et à ou environ 10 µg/mL ; ou
(b) sont des peptides individuels et la concentration de peptides individuels pour l'impulsion peptidique est entre à ou environ 0,00001 µg/ml et à ou environ 1 µg/ml, à ou environ 0,00001 µg/ml et à ou environ 0,1 µg/ml, à ou environ 0,00001 µg/ml et à ou environ 0,01 µg/ml, à ou environ 0,0001 µg/ml et à ou environ 1 µg/mL, à ou environ 0,0001 µg/mL et à ou environ 0,1 µg/mL, à ou environ 0,0001 µg/mL et à ou environ 0,1 µg/mL, à ou environ 0,0001 µg/mL et à ou environ 0,01 µg/mL, éventuellement dans lequel la concentration de peptides individuels des un ou plusieurs peptides non natifs, en moyenne, est de ou environ 0,00001 µg/mL à ou environ 0,01 µg/mL ou la concentration de peptides individuels des un ou plusieurs peptides non natifs, en moyenne, est de ou d'environ 0,0001 µg/mL à ou environ 0,001 µg/mL.

9. Procédé selon l'une quelconque des revendications 1 à 8, dans lequel le rapport de co-culture entre les cellules présentatrices d'antigène et les lymphocytes T est entre 20:1 et 1:1, entre 15:1 et 1:1, entre 10:1 et 1:1, entre 5:1 et 1:1, entre 2,5:1 et 1:1, entre 1:20 et 1:1, entre 1:15 et 1:1, entre 1:10 et 1:1, entre 1:5 et 1:1, ou entre 1:2,5 et 1:1, éventuellement dans lequel le rapport de co-culture entre les cellules présentatrices d'antigène et les lymphocytes T est de ou d'environ 1:1.

10. Procédé selon l'une quelconque des revendications 1 à 9, dans laquelle la co-culture dure de 2 heures à 24 heures, éventuellement dans lequel la co-culture dure environ 6 heures.

11. Procédé selon l'une quelconque des revendications 1 à 10, dans lequel la sélection est réalisée à l'aide d'un trieur de cellules à base de fluorescence, éventuellement dans lequel :
(i) le trieur de cellules à base de fluorescence est un trieur automatisé de cytométrie en flux à haut débit, éventuellement le trieur de cellules FX500 ou le trieur de cellules Miltenyi Tyto ; et/ou
(ii) la sélection est réalisée par 1, 2, 3 ou 4 cycles par le trieur de cellules à base de fluorescence ; et/ou
(iii) la sélection est réalisée à une vitesse entre 10 000 et 100 000 cellules/seconde à l'aide d'un trieur de cellules fluidique jetable à base de fluorescence.

12. Procédé selon l'une quelconque des revendications 1 à 11, dans lequel les un ou plusieurs marqueurs de régulation positive sont sélectionnés dans le groupe constitué de CD107, CD107a, CD39, CD103, CD137 (4-1BB), CD59, CD69, CD90, CD38, CD30, CD154, CD252, CD134 (OX40), CD258, CD256, PD-1, TIM-3 et LAG-3, éventuellement dans lequel les un ou plusieurs marqueurs de régulation positive sont sélectionnés dans le groupe constitué de CD107, CD107a, CD39, CD103, CD59, CD90, CD38, CD30, CD154, CD252, CD134, CD258 et CD256.

13. Procédé selon l'une quelconque des revendications 1 à 12, dans lequel les un ou plusieurs marqueurs de régulation positive :
(i) sont CD134 et/ou CD137 ; ou
(ii) comprennent au moins deux marqueurs sélectionnés parmi CD107a et CD39, CD107a et CD103, CD107a et CD59, CD107a et CD90, CD107a et CD38, CD39 et CD103, CD39 et CD59, CD39 et CD90, CD39 et CD38, CD103 et CD59, CD103 et CD90, CD103 et CD38, CD59 et CD90, CD59 et CD38 et CD90 et CD38, éventuellement, dans lequel les un ou plusieurs marqueurs de régulation positive de lymphocytes T comprennent en outre CD137, éventuellement dans lequel les un ou plusieurs marqueurs de régulation positive de lymphocytes T comprennent au moins deux marqueurs sélectionnés parmi CD107a et CD137, CD38 et CD137, CD103 et CD137, CD59 et CD137, CD90 et CD137 et CD38 et CD137.

14. Procédé selon l'une quelconque des revendications 1 à 13, dans lequel :
(i) la stimulation de la première population dure de 7 à 21 jours, éventuellement de 7 à 14 jours ; et/ou
(ii) la stimulation de la première population dans un système fermé s'effectue à l'aide d'un récipient de culture perméable aux gaz ; et/ou
(iii) la stimulation de la première population dans un système fermé s'effectue à l'aide d'un bioréacteur.

15. Procédé selon l'une quelconque des revendications 1 à 14, dans lequel :
(i) l'expansion de la quatrième population dure de 7 à 21 jours, éventuellement de 7 à 14 jours ; et/ou
(ii) l'expansion de la quatrième population est réalisée dans un récipient de culture perméable aux gaz ; et/ou
(iii) l'expansion de la quatrième population est réalisée à l'aide d'un bioréacteur.

16. Procédé selon l'une quelconque des revendications 1 à 15, dans lequel le temps du traitement des échantillons biologiques contenant la population de lymphocytes à la production des lymphocytes T expansés est inférieur à 30 jours.

17. Procédé selon l'une quelconque des revendications 1 à 16, dans lequel :
(i) la tumeur est une tumeur d'un cancer épithélial ;
(ii) la tumeur est une tumeur mélanique, un cancer squameux du poumon, un adénocarcinome du poumon, un cancer de la vessie, un cancer du poumon à petites cellules, un cancer de l'œsophage, un cancer colorectal (CRC), un cancer du col de l'utérus, un cancer de la tête et du cou, un cancer de l'estomac ou un cancer de l'utérus ; ou
(iii) la tumeur est une tumeur d'un cancer du poumon non à petites cellules (NSCLC), d'un CRC, d'un cancer de l'ovaire, d'un cancer du sein, d'un cancer de l'œsophage, d'un cancer gastrique, d'un cancer du pancréas, d'un cancer cholangiocarcinome, d'un cancer de l'endomètre, éventuellement dans lequel le cancer du sein est un cancer du sein HR+/Her2-, un cancer du sein triple négatif (TNBC) ou un cancer du sein HER2+.

18. Procédé selon l'une quelconque des revendications 1 à 17, dans lequel l'échantillon biologique est une tumeur et la population de lymphocytes provenant de l'échantillon biologique est constituée de lymphocytes infiltrant la tumeur.

19. Procédé selon l'une quelconque des revendications 1 à 18, dans lequel :
A) la première population de lymphocytes est traitée comme des fragments de tumeur provenant d'une tumeur réséquée, éventuellement dans lequel la première population de lymphocytes est un ou plusieurs fragments de tumeur provenant de la tumeur réséquée, encore éventuellement dans lequel la première population de lymphocytes est ensemencée pour la stimulation dans le système fermé à environ 1 fragment de tumeur pour 2 cm² ; ou
B) la première population de lymphocytes est traitée comme une suspension cellulaire unique par homogénéisation et/ou digestion enzymatique d'un ou de plusieurs fragments de tumeur provenant d'une tumeur réséquée, éventuellement dans lequel la digestion enzymatique se fait par incubation avec une collagénase, éventuellement la collagénase IV ou la collagénase I/II, encore éventuellement dans lequel la première population de lymphocytes est ensemencée pour la stimulation dans le système fermé à environ 5 × 10⁵ jusqu'à ou environ 2 × 10⁶ cellules totales pour 2 cm².

20. Procédé selon l'une quelconque des revendications 1 à 19, comprenant en outre la récolte des cellules produites par le procédé, comprenant éventuellement la formulation des cellules récoltées avec un cryoprotecteur.

21. Procédé selon l'une quelconque des revendications 1 à 20, dans lequel la population de lymphocytes T de la composition thérapeutique est capable de produire de l'IFNgamma à une concentration supérieure à ou d'environ 30 pg/mL, éventuellement supérieure à ou d'environ 60 pg/mL, à la suite d'une stimulation spécifique à un antigène.
